(19) Europäisches Patentamt
European Patent Office
Office européen des brevets

(11) Publication number: **0 620 277 A1**

(12) # EUROPEAN PATENT APPLICATION

(21) Application number: **94200605.7**

(22) Date of filing: **09.03.94**

(51) Int. Cl.$^5$: **C12N 15/44**, A61K 48/00, A61K 31/70

(30) Priority: **18.03.93 US 32383**
**08.07.93 US 89985**

(43) Date of publication of application:
**19.10.94 Bulletin 94/42**

(84) Designated Contracting States:
**AT BE CH DE DK ES FR GB GR IE IT LI LU NL PT SE**

(71) Applicant: **MERCK & CO. INC.**
**126, East Lincoln Avenue**
**P.O. Box 2000**
**Rahway New Jersey 07065-0900 (US)**
Applicant: **VICAL INCORPORATED**
**9373 Towne Centre Drive,**
**Suite 100**
**San Diego, California 92121 (US)**

(72) Inventor: **Donnelly, John J.**
**1505 Bierwood Road**
**Havertown, PA 19083 (US)**
Inventor: **Montgomery, Donna L**
**9, Hickory Lane**
**Chalfont , PA 18914 (US)**
Inventor: **Dwarki, Varavani J.**
**1175 Broadway Apt. N**
**Alameda, CA 94501 (US)**
Inventor: **Parker, Suezanne E.**
**3646 Carmel Landing**
**San Diego, CA 92130 (US)**
Inventor: **Liu, Magaret A.**
**4 Cushman Road**
**Rosemont, PA 19190 (US)**
Inventor: **Shiver, John W.**
**125 Beulah Road**
**Doylestown, PA 18901 (US)**
Inventor: **Ulmer, Jeffrey B.**
**128 Dolly Circle**
**Chalfont, PA 18914 (US)**

(74) Representative: **Cole, William Gwyn et al**
**European Patent Department**
**Merck & Co., Inc.**
**Terlings Park**
**Eastwick Road**
**Harlow Essex CM20 2OR (GB)**

(54) **Nucleic acid pharmaceuticals.**

(57) DNA constructs encoding influenza virus gene products, capable of being expressed upon direct introduction, via injection or otherwise, into animal tissues, are novel prophylactic pharmaceuticals which can provide immune protection against infection by homologous and heterologous strains of influenza virus.

EP 0 620 277 A1

## BACKGROUND OF THE INVENTION

### i. Field of the Invention:

This invention relates to the production and use of a novel pharmaceutical product: a nucleic acid which, when directly introduced into living vertebrate tissue, induces the production of immune responses which specifically recognize human influenza virus.

### ii. Background of the Invention:

Influenza is an acute febrile illness caused by infection of the respiratory tract with influenza A or B virus. Outbreaks of influenza occur worldwide nearly every year with periodic epidemics or pandemics. Influenza can cause significant systemic symptoms, severe illness (such as viral pneumonia) requiring hospitalization, and complications such as secondary bacterial pneumonia. Recent U.S. epidemics are thought to have resulted in >10,000 (up to 40,000) excess deaths per year and 5,000-10,000 deaths per year in non-epidemic years. The best strategy for prevention of the morbidity and mortality associated with influenza is vaccination. The current licensed vaccines are derived from virus grown in eggs, then inactivated, and include three virus strains (two A strains and one B strain). Three types of vaccines are available: whole-virus, subvirion, and purified surface antigen. Only the latter two are used in children because of increased febrile responses with the whole-virus vaccine. Children under the age of 9 require two immunizations, while adults require only a single injection. However, it has been suggested [see Medical Letter 32:89-90, Sept. 17, 1993] that "patients vaccinated early in the autumn might benefit from a second dose in the winter or early spring," due to the observations that in some elderly patients, the antibody titers following vaccination may decline to less-than-protective levels within four months or less. These vaccines are reformulated every year by predicting which recent viral strains will clinically circulate and evaluating which new virulent strain is expected to be predominant in the coming flu season. Revaccination is recommended annually.

A. The limitations of the licensed vaccine are:

1) Antigenic variation, particularly in A strains of influenza, results in viruses that are not neutralized by antibodies generated by a previous vaccine (or previous infection). New strains arise by point mutations (antigenic drift) and by reassortment (antigenic shift) of the genes encoding the surface glycoproteins (hemagglutinin [HA] and neuraminidase), while the internal proteins are highly conserved among drifted and shifted strains. Immunization elicits "homologous" strain-specific antibody-mediated immunity, not "heterologous" group-common immunity based on cell-mediated immunity.

2) Even if the predominant, circulating strains of influenza virus do not shift or drift significantly from one year to the next, immunization must be given each year because antibody titers decline. Although hemagglutination-inhibiting (HI) and neutralizing antibodies are reported by some to persist for months to years with a subsequent gradual decline, the Advisory Committee on immunization Practices cites the decline in antibody titers in the year following vaccination as a reason for annual immunization even when there has been no major drift or shift. (HI antibodies inhibit the ability of influenza virus to agglutinate red blood cells. Like neutralizing antibodies, they are primarily directed against the HA antigen. Hemagglutination inhibition tests are easier and less expensive to perform than neutralization assays are, and thus are often used as a means to assess the ability of antibodies raised against one strains of influenza to react to a different strain). As mentioned above, The Medical Letter suggests that certain high-risk, older individuals should be vaccinated twice in one season due to short-lived protective antibody titers.

3) The effectiveness of the vaccine is suboptimal. Development of the next season's vaccine relies upon predicting the upcoming circulating strains (via sentinel sampling in Asia), which is inexact and can result in a poor match between strains used for the vaccine and those that actually circulate in the field. Moreover, as occurred during the 1992-1993 flu season, a new H3N2 strain (A/Beijing/92) became clinically apparent during the latter phase of the flu season. This prompted a change in the composition of the 1993-1994 vaccine, due to poor cross-reactivity with A/Beijing/92 of the antibody induced by the earlier H3N2 strain (A/Beijing/89) due to antigenic shift. However, due to the length of time needed to make and formulate the current licensed vaccine, the new vaccine strain could not be introduced during the 1992-1993 season despite the evidence for poor protection from the existing vaccine and the increased virulence of the new circulating H3N2 strain.

Even when the vaccine and circulating strains are well-matched, the licensed vaccine prevents illness in only about 70% of healthy children and young adults and in 30-40% of frail older adults. Thus other criteria are used to indicate the vaccine's efficacy when the vaccine strains correspond to the circulating strains. These criteria include prevention of severe illness and secondary complications, which are reflected by prevention of hospitalization (70% for the elderly living at home vs. 50-60% for the elderly living in nursing homes) and prevention of death (80% for nursing home residents). Herd immunity to reduce the spread of infection in a nursing home is considered another benefit of immunization.

B. Characteristics of an Ideal Universal Influenza Vaccine (Objects of the Invention):

1) Generation of group-common (heterologous) protection. A universal vaccine would be able to protect against different strains, within an H3N2 subtype for example, and possibly even across subtypes, e.g., from H1N1 to H3N2. This likely would be mediated by cytotoxic T lymphocytes (CTL) recognizing antigens from internal conserved viral proteins, although neutralizing antibodies directed against conserved portions of membrane-bound proteins also might play a role.

2) Increased breadth of antibody response. Because CTL are thought to play a role in recovery from disease, a vaccine based solely upon a CTL response would be expected to shorten the duration of illness (potentially to the point of rendering illness subclinical), but it would not prevent illness completely. The method of producing the current influenza vaccine by passage in eggs has been shown experimentally to be capable of selecting for virus subpopulations that have altered HA antigenicity. As a result, vaccine efficacy could be diminished because the antibody elicited by the vaccine may not be completely effective against the predominant circulating strain. Thus, one would like to generate antibodies which would have an improved breadth of response compared to the current vaccine. The 1992-93 flu season offered an excellent case study of the limitations of the current vaccine in that the vaccine, which utilized A/Beijing/89, generated antibodies which were poorly cross-reactive (and poorly protective) against the new A/Beijing/92 strain which was also more virulent. Both strains are H3N2, i.e., of the same subtype. In terms of amino acid sequence, however, the A/Beijing/92-like strains differed from the A/Beijing/89-like strains by only 11 point mutations (positions 133, 135, 145, 156, 157, 186, 190, 191, 193, 226, and 262) in the HA1 region. It is not known whether the current manufacturing process influenced the lack of cross-reactivity, but clearly an improvement in the breadth of antibody response is desired.

3) Increased duration of antibody responses. Because one of the very groups that is at highest risk for the morbidity and mortality of influenza infection (elderly) is also the group in whom protective antibody titers may decline too rapidly for annual immunization to be effective, an improved vaccine should generate protective titers of antibody that persist longer.

C. Polynucleotides as a Vaccine

Intramuscular inoculation of polynucleotide constructs, i.e., DNA plasmids encoding proteins have been shown to result in the in situ generation of the protein in muscle cells. By using cDNA plasmids encoding viral proteins, both antibody and CTL responses were generated, providing homologous and heterologous protection against subsequent challenge with either the homologous or cross-strain protection, respectively. Each of these types of immune responses offers a potential advantage over existing vaccination strategies. The use of PNVs to generate antibodies may result in an increased duration of the antibody responses as well as the provision of an antigen that can have both the exact sequence of the clinically circulating strain of virus as well as the proper post-translational modifications and conformation of the native protein (vs. a recombinant protein). The generation of CTL responses by this means offers the benefits of cross-strain protection without the use of a live potentially pathogenic vector or attenuated virus.

D. Further Description of the Background:

Thus, a major challenge to the development of vaccines against viruses such as influenza, against which neutralizing antibodies are generated, is the diversity of the viral envelope proteins among different isolates or strains. As cytotoxic T-lymphocytes in both mice and humans are capable of recognizing epitopes derived from conserved internal viral proteins [J.W. Yewdell *et al.*, Proc. Natl. Acad. Sci. (USA) **82**, 1785 (1985); A.R.M. Townsend, *et al.*, Cell **44**, 959 (1986); A.J. McMichael *et al.*, J. Gen. Virol. **67**, 719 (1986); J. Bastin *et al.*, J. Exp. Med. **165**, 1508 (1987); A.R.M. Townsend and H. Bodmer, Annu. Rev. Immunol. **7**, 601 (1989)], and are thought to be important in the immune response against viruses [Y.-L. Lin and B.A. Askonas, J. Exp. Med. **154**, 225 (1981); I. Gardner *et al.*, Eur. J. Immunol. **4**, 68 (1974); K.L. Yap and G.L. Ada, Nature **273**, 238 (1978); A.J. McMichael *et al.*, New Engl. J. Med. **309**, 13 (1983); P.M. Taylor and B.A. Askonas, Immunol. **58**, 417 (1986)], efforts have been directed towards the development of CTL vaccines capable of providing heterologous protection against different viral strains.

$CD8^+$ CTLs kill virally-infected cells when their T cell receptors recognize viral peptides associated with MHC class I molecules [R.M. Zinkernagel and P.C. Doherty, *ibid.* **141**, 1427 (1975); R.N. Germain, Nature **353**, 605 (1991)]. These peptides are derived from endogenously synthesized viral proteins, regardless of the protein's location or function within the virus. Thus, by recognition of epitopes from conserved viral proteins, CTLs may provide cross-strain protection. Peptides capable of associating with MHC class I for CTL recognition originate from proteins that are present in or pass through the cytoplasm or endoplasmic reticulum [J.W. Yewdell and J.R. Bennink, Science **244**, 1072 (1989); A.R.M. Townsend *et al.*, Nature **340**, 443 (1989); J.G. Nuchtern *et al.*, *ibid.* **339**, 223 (1989)]. Therefore, in general, exogenous proteins, which enter the endosomal processing pathway (as in the case of antigens presented by MHC class II molecules), are not effective at generating $CD8^+$ CTL responses.

Most efforts to generate CTL responses have either used replicating vectors to produce the protein antigen within the cell [J.R. Bennink *et al.*, *ibid.* **311**, 578 (1984); J.R. Bennink and J.W. Yewdell, Curr. Top. Microbiol. Immunol. **163**, 153 (1990); C.K. Stover *et al.*, Nature **351**, 456 (1991); A. Aldovini and R.A. Young, Nature **351**, 479 (1991); R. Schafer *et al.*, J. Immunol. **149**, 53 (1992); C.S. Hahn *et al.*, Proc. Natl. Acad. Sci. (USA) **89**, 2679 (1992)], or they have focused upon the introduction of peptides into the cytosol [F.R. Carbone and M.J. Bevan, J. Exp. Med. **169**, 603 (1989); K. Deres *et al.*, Nature **342**, 561 (1989); H. Takahashi *et al.*, *ibid.* **344**, 873 (1990); D.S. Collins *et al.*, J. Immunol. **148**, 3336 (1992); M.J. Newman *et al.*, *ibid.* **148**, 2357 (1992)]. Both of these approaches have limitations that may reduce their utility as vaccines. Retroviral vectors have restrictions on the size and structure of polypeptides that can be expressed as fusion proteins while maintaining the ability of the recombinant virus to replicate [A.D. Miller, Curr. Top. Microbiol. Immunol. **158,** 1 (1992)], and the effectiveness of vectors such as vaccinia for subsequent immunizations may be compromised by immune responses against the vectors themselves [E.L. Cooney *et al.*, Lancet **337**, 567 (1991)]. Also, viral vectors and modified pathogens have inherent risks that may hinder their use in humans [R.R. Redfield *et al.*, New Engl. J. Med. **316**, 673 (1987); L. Mascola *et al.*, Arch. Intern. Med. **149**, 1569 (1989)]. Furthermore, the selection of peptide epitopes to be presented is dependent upon the structure of an individual's MHC antigens and, therefore, peptide vaccines may have limited effectiveness due to the diversity of MHC haplotypes in outbred populations.

Benvenisty, N., and Reshef, L. [PNAS **83**, 9551-9555, (1986)] showed that $CaCl_2$ precipitated DNA introduced into mice intraperitoneally, intravenously or intramuscularly could be expressed. The intramuscular (i.m.) injection of DNA expression vectors in mice has been demonstrated to result in the uptake of DNA by the muscle cells and expression of the protein encoded by the DNA [J.A. Wolff *et al.*, Science **247**, 1465 (1990); G. Ascadi *et al.*, Nature **352**, 815 (1991)]. The plasmids were shown to be maintained episomally and did not replicate. Subsequently, persistent expression has been observed after i.m. injection in skeletal muscle of rats, fish and primates, and cardiac muscle of rats [H. Lin *et al.*, Circulation **82**, 2217 (1990); R.N. Kitsis *et al.*, Proc. Natl. Acad. Sci. (USA) **88**, 4138 (1991); E. Hansen *et al.*, FEBS Lett. **290**, 73 (1991); S. Jiao *et al.*, Hum. Gene Therapy **3**, 21 (1992); J.A. Wolff *et al.*, Human Mol. Genet. **1**, 363 (1992)]. The technique of using nucleic acids as therapeutic agents was reported in WO90/11092 (4 October 1990), in which naked polynucleotides were used to vaccinate vertebrates.

It is not necessary for the success of the method that immunization be intramuscular. Thus, Tang et al., [Nature, **356**, 152-154 (1992)] disclosed that introduction of gold microprojectiles coated with DNA encoding bovine growth hormone (BGH) into the skin of mice resulted in production of anti-BGH antibodies in the mice. Furth et al., [Analytical Biochemistry, **205**, 365-368, (1992)] showed that a jet injector could be used to transfect skin, muscle, fat, and mammary tissues of living animals. Various methods for introducing nucleic acids was recently reviewed by Friedman, T., [Science, **244**, 1275-1281 (1989)]. See also Robinson et al., Abstracts of Papers Presented at the 1992 meeting on Modern Approaches to New Vaccines, Including Prevention of AIDS, Cold Spring Harbor, p92, where the im, ip, and iv administration of avian influenza DNA into chickens was alleged to have provided protection against lethal challenge. However, there was no disclosure of which avian influenza virus genes were used. In addition, only H7 specific immune responses were alleged, without any mention of induction of cross-strain protection.

Therefore, this invention contemplates any of the known methods for introducing nucleic acids into living tissue to induce expression of proteins. This invention provides a method for introducing viral proteins into the antigen processing pathway to generate virus-specific CTLs. Thus, the need for specific therapeutic agents capable of eliciting desired prophylactic immune responses against viral pathogens is met for influenza virus by this invention. Of particular importance in this therapeutic approach is the ability to induce T-cell immune responses which can prevent infections even of virus strains which are heterologous to the strain from which the antigen gene was obtained. Therefore, this invention provides DNA constructs encoding viral proteins of the human influenza virus nucleoprotein (NP), hemagglutinin (HA), neuraminidase (NM), matrix (M), nonstructural (NS), polymerase (PB1 and PB2 = basic polymerases 1 and 2; PA = acidic polymerase) or any of the other influenza genes which encode products which generate specific CTLs.

The influenza virus has a ribonucleic acid (RNA) genome, consisting of multiple RNA segments. Each RNA encodes at least one gene product. The NP gene product binds to RNA and translocates viral RNA into the nucleus of the infected cell. The sequence is conserved, with only about 7% divergence in the amino acid sequence having arisen over a period of 50 years. The P gene products (PB1, PB2, PA) are responsible for synthesizing new viral RNAs. These genes are even more highly conserved than the NP gene. HA is the major viral envelope gene product. It is less highly conserved than NP. It binds a cellular receptor and is therefore instrumental in the initiation of new influenza infections. The major neutralizing antibody response is directed against this gene product. A substantial cytotoxic T lymphocyte response is also directed against this protein. Current vaccines against human influenza virus incorporate three strains of influenza virus or their HA proteins. However, due to the variability in the protein sequence of HA in different strains, the vaccine must constantly be tailored to the strains which are current in causing pathology. However, HA does have some conserved elements for the generation of CTLs, if properly presented. The NS1 and NS2 gene products have incompletely characterized biological functions, but may be significant in production of protective CTL responses. Finally, the M1 and M2 gene products, which are slightly more conserved than in HA, induce a major CTL response. The M1 protein is a very abundant viral gene product.

The protective efficacy of DNA vaccination against subsequent viral challenge is demonstrated by immunization with nonreplicating plasmid DNA encoding one or more of the above mentioned viral proteins. This is advantageous since no infectious agent is involved, no assembly of virus particles is required, and determinant selection is permitted. Furthermore, because the sequence of nucleoprotein and several of the other viral gene products is conserved among various strains of influenza, protection against subsequent challenge by a virulent strain of influenza virus that is homologous to or heterologous to the strain from which the cloned gene is obtained is enabled.

## SUMMARY OF THE INVENTION

DNA constructs capable of being expressed upon direct introduction, via injection or otherwise, into animal tissues, are novel prophylactic pharmaceuticals. They induce cytotoxic T lymphocytes (CTLs) specific for viral antigens which respond to different strains of virus, in contrast to antibodies which are generally strain-specific. The generation of such CTLs in vivo usually requires endogenous expression of the antigen, as in the case of virus infection. To generate a viral antigen for presentation to the immune system, without the limitations of direct peptide delivery or the use of viral vectors, plasmid DNA encoding human influenza virus proteins was injected into the quadriceps of BALB/c mice, this resulted in the generation of influenza virus-specific CTLs and protection from subsequent challenge with a heterologous strain of influenza virus, as measured by decreased viral lung titers, inhibition of weight loss, and increased survival. High titer neutralizing antibodies to hemagglutinin and antibodies to nucleoprotein were generated in rhesus monkeys, and decreased nasal virus titers were observed following homologous and heterologous challenge in ferrets.

Key observations relating to our invention include:

1) Demonstration of efficacy. Heterologous protection is seen following immunization with nucleoprotein (NP) DNA as measured by increased survival, decreased viral lung titers, and inhibition of weight loss in mice challenged with a strain of influenza different from the source strain for the NP gene. In this case, the surface proteins of the two strains were quite different (H1N1 vs. H3N2), and the challenge strain arose 34 years after the initial strain. Immunization of ferrets with NP DNA and matrix (M1) DNA, either separately, together, or in conjunction with HA DNA, provided protection (decreased nasal virus shedding) against challenge with a drifted strain (a clinical isolate). Notably the protection by the DNA cocktail (NP and M1 DNA encoding the Beijing/89 proteins, and HA DNA encoding either the Beijing/89 or the Hawaii/91 HA) was greater against a drifted strain (Georgia/93) in ferrets than that afforded by the licensed vaccine (containing Beijing/89). The cocktail containing the HA DNA from Hawaii/91 appeared to be slightly more efficacious than the cocktail containing the HA DNA from Beijing/89. The protection seen with the cocktail including the HA DNA for Hawaii/91 resulted in protection identical to the protection seen with the homologous HA DNA (Georgia/93), whereas the cocktail with the HA DNA for Beijing/89 was different from the homologous protection, although it still was significantly better than the licensed product. HI antibody was generated in all species tested including mice, ferrets, Rhesus monkeys, and African green monkeys.

2) Persistence. In studies using a DNA encoding a reporter gene, the presence of DNA and protein expression persisted for at least 1.5 years (the longest time tested in mice; Wolff et al., Human Mol. Genet., 1992). Thus, if the influenza gene products also are expressed persistently, the resulting immune response also should persist. The antibodies and CTL (Yankauckas et al., DNA & Cell Biol., 1993), and homologous protective immunity (MRL data) generated by influenza DNA injection have been shown to persist for over one year in mice. Antibodies have been shown to persist in Rhesus monkeys for at least one year so far. Duration of CTL responses and heterologous protection (increased survival) persists to 6 months (the longest time point tested thus far). A slight decline in the degree of heterologous protection occurred, but the protection is boostable.

3) Dose ranging. Dosage studies have been performed in Rhesus monkeys showing that 100 μg of HA DNA given twice resulted in good titers of HI antibody that have persisted to one year so far. The generation of protection (increased survival following heterologous challenge) in mice was seen with doses as low as 6 μg (given 3 times) and with a single injection of 200 μg, but in general an increased number of injections (up to 3) improved the degree of protection. Primate studies revealed that 2 injections of 10 or 100 μg of DNA encoding 3 HAs and NP and M1 (the latter encoding the H3N2 Beijing/89 genes) resulted in HI antibody titers quite similar to those generated by the licensed vaccine. It is important to remember that all of the animals studied are naive to influenza, whereas the target clinical population (older individuals) are all experienced to flu. (Recall that children under 9 are given 2 injections of the licensed vaccine.)

## BRIEF DESCRIPTION OF THE FIGURES

**Fig. 1**. Detection of NP plasmid DNA in muscle by PCR. Mice were injected three times, at three week intervals, with RSV-NP DNA or blank vector (100 μg/leg) into both quadriceps muscles of BALB/c mice, followed by influenza infection. The muscles were removed 4 weeks after the final injection and immediately frozen in liquid nitrogen. They were then pulverized in lysis buffer (25mM Tris-$H_3PO_4$ pH8, 2mM trans-1:2-diaminocyclohexan-tetra-acetic acid (CDTA), 2mM DTT, 10% glycerol, 1% Triton X-100) in a MIKRODISMEMBRATOR™ (B. Braun Instruments), and high molecular weight DNA was extracted by phenol/chloroform and ethanol precipitation. A 40 cycle PCR reaction (PCR was performed as per instructions in Perkin Elmer Cetus GENEAMP™ kit) was performed to detect the presence of NP plasmid DNA in muscle. A 772 base-pair PCR product (see arrowhead), which spans from the CMV promoter through most of the 5' portion of the inserted NP gene was generated from an 18 base long sense oligonucleotide which primed in the promoter region, (GTGTGCACCTCAAGCTGG, SEQ. ID:1:) and a 23 base long oligonucleotide antisense primer in the of the 5' portion of the inserted NP sequence (CCCTTTGAGAATGTTGCACATTC, SEQ. ID:2:). The 772 bp product is seen on an ethidium bromide-stained agarose gel in selected NP DNA-injected muscle samples but not in the blank vector control (600L). Labeling above each lane indicates mouse identification number and right or left leg.

**Fig. 2**. Production of NP antibodies in mice injected with NP DNA. Mice were injected with 100 μg V1-NP DNA in each leg at 0,3 and 6 weeks, and blood was drawn on 0, 2, 5 and 8 weeks. The presence of anti-NP IgG in the serum was assayed by an ELISA (J.J. Donnelly *et al.*, J Immunol. **145**, 3071 (1990)), with NP purified from insect cells that had been transfected with a baculovirus expression vector. The results are plotted as mean $\log_{10}$ ELISA titer ± SEM (n = 10) against time after the first injection of NP DNA. Mice immunized with blank vector generated no detectable NP antibodies.

**Fig. 3**. Percent specific lysis, determined in a 4-hour $^{51}Cr$ release assay, for CTLs obtained from mice immunized with DNA. Mice were immunized with 400 μg V1-NP DNA (solid circles) or blank vector (solid squares) and sacrificed 3-4 weeks later. Negative control CTL were obtained from a naive mouse (open triangles) and positive controls from a mouse that had recovered from infection with A/HK/68 four weeks previously (solid triangles). Graphs depict data from representative individual mice. At least eight individuals were studied for each set of conditions. Panel A: Spleen cells restimulated with NP147-155-pulsed autologous spleen cells and assayed against NP147-155-pulsed P815 cells. Panel B: Spleen cells restimulated with NP147-155-pulsed autologous spleen cells and assayed against P815 targets infected with influenza A/Victoria/73 (H3N2) for 6 hours before addition of CTL. Panel C: Spleen cells restimulated with Con A and IL-2 without additional antigen and assayed against P815 cells pulsed with NP147-155. Panel D: Mice were injected with 200 μg per injection of V1-NP DNA or blank vector three times at three week intervals. Spleens were harvested 4 weeks after the last immunization, spleen cells were cultured with IL-2 and Con A for 7 days, and CTL were assayed against P815 target cells infected with A/Victoria/73.

**Fig. 4**. Mass loss (in grams) and recovery in DNA-immunized mice after unanesthetized intranasal challenge with $10^4$ $TCID_{50}$ of A/HK/68. Mice were immunized three times at 3-week intervals with V1-NP DNA or blank vector, or were not injected, and were challenged 3 weeks after the last immunization. Weights for groups of 10 mice were determined at the time of challenge and daily from day 4 for NP DNA-injected mice (solid circles), blank vector controls (open triangles), and uninjected controls (open circles). Shown are mean weights ± SEM. NP DNA-injected mice displayed significantly less weight loss on day 8 through 13 than blank vector-injected ($p \leqq 0.005$) and uninjected mice ($p \leqq 0.01$), as analyzed by the t-test. No significant difference was noted between the two controls ($p = 0.8$ by the t-test).

Fig. 5. Survival of DNA immunized mice after intranasal challenge (under anesthesia) with $10^{2.5}$ $TCID_{50}$ of A/HK/68. Mice immunized three times at three week intervals with V1-NP DNA (closed circles) or blank vector (open circles) and uninjected controls (open triangles) were challenged three weeks after the final immunization. Percent survival is shown for groups of 9 or 10 mice. Survival of NP DNA-injected mice was significantly greater than controls ($p = 0.0004$ by Chi-square analysis), while no significant difference was seen between blank vector-injected and uninjected mice ($p = 0.17$ by Chi-square analysis).

**Fig. 6**. Sequence of the expression vector V1J, SEQ.ID:10:.

**Fig. 7**. Sequence of the expression vector V1Jneo, SEQ. ID:18:.

**Fig. 8**. Sequence of the CMVintA-BGH promoter-terminator sequence, SEQ. ID:11.

**Fig. 9**. Monkey anti-NP antibody

**Fig. 10**. Ferret hemagglutination inhibition, with the dotted line indicating the minimal protective antibody titer, and the average value being denoted with a circle having a line through it.

**Fig. 11.** IgG Anti-NP antibody in ferrets after DNA immunization.

**Fig. 12**. Influenza virus shedding in ferrets with and without DNA immunization.

**Fig. 13**. Diagram of pRSV-PR-NP and VI-NP vectors. X denotes the inserted coding region.

**Fig. 14**. Schematic of influenza proteins and strains.

**Fig. 15**. Schematic of injected DNA processing inside a cell.

**Fig. 16.** Resistence of ferrets to influenza A/RP/8/34 induced by immunization with HA and internal protein genes.

**Fig.17**. Schematic of V1Jns vector.

**Fig. 18**. African green monkeys were injected with a cocktail of DNAs consisting of HA DNA (A/Beijing/89, B/Panama/90, A/Texas/91), NP DNA (A/PR/34) and M1 DNA (A/PR/34). Each component was either 10 $\mu$g (solid squares) or 100 $\mu$g (solid circles) administered twice with a six week interval (see arrows). For comparison, other animals were injected with licensed subvirion (open squares) and whole virion (open circles) vaccines at the full human dose (45 $\mu$g protein equivalent; 15 $\mu$g per HA). Serum samples were collected every two weeks for 18 weeks and analyzed for HI titer against A/Beijing/89 HA. Data is represented as geometric mean HI titer ± SEM where $n = 3$.

**Fig.19**. Female BALB/c mice (4-6 weeks) were injected with A/PR/34 NP DNA (200 $\mu$g) three times with three week intervals. Negative controls included mice injected with control DNA (200 $\mu$g), recombinant NP protein (10 $\mu$g), and naive, uninjected mice (mock). For comparison, mice infected with influenza virus A/HK/68 (flu) were also tested. CTL were obtained 6 months post-dose one and restimulated *in vitro* with virus-infected, syngeneic spleen cells and tested against NP peptide-pulsed P815 cells at an effector:target ratio of 10:1. Data represent % specific lysis ±sd, where $n = 3$.

**Fig. 20**. C3H/HeN mice were injected with normal $C_2C_{12}$ myoblasts (1 X $10^7$ cells), recombinant NP protein (2 $\mu$g), or NP-transfected myoblasts (1 X $10^7$ cells). This amount of NP protein (2 $\mu$g) was sufficient to generate antibody responses and was equivalent to approximately 100 times the amount of NP present in the transplanted NP-transfected myoblasts. CTL were prepared from these mice six weeks after treatment and restimulated *in vitro* with influenza virus-infected syngeneic spleen cells. As a positive control, CTL were prepared from mice that had been infected with influenza virus A/HK/68. Untreated (solid bars), influenza virus A/Victoria/73-infected (striped bars) and NP-transfected myoblasts (stippled bars) were used as target cells at an effector:target ratio of 25:1. Data are presented as % specific lysis ± sd, where $n = 3$.

**Fig. 21**. Four week old female BALB/c mice were immunized intramuscularly with 200 $\mu$g of NP DNA 3 times at 3 week intervals. Mice were challenged 3 weeks after the third immunization with 300 TCID50 of A/HK/68 administered under anesthesia (total respiratory tract challenge). The proportion of surviving mice (10 mice/group) is plotted versus time after challenge.

**Fig. 22**. Four week old female BALB/c mice were immunized intramuscularly with 100 $\mu$g of NP DNA 3 times at 3 week intervals. Mice were challenged 3 weeks after the third immunization with 300 TCID50 of A/HK/68 administered under anesthesia (total respiratory tract challenge). Mice were weighed daily and the proportion of the initial weight was calculated for each surviving mouse. Mean percent of initial weights are plotted ± SEM versus time after challenge.

**Fig. 23**. Four week old female BALB/c mice were immunized intramuscularly with 200 μg of NP DNA 3 times at 3 week intervals. Mice were challenged 3 weeks after the third immunization with 2000 TCID50 of A/HK/68 administered without anesthesia (upper respiratory tract challenge). Mice were euthanized 7 days after challenge, the lungs were removed and homogenized, and viral titers were determined by serial titration on MDCK cells.

**Fig. 24**. Four week old female BALB/c mice were immunized intramuscularly with 6.25, 25, 100, or 200 μg of NP DNA 3 times at 3 week intervals. Mice were challenged 3 weeks after the third immunization with 300 TCID50 of A/HK/68 administered under anesthesia (total respiratory tract challenge). The proportion of surviving mice (10 mice/group) is plotted versus time after challenge.

**Fig. 25**. Four week old female BALB/c mice were immunized i.m. 3 times at 3 week intervals with 200 μg of A/PR/34 NP DNA, control DNA, or sham injected. Mice were then challenged with 300 TCID50 of A/HK/68 under anesthesia 6, 12, and 25 weeks following the third injection of DNA. Selected mice were reimmunized with 200 μg of NP DNA at week 22 and then challenged at week 25 ("Reboost"). Mean weights are shown as a percentage of the initial total weight for each group. The control weight shown is the mean of the weights of all of the control groups from the 6, 12, and 25 week challenges, a total of 6 groups that received control DNA or were sham injected. Groups initially contained 10 animals each; mice were excluded from further weight analysis after death.

**Fig. 26**. Adult (22-28 week old) male ferrets were immunized i.m. with 1 mg of DNA encoding the NP from A/Beijing/89, 1 mg of DNA encoding the M1 from A/Beijing/89, or 1 mg of each DNA combined, on days 0 and 42. Control ferrets received noncoding DNA or a full human dose (15 μg/strain) of the licensed whole virus influenza vaccine (92-93 formulation) containing A/Beijing/89 on days 0 and 42. Ferrets were challenged with A/Georgia/93 on day 56. Viral shedding in nasal washes was determined as described above. Viral shedding on days 3-5 was compared with shedding in ferrets given control DNA by a two-way analysis of variance. Shedding in ferrets given NP DNA, M1 DNA, or NP + M1 DNA was significantly lower ($p<0.0001$, 0.0016, and <0.0001, respectively) than shedding in control ferrets. Shedding in ferrets given NP (data not shown), M1, or NP + M1 was not significantly different from shedding in ferrets given the licensed vaccine ($p = 0.104$, 0.533, and 0.145, respectively). The immunization dose of 1 mg was chosen arbitrarily; dose-ranging studies were conducted in nonhuman primates.

**Fig. 27**. Groups of 8 male 22-25 week old ferrets were immunized intramuscularly with control DNA, saline, or DNA encoding influenza A/PR/8/34 proteins on days 0,21, and 121, and were challenged intranasally with 200 TCID50 of A/PR/8/34 on day 148. Immunized animals received 1 mg of NP DNA, or 2 mg of NP, NS1, PB1, PB2, and M1 DNA combined (400 μg each construct). Controls received 0.5 ml/leg of saline or 1 mg of control DNA. For purposes of analysis, the groups that received saline and control DNA were combined (Control), as were the groups that received NP DNA alone or in combination with other internal protein genes (Internal). The graph shows the nasal wash infectivity titers in $TCID_{50}$ per 50 μl of a 3 ml volume of nasal wash fluid. Undiluted wash fluid (the lowest dilution tested) was assumed to be a 1:10 dilution of the original nasal exudate and a positive undiluted sample was assigned a value of 1 log. Titers above 1 log were assigned on the basis of Reed-Muench interpolation among three replicates to yield a 50% infectivity endpoint. Samples that were negative when tested undiluted were assigned a value of 0 logs. Values for p shown on the graph are computed for immunized ferrets vs controls on the indicated days by the T-test for two means. Values for p for the entire curves were computed by two-way analysis of variance and were <0.0001 for NP vs control and <0.001 for the combined DNAs vs control.

**Fig. 28**. Survival of mice immunized with DNA and then challenged with influenza virus. Mice were injected i.m. with 200 μg of DNA encoding the HA from A/PR/34 or control (noncoding) DNA, three times at three week intervals. Three weeks after the final immunization mice were given total respiratory tract challenge (by intranasal instillation under anesthesia) with 1000 TCID50 of A/PR/34. Data are plotted as % survival versus time after challenge ($n = 9$ or 10 mice per group).

**Fig. 29**. Weight loss in mice immunized with DNA and then challenged with influenza virus. Mice were injected i.m. with 200 μg of DNA encoding the HA from A/PR/34 or control (noncoding) DNA, three times at three week intervals. Three weeks after the final immunization mice were given total respiratory tract challenge (by intranasal instillation under anesthesia) with 1000 TCID50 of A/PR/34. Data are plotted as % of initial weight for each individual animal, averaged for each group, versus time. (Dead animals are excluded from the mean.)

**Fig. 30**. Survival of mice immunized with DNA and then challenged with influenza virus. Mice were injected i.m. with 1, 10, or 100 µg of DNA encoding the HA from A/PR/34 or control (noncoding) DNA, three times at three week intervals. Three weeks after the final immunization mice were given total respiratory tract challenge (by intranasal instillation under anesthesia) with 1000 $TCID_{50}$ of A/PR/34. Data are plotted as % survival versus time after challenge (n = 9 or 10 mice per group).

**Fig. 31**. Groups of 8 male 22-25 week old ferrets were immunized intramuscularly with control DNA, saline, or DNA encoding influenza A/PR/34 proteins on days 0, 21, and 121, and were challenged intranasally with 200 TCID50 of A/PR/34 on day 148. Immunized animals received 1 mg of HA DNA, or 2 mg of HA, NP, NS1, PB1, PB2, and M1 DNA combined (330 µg each construct). Controls received 0.5 ml/leg of saline or 1 mg of control DNA. For purposes of analysis, the groups that received saline and control DNA were combined (Control), as were the groups that received HA DNA alone or in combination with other internal protein genes (HA, HA + Internal). The graph shows the nasal wash infectivity titers in $TCID_{50}$ per 50 µl of a 3 ml volume of nasal wash fluid. Undiluted wash fluid (the lowest dilution tested) was assumed to be a 1:10 dilution of the original nasal exudate and a positive undiluted sample was assigned a value of 1 log. Titers above 1 log were assigned on the basis of Reed-Muench interpolation among three replicates to yield a 50% infectivity endpoint. Samples that were negative when tested undiluted were assigned a value of 0 logs. Values for p for the entire curves were computed by two-way analysis of variance and were <0.0001 for HA vs control and <0.0001 for the combined DNAs vs control.

**Fig. 32**. Adult (22-28 week old) male ferrets were immunized i.m. with 1 mg of DNA encoding the HA from A/Georgia/93, on days 0 and 42. Control ferrets received noncoding DNA or a full human dose (15 µg/strain) of the licensed whole virus influenza vaccine (92-93 formulation) containing A/Beijing/89 on days 0 and 42. Ferrets were challenged with A/Georgia/93 on day 56. Viral shedding in nasal washes was determined as described above. Viral shedding on days 1-6 was compared with shedding in ferrets given control DNA by a two-way analysis of variance. Shedding in ferrets given HA DNA was significantly lower (p<0.0001) than shedding in control ferrets.

**Fig. 33**. Adult (22-28 week old) male ferrets were immunized i.m. with 1 mg of DNA encoding the HA from A/Hawaii/91 or A/Beijing/89 (data not shown), on days 0 and 42. Control ferrets received noncoding DNA or a full human dose (15 µg/strain) of the licensed whole virus influenza vaccine (92-93 formulation) containing A/Beijing/89 on days 0 and 42. Ferrets were challenged with A/Georgia/93 on day 56. Viral shedding in nasal washes was determined as described above. Viral shedding on days 1-6 was compared with shedding in ferrets given control DNA by a two-way analysis of vanance. Shedding in ferrets given A/Hawaii/91 HA DNA was significantly lower (p<0.0001) than shedding in control ferrets. Shedding in ferrets given A/Hawaii/91 HA DNA was significantly less than shedding in ferrets given licensed product (p = 0.021 for A/Hawaii/91 HA DNA; two-way ANOVA for days 1-6); shedding in ferrets given A/Beijing/89 HA DNA (data not shown) was not significantly different from shedding in ferrets given licensed product (p = 0.058; two-way ANOVA for days 1-6).

**Fig. 34**. Adult (22-28 week old) male ferrets were immunized i.m. with 1 mg of DNA encoding the HA from A/Hawaii/91 (see Figure 13), or with 330 µg each of DNAs encoding the HA from A/Hawaii/91, and the NP and M1 from A/Beijing/89, on days 0 and 42. Control ferrets received noncoding DNA or a full human dose (15 µg/strain) of the licensed whole virus influenza vaccine (92-93 formulation) containing A/Beijing/89 on days 0 and 42. Ferrets were challenged with A/Georgia/93 on day 56. Viral shedding in nasal washes was determined as described above. Viral shedding on days 1-6 was compared with shedding in ferrets given control DNA by a two-way analysis of variance. Shedding in ferrets given HA + NP + M1 DNA was significantly lower than shedding in ferrets given licensed vaccine (p<0.0001) or HA DNA alone (p = 0.0053).

**Fig. 35**. Adult (22-28 week old) male ferrets were immunized i.m. with 1 mg of DNA encoding the HA from A/Georgia/93, or with 330 µg each of DNAs encoding the HA from A/Hawaii/91, and the NP and M1 from A/Beijing/89, on days 0 and 42. Control ferrets received noncoding DNA or a full human dose (15 µg/strain) of the licensed whole virus influenza vaccine (92-93 formulation) containing A/Beijing/89 on days 0 and 42. Ferrets were challenged with A/Georgia/93 on day 56. Viral shedding in nasal washes was determined as described above. Viral shedding on days 1-6 was compared with shedding in ferrets given control DNA by a two-way analysis of variance. Shedding in ferrets given HA + NP + M1 DNA was not significantly different from virus shedding in ferrets given the homologous HA DNA (p = 0.064).

## DETAILED DESCRIPTION OF THE INVENTION

This invention provides nucleic acid pharmaceuticals which, when directly introduced into an animal, including vertebrates, such as mammals and humans, induce the expression of encoded proteins within the animal. Where the protein is one which does not normally occur in that animal except in pathological conditions, such as proteins associated with influenza virus, for example but not limited to the influenza nucleoprotein, neuraminidase, hemagglutinin, polymerase, matrix or nonstructural proteins, the animals' immune system is activated to launch a protective response. Because these exogenous proteins are produced by the animals' own tissues, the expressed proteins are processed and presented by the major histocompatibility complex, MHC. This recognition is analogous to that which occurs upon actual infection with the related organism. The result, as shown in this disclosure, is induction of immune responses which protect against virulent infection.

This invention provides nucleic acids which, when introduced into animal tissues in vivo, by injection, inhalation, or impression by an analogous mechanism (see BACKGROUND OF THE INVENTION above), the expression of the human influenza virus gene product occurs. Thus, for example, injection of DNA constructs of this invention into the muscle of mice, induces expression of the encoded gene products. Likewise, in ferrets and rhesus monkeys. Upon subsequent challenge with virulent influenza virus, using doses which uniformly kill control animals, animals injected with the polynucleotide vaccine exhibit much reduced morbidity and mortality. Thus, this invention discloses a vaccine useful in humans to prevent influenza virus infections.

We have shown that DNA constructs encoding influenza viral proteins elicit protective immune responses in animals. As will be described in more detail below, immune responses in animals have included antibody and CTL generation in mice, antibody generation in ferrets and primates, and protection from viral challenge in mice and ferrets with homologous, drifted and shifted strains of influenza. Perhaps the most striking result of immunization with DNA encoding viral proteins was the ability to confer protection against distinct subtypes of virus. This suggests that adding a CTL-eliciting component to a vaccine should serve to mitigate the impact of new variants which arise in mid-season or are unanticipated when the vaccine strains are chosen each year for the following year. Importantly, immunization with cDNA vectors encoding an HA, NP and M1 gene was able to protect more effectively against a drifted strain of virus in ferrets than was the licensed vaccine. This provides a justification for the use of constructs encoding internal genes in the PNV.

In one embodiment, the vaccine product will consist of separate DNA plasmids encoding, for example, HA from the 3 prevalent clinical strains representing A/H1N1 (A/Texas/91), A/H3N2 (A/Georgia/93), and B (B/Panama/90) viruses as well as DNA constructs encoding the internal conserved proteins NP and M1 (matrix) from both A (Beijing/89; H3N2) and B strains in order to provide group-common protection against drifted and shifted antigens. The HA DNAs will function by generating HA and resulting neutralizing antibodies against HA. This will be type-specific, with some increased breadth of protection against a drifted strain compared to the current licensed, protein-based vaccine. The NP and M1 constructs will result in the generation of CTL which will provide cross-strain protection with potentially lower viral loads and with acceleration of recovery from illness. The expected persistence of the DNA constructs (in an episomal, non-replicating, non-integrated form in the muscle cells) is expected to provide an increased duration of protection compared to the current vaccine.

The anticipated advantages over the current, licensed vaccines include: increased breadth of protection due to CTL responses ± increased breadth of antibody, and increased duration of protection. The PNV approach avoids the need to make, select and propagate reassortants as is done for the current licensed vaccine since a new DNA construct can be made more directly from a clinical field isolate.

In one embodiment of the invention, the human influenza virus nucleoprotein, NP, sequence, obtained from the A/PR/8/34 strain, is cloned into an expression vector. The vector contains a promoter for RNA polymerase transcription, and a transcriptional terminator at the end of the NP coding sequence. In one preferred embodiment, the promoter is the Rous sarcoma virus (RSV) long terminal repeat (LTR) which is a strong transcriptional promoter. A more preferred promoter is the cytomegalovirus promoter with the intron A sequence (CMV-intA). A preferred transcriptional terminator is the bovine growth hormone terminator. The combination of CMVintA-BGH terminator is particularly preferred. In addition, to assist in preparation of the pharmaceutical, an antibiotic resistance marker is also preferably included in the expression vector. Ampicillin resistence genes, neomycin resistance genes or any other pharmaceutically acceptable antibiotic resistance marker may be used. In a preferred embodiment of this invention, the antibiotic resistence gene encodes a gene product for neomycin resistence. Further, to aid in the high level production of the pharmaceutical by fermentation in prokaryotic organisms, it is advantageous for the vector to contain an origin of replication and be of high copy number. Any of a number of commercially available prokaryotic cloning vectors provide these benefits. In a preferred embodiment of this invention, these functionalities are provided by the commercially available vectors known as pUC. It is desirable to remove non-essential DNA sequences. Thus, the lacZ and lacI coding sequences of pUC are removed in one embodiment of the invention.

In one embodiment, the expression vector pnRSV is used, wherein the rous sarcoma virus (RSV) long terminal repeat (LTR) is used as the promoter. In another embodiment, V1, a mutated pBR322 vector into which the CMV promoter and the BGH transcriptional terminator were cloned is used. The V1-NP construct was used to immunize mice and induce CTLs which protect against heterologous challenge. In a particularly preferred embodiment of this invention, the elements of V1 have been been combined to produce an expression vector named V1J. Into V1J is cloned an influenza virus gene, such as an A/PR/8/34 NP, PB1, NS1, HA, PB2, or M1 gene. In yet another emobodiment, the ampicillin resistance gene is removed from V1J and replaced with a neomycin resistance gene, to generate V1J-neo (SEQ.ID:18:, Figure 7), into which any of a number of different influenza virus genes have been cloned for use according to this invention. In yet another embodiment, the vector is V1Jns, which is the same as V1J except that a unique Sfi1 restriction site has been engineered into the single Kpn1 site at position 2114 of V1J-neo. The incidence of Sfi1 sites in human genomic DNA is very low (aproximately 1 site per 100,000 bases). Thus, this vector allows careful monitoring for expression vector integration into host DNA, simply by Sfi1 digestion of extracted genomic DNA. In a futher refinement, the vector is V1R. In this vector, as much non-essential DNA as possible was "trimmed" from the vector to produce a highly compact vector. This vector is a derivative of V1Jns and is shown in Figure 36, (SEQ.ID.:45:). This vector allows larger inserts to be used, with less concern that undesirable sequences are encoded and optimizes uptake by cells when the construct encoding specific influenza virus genes is introduced into surrounding tissue. In figure 36, the portions of V1Jneo (Figure 7) that are deleted are shown as a gap, and inserted sequence is in bold text, but the numbering of V1Jneo is unchanged. The foregoing vector modification and development proceedures may be accomplished according to methods known by those skilled in the art. The particular products described however, though obtained by conventional means, are epecially useful for the particular purpose to which they are adapted.

While one embodiment of this invention incorporates the influenza NP gene from the A/PR/8/34 strain, more preferred embodiments incorporate an NP gene, an HA gene, an NA gene, a PB gene, a M gene, or an NS gene from more recent influenza virus isolates. This is accomplished by preparing DNA copies of the viral genes and then subcloning the individual genes. Sequences for many genes of many influenza virus strains are now publicly available on GENBANK (about 509 such sequences for influenza A genes). Thus, any of these genes, cloned from the recent Texas, Beijing or Panama isolates of the virus, which are strains recommended by the Center for Disease Control as being desirable in anti-influenza vaccines, are preferred in this invention (see FLU-IMMUNE® influenza virus vaccine of Lederle, Physicians Desk Reference, 1993, p1232, a trivalent purified influenza surface antigen vaccine containing the hemagglutinin protein from A/Texas/36/91, H1N1; A/Beijing/353/89, H3N2; and B/Panama/45/90). To keep the terminology consistent, the following convention is followed herein for describing DNA constructs: "Vector name-flu strain-gene". Thus, a construct wherein the NP gene of the A/PR/8/34 strain is cloned into the expression vector V1Jneo, the name it is given herein is: "V1Jneo-PR-NP". Naturally, as the etiologic strain of the virus changes, the precise gene which is optimal for incorporation in the pharmaceutical may change. However, as is demonstrated below, because cytotoxic lymphocyte responses are induced which are capable of protecting against heterologous strains, the strain variability is less critical in the novel vaccines of this invention, as compared with the whole virus or subunit polypeptide based vaccines. In addition, because the pharmaceutical is easily manipulated to insert a new gene, this is an adjustment which is easily made by the standard techniques of molecular biology.

Because the sequence of nucleoprotein is conserved among various strains of influenza, protection was achieved against subsequent challenge by a virulent strain of influenza A that was heterologous to the strain from which the gene for nucleoprotein was cloned. Comparisons of NP from numerous strains of influenza A have shown no significant differences in secondary structure [M. Gammelin *et al.*, Virol. **170**, 71, 1989] and very few changes in amino acid sequence [O. T. Gorman *et al* ., J. Virol. **65**, 3704, 1991]. Over an approximately 50 year period, NP in human strains evolved at a rate of only 0.66 amino acid changes per year. Moreover, our results which show that A/HK/68-specific CTLs recognize target cells pulsed with the synthetic peptide NP(147-155) derived from the sequence of A/PR8/34 NP indicate that this H-2$K^d$-restricted CTL epitope has remained functionally intact over a 34 year span (see Figure 2). It should be noted also that where the gene encodes a viral surface antigen, such as hemagglutinin or even neurammidase, a significant neutralizing humoral (antibody) immune response is generated in addition to the very important cytotoxic lymphocyte response.

The i.m. injection of a DNA expression vector encoding a conserved, internal protein of influenza A resulted in the generation of significant protective immunity against subsequent viral challenge. In particular, NP-specific antibodies and primary CTLs were produced. NP DNA immunization resulted in decreased viral lung titers, inhibition of weight loss, and increased survival, compared to controls. The protective immune response was not mediated by the NP-specific antibodies, as demonstrated by the lack of effect of NP antibodies alone (see Example 4) in combating a virus infection, and was thus likely due to NP-specific cellular immunity. Moreover, significant levels of primary CTLs directed against NP were generated. The protection was against a virulent strain of influenza A that was heterologous to the strain from which the DNA was cloned. Additionally, the challenge strain arose more than three decades after the A/PR/8/34 strain, indicating that immune responses directed against conserved proteins can be effective despite the antigenic shift and drift of the variable envelope proteins. Because each of the influenza virus gene products exhibit some degree of conservation, and because CTLs may be generated in response to intracellular expression and MHC processing, it is predictable that other influenza virus genes will give rise to responses analogous to that achieved for NP. Methods for identifying immunogenic epitopes are now well known in the art [see for example Shirai et al., J. Immunol 148:1657-1667, 1992; Choppin et al., J. Immunol 147:575-583, 1991; Calin-Laurens, et al., Vaccine 11:974-978, 1993]. Thus, many of these genes have been cloned, as shown by the cloned and sequenced junctions in the expression vector (see below) such that these constructs are prophylactic agents in available form.

Therefore, this invention provides expression vectors encoding an influenza viral protein as an immunogen. The invention offers a means to induce cross-strain protective immunity without the need for self-replicating agents or adjuvants. In addition, immunization with DNA offers a number of other advantages. First, this approach to vaccination should be applicable to tumors as well as infectious agents, since the $CD8^+$ CTL response is important for both pathophysiological processes [K. Tanaka *et al.*, Annu. Rev. Immunol. **6**, 359 (1988)]. Therefore, eliciting an immune response against a protein crucial to the transformation process may be an effective means of cancer protection or immunotherapy. Second, the generation of high titer antibodies against expressed proteins after injection of viral protein (NP and hemagglutinin) and human growth hormone DNA, [see for example D.-c. Tang *et al.*, Nature **356**, 152, 1992], indicates this is a facile and highly effective means of making antibody-based vaccines, either separately or in combination with cytotoxic T-lymphocyte vaccines targeted towards conserved antigens.

The ease of producing and purifying DNA constructs compares favorably with traditional protein purification, facilitating the generation of combination vaccines. Thus, multiple constructs, for example encoding NP, HA, M1, PB1, NS1, or any other influenza virus gene may be prepared, mixed and co-administered. Finally, because protein expression is maintained following DNA injection [H. Lin *et al.*, Circulation **82**, 2217 (1990); R.N. Kitsis *et al.*, Proc. Natl. Acad. Sci. (USA) **88**, 4138 (1991); E. Hansen *et al.*, FEBS Lett. **290**, 73 (1991); S. Jiao *et al.*, Hum. Gene Therapy **3**, 21 (1992); J.A. Wolff *et al.*, Human Mol. Genet. **1**, 363 (1992)], the persistence of B- and T-cell memory may be enhanced [D. Gray and P. Matzinger, J. Exp. Med. **174**, 969 (1991); S. Oehen *et al.*, *ibid.* **176**, 1273 (1992)], thereby engendering long-lived humoral and cell-mediated immunity.

The current limitations of licensed influenza vaccines emphasize the need for development of more effective means for prevention of infection and amelioration of disease. The older vaccines provide limited protection, are effective against only a few, selected strains of virus, and wane in their efficacy after a short period. Thus, the current vaccines must be reformulated for yearly inoculation in order to be effective. Generation of an improved CTL response against internal proteins would likely provide significant long-term, cross-reactive immunity not now elicited by licensed vaccine.

We have demonstrated protein expression from PNV constructs in mice, ferrets, and non-human primates by detection of host immune response directed against influenza antigens. Injection of mice with DNA encoding influenza NP has resulted in increased survival, decreased viral lung titers and less weight loss in comparison with control animals following challenge with influenza subtypes (shifted strains) different from that included in the DNA constructs. We have also observed decreased viral shedding following challenge with shifted strains in ferrets inoculated with NP DNA. These results indicate that protection against a major shift in influenza strains is aided by a DNA vaccine that includes genes encoding NP. Injection with HA DNA followed by challenge of experimental animals with drifted virus strains resulted in an even more substantial decrease in virus shedding. The addition of the internal protein DNA slightly augmented the high degree of protection observed after injection of HA DNA alone.

The immune response to influenza DNA has been followed in mice for as long as six months after injection, with persistence of antibodies, CTL activity, and *in vivo* protection. Repeat injection of DNA further increased survival following challenge at 25 weeks with an influenza strain of different subtype and indicated an ability to boost protective cell-mediated immunity. Antibody persistence has also been documented for at least one year following two injections of HA DNA, with persistence for at least nine months following a single injection of HA DNA in African green monkeys.

The results of these animal experiments indicate that direct DNA injection provides an improved method for protection of humans against influenza infection and disease. Of note, experimental protection by DNA injection was achieved through vaccination of unprimed mice and ferrets. Adult humans vaccinated with DNA will have previously been exposed to influenza. These persons will demonstrate an even more substantial immune response, possibly of increased duration, following immunization with DNA constructs.

A range of doses is compared for immunogenicity in order to optimize concentrations for use. In small mammal experiments, as little as 1 ug of NP DNA induced antibody and CTL responses. Immunization of Rhesus monkeys demonstrated antibody response in 2 of 2 animals with doses of 100 and 1000 ug of HA DNA (A/PR/08/34), while 1 of 2 animals responded to a single 10 ug injection. In separate experiments, naive African Green monkeys were injected with a mixture of five different DNA constructs encoding HA from three virus subtypes as well as DNA encoding NP and M1 from influenza A virus. Three of three monkeys in each group responded to vaccines which included 10 ug or 100 ug of each of the five constructs. Based on these findings, it is predictable that dosages of 10, 50, 100, and 200 ug of DNA are efficacious in man.

Prevention of infection by licensed, inactivated, vaccine correlates with serum and mucosal antibody levels directed against HA but is not correlated with antibody responses to internal influenza proteins. Thus, HA must be included in the development of the influenza DNA vaccine. However, immune response to NP enhances antibody response to HA, and influenza internal proteins provide a CTL response cross-reactive with antigenically diverse strains of influenza. As noted above, animal experimentation has also indicated improved immunogenicity and protection when injections included DNA constructs encoding internal proteins as well as HA. Inclusion of DNA constructs encoding internal proteins would likely enhance the efficacy of the DNA vaccine in humans. Since dosage levels are likely to be dependent upon interactions of these components, routine testing will allow one skilled in the art to determine the amount of DNA in the vaccine to make a mixture of HA, NP and M1 DNA constructs. Host response to each of these components can be measured separately, with comparisons of hemagglutinin inhibition (HI) titers and neutralizing against the HA components and CTL responses against M1 and NP epitopes. Results are compared with antibody responses following injection of constructs which express only HA. These studies allow evaluation of the potential enhanced response to a vaccine containing DNA encoding HA as well as internal proteins.

Human efficacy is shown in volunteers who receive influenza DNA vaccine, followed by an intranasal challenge in order to show vaccine efficacy against similar virus strains as well as influenza strains of different subtype. The composition, dosage and administration regimens for the vaccine are based on the foregoing studies. Clinical efficacy is shown by infection rate, illness scores, and duration of illness. These clinical findings are compared with laboratory evaluation of host immune response and viral shedding in order to determine surrogate markers which correlate with protection.

The standard techniques of molecular biology for preparing and purifying DNA constructs enable the preparation of the DNA therapeutics of this invention. While standard techniques of molecular biology are therefore sufficient for the production of the products of this invention, the specific constructs disclosed herein provide novel therapeutics which surprisingly produce cross-strain protection, a result heretofore unattainable with standard inactivated whole virus or subunit protein vaccines.

The amount of expressible DNA to be introduced to a vaccine recipient will depend on the strength of the transcriptional and translational promoters used in the DNA construct, and on the immunogenicity of the expressed gene product. In general, an immunologically or prophylactically effective dose of about 1 $\mu$g to 1 mg, and preferably about 10 $\mu$g to 300 $\mu$g is administered directly into muscle tissue. Subcutaneous injection, intradermal introduction, impression through the skin, and other modes of administration such as intraperitoneal, intravenous, or inhalation delivery are also contemplated. It is also contemplated that booster vaccinations are to be provided.

The DNA may be naked, that is, unassociated with any proteins, adjuvants or other agents which impact on the recipients immune sytem. In this case, it is desirable for the DNA to be in a physiologically acceptable solution, such as, but not limited to, sterile saline or sterile buffered saline. Alternatively, the DNA may be associated with liposomes, such as lecithin liposomes or other liposomes known in the art, as a DNA-liposome mixture, (see for example WO9324640) or the DNA may be associated with an adjuvant known in the art to boost immune responses, such as a protein or other carrier. Agents which assist in the cellular uptake of DNA, such as, but not limited to, calcium ions, viral proteins and other transfection facilitating agents may also be used to advantage. These agents are generally referred to as transfection facilitating agents and as pharmaceutically acceptable carriers. As used herein, the term gene refers to a segment of nucleic acid which encodes a discrete polypeptide. The term pharmaceutical, and vaccine are used interchangeably to indicate compositions useful for inducing immune responses. The terms construct, and plasmid are used interchangeably. The term vector is used to indicate a DNA into which genes may be cloned for use according to the method of this invention.

Accordingly, one embodiment of this invention is a method for using influenza virus genes to induce immune responses in vivo, in a vertebrate such as a mammal, including a human, which comprises:

a) isolating the gene,

b) linking the gene to regulatory sequences such that the gene is operatively linked to control sequences which, when introduced into a living tissue direct the transcription initiation and subsequent translation of the gene,

c) introducing the gene into a living tissue, and

d) optionally, boosting with additional influenza gene.

A preferred embodiment of this invention is a method for protecting against heterologous strains of influenza virus. ` This is accomplished by administering an immunologically effective amount of a nucleic acid which encodes a conserved influenza virus epitope. For example, the entire influenza gene for nucleoprotein provides this function, ad it is contemplated that coding sequences for the other influenza genes and portions thereof encoding conserved epitopes within these genes likewise provide cross-strain protection.

In another embodiment of this invention, the DNA vaccine encodes human influenza virus nucleoprotein, hemagglutinin, matrix, nonstructural, or polymerase gene product. Specific examples of this embodiment are provided below wherein the human influenza virus gene encodes the nucleoprotein, basic polymerase1, nonstructural protein1, hemagglutinin, matrix1, basic polymerase2 of human influenza virus isolate A/PR/8/34, the nucleoprotein of human influenza virus isolate A/Beijing/353/89, the hemagglutinin gene of human influenza virus isolate A/Texas/36/91, or the hemagglutinin gene of human influenza virus isolate B/Panama/46/90.

In specific embodiments of this invention, the DNA construct encodes an influenza virus gene, wherein the DNA construct is capable of being expressed upon introduction into animal tissues in vivo and generating an immune response against the expressed product of the encoded influenza gene. Combinations comprising such constructs with polynucleotides encoding other antigens, unrelated to influenza virus, are clearly contemplated by the instant invention. Examples of preferred influenza gene encoding DNA constructs include:

a) pnRSV-PR-NP,

b) V1-PR-NP,

c) V1J-PR-NP, the 5' end of which is SEQ. ID:12:,

d) V1J-PR-PB1, the 5' end of which is SEQ. ID:13:,

e) V1J-PR-NS, the 5' end of which is SEQ. ID:14:,

f) V1J-PR-HA, the 5' end of which is SEQ. ID:15:,

g) V1J-PR-PB2, the 5' end of which is SEQ. ID:16:,

h) V1J-PR-M1, the 5' end of which is SEQ. ID:17:,

i) V1Jneo-BJ-NP, the 5' end of which is SEQ. ID:20: and
the 3' end of which is SEQ. ID:21:,

j) V1Jneo-TX-NP, the 5' end of which is SEQ. ID:24 and
the 3' end of which is SEQ. ID:25: and

k) V1Jneo-PA-HA, the 5' end of which is SEQ. ID:26: and
the 3' end of which is SEQ. ID:27:

l) V1Jns-GA-HA (A/Georgia/03/93), construct size 6.56 Kb,
the 5' end of which is SEQ.ID:46: and
the 3' end of which is SEQ. ID:47:,

m) V1Jns-TX-HA (A/Texas/36/91), construct size 6.56 Kb,
the 5' end of which is SEQ.ID:48: and
the 3' end of which is SEQ. ID:49:,

n) V1Jns-PA-HA (B/Panama/45/90), construct size 6.61 Kb,
the 5' end of which is SEQ.ID:50: and
the 3' end of which is SEQ. ID:51:,

o) V1Jns-BJ-NP (A/Beijing/353/89), construct size 6.42 Kb,
the 5' end of which is SEQ.ID:52: and
the 3' end of which is SEQ. ID:53:,

p) V1Jns-BJ-M1 (A/Beijing/353/89), construct size 5.62 Kb,
the 5' end of which is SEQ.ID:54:, and
the 3' end of which is SEQ. ID:55:,

q) V1Jns-PA-NP (B/Panama/45/90), construct size 6.54 Kb,
the 5' end of which is SEQ.ID:56: and

the 3' end of which is SEQ. ID:57:,
r) V1Jns-PA-M1 (B/Panama/45/90), construct size 5.61 Kb,
the 5' end of which is SEQ.ID:58: and
the 3' end of which is SEQ. ID:59:,.

The following examples are provided to further define the invention, without limiting the invention to the specifics of the examples.

EXAMPLE 1

PREPARATION OF DNA CONSTRUCTS ENCODING HUMAN INFLUENZA VIRUS PROTEINS:

i). pnRSV-PRNP: The A/PR/8/34 NP gene was isolated from pAPR-501 [J.F. Young *et al.*, in *The Origin of Pandemic Influenza Viruses*, W.G. Laver, Ed. (Elsevier Science Publishing Co., Inc., 1983)] as a 1565 base-pair EcoRI fragment, Klenow filled-in and cloned into the Klenow filled-in and phosphatase-treated XbaI site of pRSV-BL. pRSV-BL was constructed by first digesting the pBL-CAT3 [B. Luckow and G. Schutz, Nuc. Acids Res. **15**, 5490 (1987)] vector with Xho I and Nco I to remove the CAT coding sequence and Klenow filled-in and self-ligated. The RSV promoter fragment was isolated as an Nde I and Asp718 fragment from pRshgrnx [V. Giguere et al., Nature **330**, 624 (1987)], Klenow filled-in and cloned into the HindIII site of the intermediate vector generated above (pBL-CAT lacking the CAT sequence).

ii) V1-NP: The expression vector V1 was constructed from pCMVIEAKI-DHFR [Y. Whang *et al.*, J. Virol. **61**, 1796 (1987)]. The AKI and DHFR genes were removed by cutting the vector with EcoR I and self-ligating. This vector does not contain intron A in the CMV promoter, so it was added as a PCR fragment that had a deleted internal Sac I site [at 1855 as numbered in B.S. Chapman *et al.*, Nuc. Acids Res. **19**, 3979 (1991)]. The template used for the PCR reactions was pCMVintA-Lux, made by ligating the Hind III and Nhe I fragment from pCMV6a120 [see B.S. Chapman *et al.*, *ibid.*,] which includes hCMV-IE1 enhancer/promoter and intron A, into the Hind III and Xba I sites of pBL3 to generate pCMVIntBL. The 1881 base pair luciferase gene fragment (Hind III-Sma I Klenow filled-in) from RSV-Lux [J.R. de Wet *et al.*, Mol. Cell Biol. 7, 725, 1987] was cloned into the Sal I site of pCMVIntBL, which was Klenow filled-in and phosphatase treated.

The primers that spanned intron A are:
5' primer, SEQ. ID:5:
5'-CTATATAAGCAGAG CTCGTTTAG-3'.
The 3' primer, SEQ ID:6:
5'-GTAGCAAAGATCTAAGGACGGTGA CTGCAG-3'.
The primers used to remove the Sac I site are:
sense primer, SEQ ID:7:
5-GTATGTGTCTGAAAATGAGCGTGGAGATTGGGCTCGCAC-3'
and the antisense primer, SEQ ID:8:
5'-
GTGCGAGCCCAATCTCCACGCTCATTTTCAGACACA TAC-3'.

The PCR fragment was cut with Sac I and Bgl II and inserted into the vector which had been cut with the same enzymes. The NP gene from Influenza A (A/PR/8/34) was cut out of pAPR501 [J.F. Young *et al.*, in *The Origin of Pandemic Influenza Viruses*, W.G. Laver, Ed. (Elsevier Science Publishing Co., Inc., 1983)] as a 1565 base-pair EcoR I fragment and blunted. It was inserted into V1 at the blunted Bgl II site, to make V1-NP. Plasmids were propagated in E. coli and purified by the alkaline lysis method [J. Sambrook, E.F. Fritsch, and T. Maniatis, in *Molecular Cloning*, *A Laboratory Manual*, second edition (Cold Spring Harbor Laboratory Press, 1989)]. CsCl banded DNA was ethanol precipitated and resuspended in 0.9% saline at 2mg/ml for injection.

EXAMPLE 2

ASSAY FOR HUMAN INFLUENZA VIRUS CYTOTOXIC T-LYMPHOCYTES:

Cytotoxic T lymphocytes were generated from mice that had been immunized with DNA or that had recovered from infection with A/HK/68. Control cultures were derived from mice that had been injected with control DNA and from uninjected mice. Single cell suspensions were prepared, red blood cells were removed by lysis with ammonium chloride, and spleen cells were cultured in RPMI 1640 supplemented with 10% Fetal Bovine Serum (FBS), 100 U/ml penicillin, 100 μg/ml streptomycin, 0.01 M HEPES (pH 7.5), and 2 mM l-glutamine. An equal number of autologous, irradiated stimulator cells, pulsed for 60 min. with the H-$2K^d$-restricted peptide epitope NP147-155 (Thr Tyr Gln Arg Thr Arg Ala Leu Val, SEQ ID:9:) at 10 $\mu$M or infected with influenza A/PR8/34 (H1N1), and 10 U/ml recombinant human IL-2 (Cellular Products, Buffalo, NY) were added and cultures were maintained for 7 days at 37°C with 5% $CO_2$ and 100% relative humidity. In selected experiments, rhIL-2 ( 20 U/ml) and Con A (2 $\mu$g/ml) were added in place of autologous stimulator cells. Cytotoxic T cell effector activity was determined with P815 cells labeled for 3 hr with 60 $\mu$Ci of $^{51}$Cr per $10^6$ cells, and pulsed as above with NP147-155, or infected with influenza A/Victoria/73 (H3N2). Control targets (labeled P815 cells without peptide or virus) were not lysed. Targets were plated at 1 x $10^4$ cells/well in round-bottomed 96-well plates and incubated with effectors for 4 hours in triplicate. Supernatant (30 $\mu$l) was removed from each well and counted in a Betaplate scintillation counter (LKB-Wallac, Turku, Finland). Maximal counts, released by addition of 6M HCl, and spontaneous counts released without CTL were determined for each target preparation. Percent specific lysis was calculated as: [-(experimental - spontaneous)/(maximal - spontaneous)] X 100.

EXAMPLE 3

PRODUCTION OF NP SPECIFIC CTLs AND ANTIBODIES IN VIVO:

BALB/c mice were injected in the quadriceps of both legs with plasmid cDNA encoding A/PR/8/34 nucleoprotein driven by either a Rous sarcoma virus or cytomegalovirus promoter.
Expression vectors used were:
i) pnRSV-PRNP, see Example 1;
ii) V1-NP, see Example 1.
Animals used were female BALB/c mice, obtained from Charles River Laboratories, Raleigh, NC. Mice were obtained at 4-5 weeks of age and were initially injected with DNA at 5-6 weeks of age. Unless otherwise noted, injections of DNA were administered into the quadriceps muscles of both legs, with each leg receiving 50 $\mu$l of sterile saline containing 100 $\mu$g of DNA. Mice received 1, 2 or 3 sets of inoculations at 3 week intervals. Negative control animals were uninjected or injected with the appropriate blank vector lacking the inserted NP gene.

The presence or absence of NP plasmid DNA in the muscles of selected animals was analyzed by PCR (Fig. 1). Plasmid DNA (either NP or luciferase DNA) was detected in 44 of 48 injected muscles tested. In mice injected with luciferase DNA, protein expression was demonstrated by luciferase activity recovered in muscle extracts according to methods known in the art [J.A. Wolff *et al.*, Science **247**, 1465 (1990); G. Ascadi *et al.*, Nature **352**, 815 (1991); H. Lin *et al.*, Circulation **82**, 2217 (1990); R.N. Kitsis *et al.*, Proc. Natl. Acad. Sci. (USA) **88**, 4138 (1991); E. Hansen *et al.*, FEBS Lett. **290**, 73 (1991); S. Jiao *et al.*, Hum. Gene Therapy **3**, 21 (1992); J.A. Wolff *et al.*, Human Mol. Genet. **1**, 363 (1992)].

NP expression in muscles after injection of NP DNA was below the limit of detection for Western blot analysis (< 1 ng) but was indicated by the production of NP-specific antibodies (see Fig. 2). For analysis of NP-specific CTL generation, spleens were removed 1-4 weeks following immunization, and spleen cells were restimulated with recombinant human IL-2 plus autologous spleen cells that had been either infected with influenza A (A/PR/8/34) or pulsed with the H-$2K^d$-restricted nucleoprotein peptide epitope (NP residues 147-155, see O.K. Rötzscke *et al.*, Nature **348**, 252 (1990)). Spleen cells restimulated with virally-infected or with epitope-pulsed syngeneic cells were capable of killing nucleoprotein epitope-pulsed target cells (Fig. 3A). This indicates that i.m. injection of NP DNA generated the appropriate NP-derived peptide in association with MHC class I for induction of the specific CTL response. These CTLs were capable of recognizing and lysing virally infected target cells, (Fig 3B), or target cells pulsed with the H-$2K^d$-restricted nucleoprotein peptide epitope and virally-infected target cells. This demonstrates their specificity as well as their ability to detect the epitope generated naturally in infected cells.

A more stringent measure of immunogenicity of the NP DNA vaccine was the evaluation of the primary CTL response. Spleen cells taken from NP DNA-injected mice were activated by exposure to Con A and IL-2, but did not undergo in vitro restimulation with antigen-expressing cells prior to testing their ability to kill appropriate targets. Splenocytes from mice immunized with NP DNA, when activated with Con A and IL-2 in vitro without antigen-specific restimulation, lysed both epitope-pulsed and virally-infected target cells (Fig. 3C and D). This lytic activity of both the restimulated and activated spleen cells compares favorably with that of similarly treated splenocytes derived from mice that had been previously infected with influenza A/HK/68, a virulent mouse-adapted H3N2 strain that arose 34 years after A/PR/8/34 (H1N1). Thus, injection of NP DNA generated CTL that were specific for the nucleoprotein epitope and that were capable of identifying the naturally processed antigen (*i.e.*, could kill virally-infected cells). NP CTL have also been generated in C3H and B6 transgenic mice expressing human HLA-A2.

NP CTL have been detected in spleens of BALB/c mice injected with as little as 1 dose of 1 μg NP DNA (the lowest dose tested) (Table 3-IV):

Table 3-IV

| CTL Responses in Mice After a Single Injection of NP DNA | | | |
|---|---|---|---|
| inoculum | dose (μg) % | specific lysis | sd |
| NP DNA | 400 | 52.5 | 10.7 |
| | 400 | 80.4 | 10.3 |
| NP DNA | 200 | 75.6 | 5.4 |
| | 200 | 44.6 | 4.4 |
| NP DNA | 100 | 76.7 | 2.9 |
| | 100 | 35.6 | 8.9 |
| NP DNA | 50 | 62.9 | 0.6 |
| | 50 | 76.7 | 7.4 |
| NP DNA | 10 | 83.2 | 10.1 |
| | 10 | 37.7 | 2.5 |
| NP DNA | 1 | 44.2 | 0.3 |
| | 1 | 13 | 2 |
| Control DNA | 100 | 4.9 | 1 |
| Flu | | 79.3 | 8.3 |

Table 3-IV: Female BALB/c mice (4-6 weeks) were injected with a single dose of A/PR/34 NP DNA (V1JNP) or control DNA (V1J) at the indicated doses. For comparison, mice were infected with influenza virus A/PR/34. CTL were obtained after 8 weeks, restimulated *in vitro* with NP peptide-pulsed syngeneic spleen cells, and assayed against NP peptide-pulsed P815 cells at an effector:target ratio of 50:1. Data is represented as % specific lysis for representative individual mice.

Followup experiments have shown that mice receiving 1 dose of 1 μg NP DNA maintained NP CTL for at least 4.5 months (latest time point tested). The magnitude of the CTL responses after DNA injection were comparable to that in influenza-infected mice. However, it should be noted that analysis of CTL after antigen restimulation *in vitro* is not strictly quantitative. Therefore, we are currently developing limiting dilution assays to more quantitatively assess the levels of NP-specific CTL in mice. In mice that were injected with 3 doses of 100 μg NP DNA, CTL responses have been detected at least 6 months after immunization (Figure 19). Therefore, an influenza PNV has the potential to generate long-lived CTL responses directed toward conserved influenza antigens.

Injection of mice with NP DNA resulted in the production of high titer anti-NP IgG antibodies (Fig. 2). Generation of high titer IgG antibodies in mice is thought to require $CD4^+$ T cell help (P. Vieira and K. Rajewsky, Int. Immunol. **2**, 487 (1990); J. J. Donnelly *et al.*, J. Immunol. **145**, 3071 (1990)). This shows that NP expressed from the plasmid in situ was processed for presentation by both MHC class I and class II.

EXAMPLE 4

PROTECTION OF MICE UPON CHALLENGE WITH VIRULENT HUMAN INFLUENZA VIRUS:

The role of NP antibodies in protective immunity to influenza is shown by two approaches: First, viral lung titers were determined in a passive-transfer experiment. Female BALB/c mice ≧ 10 weeks of age were injected intraperitoneally with 0.5 ml of pooled serum (diluted in 2.0 ml of PBS) from mice that had been injected 3 times with 200 μg of NP DNA. Control mice were injected with an equal volume of pooled normal mouse serum, or with pooled serum from mice that had recovered from infection with A/HK/68, also in 2.0 ml of PBS. The dose of A/HK/68 immune serum was adjusted such that the ELISA titer of anti-NP antibody was equal to that in the pooled serum from NP DNA-injected mice. Mice were challenged unanesthetized in a blinded fashion with $10^4$ $TCID_{50}$ of A/HK/68 2 hours after serum injection, and a further injection of an equal amount of serum was given 3 days later. Mice were sacrificed 6 and 7 days after infection and viral lung titers in $TCID_{50}$ per ml were determined as described by Moran [J. Immunol. **146**, 321, 1991].

Naive mice were infused with anti-NP antiserum, obtained from mice that were injected with NP DNA, and then challenged with A/HK/68. Viral challenges were performed with a mouse-adapted strain of A/HK/68 and maintained subsequently by *in vivo* passage in mice (Dr. I. Mbawuike, personal communication). The viral seed stock used was a homogenate of lungs from infected mice and had an infectivity titer of 5 x $10^8$ $TCID_{50}$/ml on MDCK cells. For viral lung titer determinations and weight loss studies, viral challenges were performed in blinded fashion by intranasal instillation of 20 μl containing $10^4$ $TCID_{50}$ onto the nares of unanesthetized mice, which leads to progressive infection of the lungs with virus but is not lethal in BALB/c mice [Yetter, R.A. *et al.*, Infect. Immunity **29**, 654, 1980]. In survival experiments, mice were challenged by instillation of 20 μl containing $10^{2.5}$ $TCID_{50}$ onto the nares under full anesthesia with ketamine and xylazine; infection of anesthetized mice with this dose causes a rapid lung infection which is lethal to 90-100% of nonimmunized mice [J.L. Schulman and E.D. Kilbourne, J. Exp. Med. **118**, 257, 1963; G.H. Scott and R.J. Sydiskis, Infect. Immunity **14**, 696, 1976; R.A. Yetter *et al.*, Infect. Immunity **29**, 654, 1980]. Viral lung titers were determined by serial titration on MDCK cells (obtained from ATCC, Rockville, MD) in 96-well plates as described by Moran *et al.*, [*ibid.*].

No reduction in viral lung titers was seen in mice that had received anti-NP antiserum (6.3 ± 0.2; mean ± SEM; n = 4) as compared to control mice that had received normal serum (6.1 ± 0.3; mean ± SEM; n = 4). As a positive control, serum was collected from mice that had been infected with A/HK/68 and passively transferred to four naive mice. After a challenge with A/HK/68, no viral infection was detectable in their lungs, indicating that this serum against whole virus was completely protective for challenge with the homologous virus. Second, naive mice were immunized with purified NP (5 μg/leg, 3 times over a period of 6 weeks) by i.m. injection. These mice generated high titer NP-specific antibodies but failed to produce NP-specific CTLs and were not protected from a lethal dose of virus. Therefore, unlike the neutralizing effect of antibodies to whole virus, circulating anti-NP IgG did not confer protective immunity to the mice.

The in vivo protective efficacy of NP DNA injections was evaluated to determine whether a cell-mediated immune response was functionally significant. One direct measure of the effectiveness of the immune response was the ability of mice first immunized with NP DNA to clear a progressive, sublethal lung infection with a heterologous strain of influenza (A/HK/68; H3N2). Viral challenges were conducted as described above. Mice immunized with NP DNA had viral lung titers after challenge that were three orders of magnitude lower on day 7 (1.0 ± 1.0; mean ± SEM; n = 4) than those of control mice that had not been immunized (4.1 ± 0.3; mean ± SEM; n = 4), or that had been immunized with blank vector (4.5 ± 0.0; mean ± SEM; n = 4). In fact, three of four immunized mice had undetectable levels of virus in their lungs, while none of the controls had cleared virus at this point. The substantial difference in the viral lung titers seen in this experiment and six others demonstrates that the immune response accelerated clearance of the virus. The lack of protective effect of the blank vector control confirms that DNA per se was not responsible for the immune response. Moreover, because the challenge strain of virus, A/HK/68 (a virulent, mouse-adapted H3N2 strain), was heterologous to the strain A/PR8/34 (H1N1) from which the NP gene was cloned, the immunity was clearly heterotypic.

As a measure of virus-induced morbidity, the mass loss was monitored in mice that were infected sublethally with influenza A/HK/68 following immunization with NP DNA (Fig. 4). Uninjected mice or mice injected with the blank vector were used as controls. Mice immunized with NP DNA exhibited less weight loss and a more rapid return to their pre-challenge weights following influenza A infection compared to control mice.

Intranasal infection of fully anesthetized mice with influenza A causes rapid widespread viral replication in the lung and death in 6-8 days if the infection is not controlled (R.A. Yetter *et al.*, Infect. Immunity **29**, 654 (1980)). Survival of mice challenged by this method reflects their ability to limit the severity of an acute lung infection. The capacity of mice to survive challenge with two different strains of influenza, A/HK/68 (see Fig. 5) and A/PR/8/34, was studied. Mice previously immunized with NP DNA showed a 90% survival rate compared to 0% in blank vector injected and 20% in uninjected control animals (Fig. 5). In a total of 14 such studies, mice immunized with NP DNA showed at least a 50% greater survival rate than controls. Thus, the ability of the NP DNA-induced immune response to effectively accelerate recovery and decrease disease caused by a virus of a different strain arising 34 years later supports the rationale of targeting a conserved protein for the generation of a cytotoxic T-lymphocyte response.

EXAMPLE 5

ISOLATION OF GENES FROM INFLUENZA VIRUS ISOLATES:

Many of the older influenza virus strains are on deposit with the ATCC (the 1990 Catalogue of Animal Viruses & Antisera, Chlamydiae & Rickettsiae, 6th edition, lists 20 influenza A strains and 14 influenza B strains.

A. Viral Strains and Purification:

Influenza strains which comprise the current, 1992 flu season vaccine were obtained from Dr. Nancy J. Cox at the Division of Viral and Rickettsial Diseases, Centers of Disease Control, Atlanta, GA. These strains are: (1) A/Beijing/353/89 (H3N2); (2) A/Texas/36/91 (H1N1); (3) B/Panama/45/90; and (4) A/Georgia/03/93.

All of these viruses were grown by passage in 9- to 11-day-old embryonated chicken eggs (except A/Georgia which was grown in MDCK cells), (100-200 per viral preparation) and purified by a modification of the method described by Massicot et al. (Virology 101, 242-249 (1980)). In brief, virus suspensions were clarified by centrifugation at 8000 rpm (Sorvall RC5C centrifuge, GS-3 rotor) and then pelleted by centrifugation at 18,000 rpm for 2 h in a Beckman Type 19 rotor. The pelleted virus was resuspended in STE (0.1 M NaCl, 20 mM Tris, pH 7.4, 1 mM EDTA) and centrifuged at 4,000 rpm for 10 min (Hermle Z 360 K centrifuge) to remove aggregates. 2 ml of supernatant was layered onto a discontinuous sucrose gradient consisting of 2 ml of 60% sucrose overlayed with 7 ml of 30% sucrose buffered with STE and centrifuged at 36,000 rpm (SW-40 rotor, Beckman) for 90 minutes. Banded virus was collected at the interface, diluted 10-fold with STE, and pelleted at 30,000 rpm for 2 h (Beckman Ti45 rotor). The pelleted virus was then frozen at -70°C.

B. Extraction of Viral RNA and cDNA Synthesis:

Viral RNA was purified from frozen virus by guanidinium isothiocyanate extraction using a commercially available kit (Stratagene, La Jolla, CA) employing the method of Chomczynski and Sacchi (Anal. Biochem. 162, 156-159 (1987)). Double-stranded cDNA was prepared from viral RNA using a commercially available cDNA synthesis kit (Pharmacia) as directed by the manufacturers with several modifications. The first strand of cDNA was pruned using a synthetic oligodeoxyribonucleotide, 5'-AGCAAAAGCAGG-3', SEQ. ID:30:, which is complementary to a conserved sequence located at the 3'-terminus of the viral RNA for all A strain genes. This sequence is common to all type A influenza viral RNAs and therefore provides a method for cloning any A strain influenza virus gene. After synthesis of first and second strands of cDNA the reactions were extracted with phenol/chloroform and ethanol precipitated rather than continuing with the kit directions. These blunt-ended cDNA's were then directly ligated into the V1Jneo or V1Jns vector which had been digested with the BglII restriction enzyme, blunt-ended with T4 DNA polymerase, and treated with calf intestinal alkaline phosphatase.

To screen for particular full-length viral genes we used synthetic oligodeoxyribonucleotides which were designed to complement the 3'-terminus of the end of the translational open reading frame of a given viral gene. Samples which appeared to represent full-length genes by restriction mapping and size determination on agarose electrophoresis gels were verified by dideoxynucleotide sequencing of both junctions of the viral gene with V1Jneo. The sequence junctions for each gene cloned from these viruses is given below in Example 8.

Similar strategies were used for cloning cDNA's from each of the viruses named above except that for B/Panama/45/90, which does not have common sequences at each end of viral RNA, a mixture of oligodeoxyribonucleotides were used to prime first strand cDNA synthesis. These primers were:

(1) 5'-AGCAGAAGCGGAGC-3', SEQ. ID:31: for PB1 and PB2;
(2) 5'-AGCAGAAGCAGAGCA-3', SEQ. ID:19: for NS and HA;
(3) 5'-AGCAGAAGCACGCAC-3', SEQ. ID:22: for M; and
(4) 5'-AGCAGAAGCACAGCA-3', SEQ. ID:23: for NP.

For genes that were cloned by PCR, the blunt-ended cDNA solution was used directly in PCR reactions as the DNA template. The primers used for cloning the 6 influenza genes obtained by PCR are as follows:

<u>1. HA gene from A/Georgia/03/93</u>
sense primer: SEQ.ID:33:
5' GGT ACA ACC ATG AAG ACT ATC ATT GCT TTG AGC 3'
anti-sense primer: SEQ.ID:34:
5' CCA CAT AGA TCT TCA AAT GCA AAT GTT GCA CCT AAT G 3'

<u>2. HA gene from A/Texas/36/91</u>
sense primer: SEQ.ID:35:
5' GGT ACA ACC ATG AAA GCA AAA CTA CTA GTC CTG TTA TG 3'
anti-sense primer: SEQ.ID:36:
5' CCA CAT TCA GAT GCA TAT TCT ACA CTG CAA AG 3'

<u>3. HA gene from B/Panama/45/90</u>
sense primer: SEQ.ID:37:
5' GGT ACA ACC ATG AAG GCA ATA ATT GTA CTA CTC ATG 3'
anti-sense primer: SEQ.ID:38:
5' CCA CAT TTA TAG ACA GAT GGA GCA AGA AAC ATT GTC 3'

<u>4. M1 gene from A/Beijing/353/89</u>
sense primer: SEQ.ID:39:
5' GGT ACA AGA TCT ACC ATG CTT CTA ACC GAG GTC 3'
anti-sense primer: SEQ.ID:40:
5' CCA CAT AGA TCT TCA CTT GAA CCG TTG CAT CTG CAC 3'

<u>5. NP gene from B/Panama/45/90</u>
sense primer: SEQ.ID:41:
5' GGT ACA GGA TCC ACC ATG TCC AAC ATG GAT ATT GAC GGC 3'
anti-sense primer: SEQ.ID:42:
5' CCA CAT GGA TCC TTA ATA ATC GAG GTC ATC ATA ATC CTC 3'

<u>6. M1 gene from B/Panama/45/90</u>
sense primer: SEQ.ID:43:
5' GGT ACA GGA TCC ACC ATG TCG CTG TTT GGA GAC ACA ATT GCC 3'
anti-sense primer: SEQ.ID:44:
5' CCA CAT GGA TCC TTA TAG GTA TTT CTT CAC AAG AGC TG 3'

All influenza gene clones, whether cDNA or PCR generated, were verified by sequencing through the ligation sites into the gene and expressing the gene in transfected RD cells. Expression was detected by immunoblot.

The NP and M1 constructs for the A/H3N2 strain (vectors 4 and 5) were made from the A/Beijing/353/89 genes. These genes were chosen because of the expected high degree of conservation of both NP and M1 genes and because of their availablilty.

From the foregoing work, a particularly preferred group of 7 expression vectors that are combined to form a vaccine include:

1. V1Jns-HA (A/Georgia/03/93) 6.56 Kb

2. V1Jns-HA (A/Texas/36/91) 6.56 Kb
3. V1Jns-HA (B/Panama/45/90) 6.61 Kb
4. V1Jns-NP (A/Beijing/353/89) 6.42 Kb
5. V1Jns-M1 (A/Beijing/353/89) 5.62 Kb
6. V1Jns-NP (B/Panama/45/90) 6.54 Kb
7. V1Jns-M1 (B/Panama/45/90) 5.61 Kb.

The relevant sequences for junctions of these genes in the expression vectors are provided below. Only small portions of the constructs need be sequenced to confirm that the correct gene has been cloned. By comparison with similar known genes, it is easy to confirm that the given gene is an NP gene, an HA gene, an M1 gene etc. For example, the A/Texas HA gene sequence is very similar to the HA gene sequence of A/Kiev/59/79, the sequence of which is available in GENBANK as accession number M38353. Likewise for the B/Panama HA sequence, which is very similar to the B/England/222/82 HA sequence which is available on GENBANK as accession number M18384. In like manner, the identity of any cloned sequence for a given gene from any human influenza virus may be confirmed. In each case below, both a 5' sequence and a 3' sequence was confirmed to ensure that the entire gene was present. In each case, the bolded ATG shows the start codon for the influenza gene, while bolded sequence in the 3' portion is the stop codon:

1. V1Jns-HA (A/Georgia/03/93) 6.56 Kb:

5' Sequence: (SEQ.ID:46:)

...TCA CCG TCC TTA GAT C/GG TAC AAC **CAT G**AA GAC TAT CAT TGC TTT GAG CTA CAT TTT ATG TCT GGT TTT CGC....

3' Sequence: (SEQ.ID:47:)

...TCA TGC TTT TTG CTT TGT GTT GTT TTG CTG GGG TTC ATC ATG TGG GCC TGC CAA AAA GGC AAC ATT AGG TGC AAC ATT TGC ATT **TGA** A/GA TCT ATG TGG GAT CTG CTG TGC...

2. V1Jns-HA (A/Texas/36/91) 6.56 Kb:

5' Sequence: (SEQ.ID:48:)

... TTA GAT C/GG AAC **ATG** AAA GCA AAA CTA CTA GTC CTG TTA TGT GCA TTT ACA GCT ACA TAT GCA ....

3' Sequence: (SEQ.ID:49:)
...CTG GTG CTT TTG GTC TCC CTG GGG GCA ATC AGC TTC TGG ATG TGT TCT AAT GGG TCT TTG CAG TGT AGA ATA TGC ATC **TGA** ATG TGG /GAT CTG CTG TGC CTT....

3. V1Jns-HA (B/Panama/45/90) 6.61 Kb:

5' Sequence: (SEQ.ID:50:)
...CCT TAG ATC/ GGT ACA ACC **ATG** AAG GCA ATA ATT GTA CTA CTC ATG GTA GTA ACA TCC AAC GCA GAT CGA ATC TGC ACT GGG ATA ACA TCT TCA AAC TCA CCT CAT GTG....

3' Sequence: (SEQ.ID:51:)

...TTG GCT GTA ACA TTG ATG ATA GCT ATT TTT ATT GTT TAT ATG GTC TCC AGA GAC AAT GTT TCT TGC TCC ATC TGT CTA **TAA** ATG TGG /GAT CTG CTG TGC CTT...

4. V1Jns-NP (A/Beijing/353/89) 6.42 Kb

5' Sequence: (SEQ.ID:52:)
...GTC CTT AGA TC/C ACC **ATG** GCG TCC CAA GGC ACC AAA CGG TCT TAT GAA CAG ATG GAA ACT GAT GGG GAA CGC CAG AAT GCA ACT ...

3' Sequence: (SEQ.ID:53:)
...GAA AAG GCA ACG AAC CCG ATC GTG CCC TCT TTT GAC ATG AGT AAT GAA GGA TCT TAT TTC TTC GGA GAC AAT GCA GAA GAG TAC GAC AAT **TAA** G/GA TCT GCT GTG CCT...

5. V1Jns-M1 (A/Beijing/353/89) 5.62 Kb

5' Sequence: (SEQ.ID:54:)

...CTT AGA TC/C AGA TCT ACC **ATG** AGT CTT CTA ACC GAG GTC GAA ACG TAT GTT CTC TCT ATC GTT CCA TCA GGC CCC CTC AAA GCC GAA ATC GCG CAG AGA CTT GAA GAT GTC TTT GCT GGG AAA AAC ACA GAT...

3' Sequence: (SEQ.ID:55:)

GGG ACT CAT CCT AGC TCC AGT ACT GGT CTA AAA GAT GAT CTT CTT GAA AAT TTG CAG ACC TAT CAG AAA CGA ATG GGG GTG CAG ATG CAA CGG TTC AAG **TGA** AGA TCT ATG TGG/ GAT CTG CTG TGC CTT...

6. V1Jns-NP (B/Panama/45/90) 6.54 Kb

5' Sequence: (SEQ.ID:56:)

...CTT AGA TC/C ACC **ATG** TCC AAC ATG GAT ATT GAC GGT ATC AAC ACT GGG ACA ATT GAC AAA ACA CCG GAA GAA ATA ACT TCT...

3' Sequence: (SEQ.ID:57:)

...GTT GAA ATT CCA ATT AAG CAG ACC ATC CCC AAT TTC TTC TTT GGG AGG GAC ACA GCA GAG GAT TAT GAT GAC CTC GAT TAT **TAA** G/GA TCT GCT GTG...

7. V1Jns-M1 (B/Panama/45/90) 5.61 Kb.

5' Sequence: (SEQ.ID:58:)

...CTT AGA TC/C ACC **ATG** TCG CTG TTT GGA GAC ACA ATT GCC TAC CTG CTT TCA TTG ACA GAA GAT GGA GAA GGC AAA GCA GAA CTA GCA GAA AAA TTA ...

3' Sequence: (SEQ.ID:59:)

...AGA TCT CTT GGG GCA AGT CAA GAG AAT GGG GAA GGA ATT GCA AAG GAT GTG ATG GAA GTG CTA AAG CAG AGC TCT ATG GGA AAT TCA GCT CTT GTG AAG AAA TAC CTA TAA G/GA TCT GCT GTG...

EXAMPLE 6

V1J EXPRESSION VECTOR, SEQ. ID: 10:

Our purpose in creating V1J was to remove the promoter and transcription termination elements from our vector, V1, in order to place them within a more defined context, create a more compact vector, and to improve plasmid purification yields.

V1J is derived from vectors V1, (see Example 1) and pUC18, a commercially available plasmid. V1 was digested with SspI and EcoRI restriction enzymes producing two fragments of DNA. The smaller of these fragments, containing the CMVintA promoter and Bovine Growth Hormone (BGH) transcription termination elements which control the expression of heterologous genes (SEQ ID:11:), was purified from an agarose electrophoresis gel. The ends of this DNA fragment were then "blunted" using the T4 DNA polymerase enzyme in order to facilitate its ligation to another "blunt-ended" DNA fragment.

pUC18 was chosen to provide the "backbone" of the expression vector. It is known to produce high yields of plasmid, is well-characterized by sequence and function, and is of minimum size. We removed the entire *lac* operon from this vector, which was unnecessary for our purposes and may be detrimental to plasmid yields and heterologous gene expression, by partial digestion with the HaeII restriction enzyme. The remaining plasmid was purified from an agarose electrophoresis gel, blunt-ended with the T4 DNA polymerase, treated with calf intestinal alkaline phosphatase, and ligated to the CMVintA/BGH element described above. Plasmids exhibiting either of two possible orientations of the promoter elements within the pUC backbone were obtained. One of these plasmids gave much higher yields of DNA in E. coli and was designated V1J (SEQ. ID: 10:). This vector's structure was verified by sequence analysis of the junction regions and was subsequently demonstrated to give comparable or higher expression of heterologous genes compared with V1.

EXAMPLE 7

INFLUENZA VIRUS GENE CONSTRUCTS IN EXPRESSION VECTOR V1J:

Many of the genes from the A/PR/8/34 strain of influenza virus were cloned into the expression vector V1J, which, as noted in Example 4, gives rise to expression at levels as high or higher than in the V1 vector. The PR8 gene sequences are known and available in the GENBANK database. For each of the genes cloned below, the size of the fragment cloned was checked by sizing gel, and the GENBANK accession number against which partial sequence was compared are provided. For a method of obtaining these genes from virus strains, for example from virus obtained from the ATCC (A/PR/8/34 is ATCC VR-95; many other strains are also on deposit with the ATCC), see Example 5.

A. Subcloning the PR8 Genes into V1J:

1. NP gene

The NP gene was subcloned from pAPR501 (J.F. Young, U. Desselberber, P. Graves, P. Palese, A. Shatzman, and M. Rosenberg (1983), in The Origins of Pandemic Influenza Viruses. ed. W.G. Layer, (Elsevier, Amsterdam) pp.129-138). It was excised by cutting pAPR501 with EcoRI, the fragment gel purified, and blunted with T4 DNA Polymerase. The blunted fragment was inserted into V1J cut with Bgl II and also blunted with T4 DNA Polymerase. The cloned fragment was 1.6 kilobases long.

2. NS

The NS gene was subcloned from pAPR801 (J.F. Young, U. Desselberber, P. Graves, P. Palese, A. Shatzman, and M. Rosenberg (1983), in The Origins of Pandemic Influenza Viruses, ed. W.G. Laver, (Elsevier, Amsterdam) pp.129-138). It was excised by cutting pAPR801 with EcoRI, the fragment gel purified, and blunted with T4 DNA Polymerase. The blunted fragment was inserted into V1J cut with Bgl II and also blunted with T4 DNA Polymerase. The cloned fragment was 0.9 kilobases long (the complete NS coding region including NS1 and NS2).

3. HA

The HA gene was subcloned from pJZ102 (J.F. Young, U. Desselberber, P. Graves, P. Palese, A. Shatzman, and M. Rosenberg (1983), in The Origins of Pandemic Influenza Viruses, ed. W.G. Laver, (Elsevier, Amsterdam) pp.129-138). It was excised by cutting pJZ102 with Hind III, the fragment gel purified, and blunted with T4 DNA Polymerase. The blunted fragment was inserted into V1J cut with Bgl II and also blunted with T4 DNA Polymerase. The cloned fragment was 1.75 kilobases long.

4. PB1

The PB1 gene was subcloned from pGem1-PB1 (The 5' and 3' junctions of the genes with the vector were sequenced to verify their identity. See J.F. Young, U. Desselberber, P. Graves, P. Palese, A. Shatzman, and M. Rosenberg (1983), in The Origins of Pandemic Influenza Viruses, ed. W.G. Laver, (Elsevier, Amsterdam) pp.129-138). It was excised by cutting pGem-PB1 with Hind III, the fragment gel purified, and blunted with T4 DNA Polymerase. The blunted fragment was inserted into V1J cut with Bgl II and also blunted with T4 DNA Polymerase. The cloned fragment was 2.3 kilobases long.

5. PB2

The PB2 gene was subcloned from pGem1-PB2 (The 5' and 3' junctions of the genes with the vector were sequenced to verify their identity. See J.F. Young, U. Desselberber, P. Graves, P. Palese, A. Shatzman, and M. Rosenberg (1983), in The Origins of Pandemic Influenza Viruses, ed. W.G. Laver, (Elsevier, Amsterdam) pp.129-138). It was excised by cutting pGem-PB2 with BamH I, and gel purifying the fragment. The sticky-ended fragment was inserted into V1J cut with Bgl II. The cloned fragment was 2.3 kilobases long.

6. M1

The M1 gene was generated by PCR from the plasmid p8901 MITE. The M sequence in this plasmid was generated by PCR from pAPR701 (J.F. Young, U. Desselberber, P. Graves, P. Palese, A. Shatzman, and M. Rosenberg (1983), in The Origins of Pandemic Influenza Viruses, ed. W.G. Laver, (Elsevier, Amsterdam) pp.129-138.), using the oligomer 5' -GGT ACA AGA TCT ACC ATG CTT CTA ACC GAG GTC-3' , SEQ. ID:3:, for the "sense" primer and the oligomer 5'-CCA CAT AGA TCT TCA CTT GAA CCG TTG CAT CTG CAC-3', SEQ. ID:4:, for the "anti-sense" primer. The PCR fragment was gel purified, cut with Bgl II and ligated into V1J cut with Bgl II. The cloned fragment was 0.7 kilobases long. The amino terminus of the encoded M1 is encoded in the "sense" primer shown above as the "ATG" codon, while the M1 translation stop codon is encoded by the reverse of the "TCA" codon, which in the sense direction is the stop codon "TGA".

B. Influenza Gene-V1J Expression Constructs:

In each case, the junction sequences from the 5' promoter region (CMVintA) into the cloned gene is shown. The sequences were generated by sequencing off the primer: CMVinta primer 5'- CTA ACA GAC TGT TCC TTT CCA TG- 3', SEQ. ID:28:, which generates the sequence of the coding sequence. The position at which the junction occurs is demarcated by a "/", which does not represent any discontinuity in the sequence. The method for preparing these constructs is summarized after all of the sequences below. Each sequence provided represents a complete, available, expressible DNA construct for the designated influenza gene.

Each construct was transiently transfected into RD cells, (ATCC CCL136), a human rhabdomyosarcoma cell line in culture. Forty eight hours after transfection, the cells were harvested, lysed, and western blots were run (except for the V1J-PR-HA construct which was tested in mice and gave anti-HA specific antibody before a western blot was run, thus obviating the need to run a western blot as expression was observed in vivo). Antibody specific for the PB1, PB2 and NS proteins was provided by Stephen Inglis of the University of Cambridge, who used purified proteins expressed as $\beta$-galactosidase fusion proteins to generate polyclonal antisera. Anti-NP polyclonal antiserum was generated by immunization of rabbits with whole A/PR/8/34 virus. Anti-M1 antibody is commercially available from Biodesign as a goat, anti-fluA antiserum, catalog number B65245G. In each case, a protein of the predicted size was observed, confirming expression in vitro of the encoded influenza protein.

The nomenclature for these constructs follows the convention: "Vector name-flu strain-gene". In every case, the sequence was checked against known sequences from GENBANK for the cloned and sequenced A/PR/8/34 gene sequence. The biological efficacy of each of these constructs is demonstrated as in Examples 2, 3 , and 4 above:

SEQUENCE ACROSS THE 5' JUNCTIONS OF CMVINTA AND FLU GENES FROM A/PR/8/34:

1. V1J-PR-NP, SEQ. ID:12:, GENBANK ACCESSION #:M38279

5' GTC ACC GTC CTT AGA TC/A ATT CCA GCA AAA GCA GGG
**CMVintA** **NP....**

TAG ATA ATC ACT CAC TGA GTG ACA TCA AAA TCA TG

2. V1J-PR-PB1, SEQ. ID:13:, GENBANK ACCESSION #J02151

5' ACC GTC CTT AGA TC/A GCT TGG CAA AAG CAG GCA AAC
**CMVintA** **PB1....**

CAT TTG AAT GGA TGT CAA TCC GAC CTT ACT TTT CTT AAA AGT GCC AGC ACA AAA TGC TAT AAG CAC AAC TTT CCC TTA TAC

3. V1J-PR-NS, SEQ. ID:14:, GENBANK ACCESSION #J02150

5' GTC ACC GTC CTT AGA TC/A ATT CCA GCA AAA GCA GGG
**CMVintA** **NS....**

TGA CAA AAA CAT AAT GGA TCC AAA CAC TGT GTC AAG CTT TCA GGT AGA TTG CTT TCT TTG GCA TGT CCG CAA ACG AGT TGC AGA CCA AGA ACT AGG TGA T...

4. V1J-PR-HA, SEQ. ID:15:, GENBANK ACCESSION #J02143

5' TCT GCA GTC ACC GTC CTT AGA TC/ A GCT TGG AGC AAA
**CMVintA** **HA...**

AGCAGG GGA AAA TAA AAA CAA CCA AAA TGA AGG CAA ACC TAC TGG TCC TGT TAA GTG CAC TTG CAG CTG CAG ATG CAG ACA CAA TAT GTA TAG GCT ACC ATG CGA ACA ATT CAA CC...

5. V1J-PR-PB2, SEQ. ID:16:, GENBANK ACCESSION #J02153

5'TTT TCT GCA GTC ACC GTC CTT AGA TC/ C CGA ATT CCA
CMVintA PB2....

GCA AAA GCA GGT CAA TTA TAT TCA ATA TGG AAA GAA TAA AAG AAC TAA GAA ATC TAA TGT CGC AGT CTG CCA CCC CGG AGA TAC TCA CAA AAA CCA CCG TGG ACC ATA TGG CCA TAA TCA AGA AGT...

6. V1J-PR-M1, SEQ. ID:17:, GENBANK ACCESSION #J02145

5' GTC ACC GTC CTT AGA TCT/ ACC ATG AGT CTT CTA ACC
CMVINTA M1.....

GAG GTC GAA ACG TAC GTA CTC TCT ATC ATC CCG TCA GGC CCC CTC AAA GCC GAG ATC GCA CAG AGA CTT GAA GAG TTG ACG GAA GA...

How Fragments were joined:

1. V1J-PR-NP: Blunted BglII (vector) to blunted EcoRI (NP)
2. V1J-PR-PB1: Blunted BglII (vector) to blunted HinDIII (PB1)
3. V1J-PR-NS: Blunted BglII (vector) to blunted EcoRI (NS1)
4. V1J-PR-HA: Blunted BglII (vector) to blunted HinDIII (HA)
5. V1J-PR-PB2: Sticky BglII (vector) to sticky BamHI (PB2)
6. V1J-PR-M1: Sticky BglII (vector) to sticky BglII (M1)

M1 was obtained by PCR, using p8901-M1TE as template and Primers that add a BglII site at both ends and start 3 bases befor the ATG and end right after the termination codon for M1 (TGA).

EXAMPLE 8

V1Jneo EXPRESSION VECTOR, SEQ. ID:18:

It was necessary to remove the $amp^r$ gene used for antibiotic selection of bacteria harboring V1J because ampicillin may not be used in large-scale fermenters. The $amp^r$ gene from the pUC backbone of V1J was removed by digestion with SspI and Eam 1 105I restriction enzymes. The remaining plasmid was purified by agarose gel electrophoresis, blunt-ended with T4 DNA polymerase, and then treated with calf intestinal alkaline phosphatase. The commercially available $kan^r$ gene, derived from transposon 903 and contained within the pUC4K plasmid, was excised using the PstI restriction enzyme, purified by agarose gel electrophoresis, and blunt-ended with T4 DNA polymerase. This fragment was ligated with the V1J backbone and plasmids with the $kan^r$ gene in either orientation were derived which were designated as V1Jneo #'s 1 and 3. Each of these plasmids was confirmed by restriction enzyme digestion analysis, DNA sequencing of the junction regions, and was shown to produce similar quantities of plasmid as V1J. Expression of heterologous gene products was also comparable to V1J for these V1Jneo vectors. We arbitrarily selected V1Jneo#3, referred to as V1Jneo hereafter (SEQ. ID:18:), which contains the $kan^r$ gene in the same orientation as the $amp^r$ gene in V1J as the expression construct.

Genes from each of the strains A/Beijing/353/89, A/Texas/36/91, and B/Panama/46/90 were cloned into the vector V1Jneo as cDNAs. In each case, the junction sequences from the 5' promoter region (CMVintA) into the cloned gene was sequenced using the primer:

CMVinta primer 5'- CTA ACA GAC TGT TCC TTT CCA TG- 3', SEQ. ID:28:, which generates the sequence of the coding sequence. This is contiguous with the terminator/coding sequence, the junction of which is also shown. This sequence was generated using the primer: BGH primer 5'- GGA GTG GCA CCT TCC AGG -3', SEQ. ID:29:, which generates the sequence of the non-coding strand. In every case, the sequence was checked against known sequences from GENBANK for cloned and sequenced genes from these or other influenza isolates. The position at which the junction occurs is demarcated by a "/", which does not represent any discontinuity in the sequence. In the case of the V1Jneo-TX-HA junction, the sequencing gel was compressed and the initial sequence was difficult to read. Therefore, the first 8 bases at that junction were shown as "N". These nucleotides have been confirmed and the identified nucleotides are provided. The first "ATG" encountered in each sequence is the translation initiation codon for the respective cloned gene. Each sequence provided represents a complete, available, expressible DNA construct for the designated influenza gene. The nomenclature follows the convention: "Vector name-flu strain-gene". The biological efficacy of each of these constructs is shown in the same manner as in Examples 2, 3, and 4 above:

## SEQUENCE ACROSS THE 5' JUNCTIONS OF CMVintA AND THE FLU GENES AND ACROSS THE 3' JUNCTIONS OF THE FLU GENES AND THE BGH TERMINATOR EXPRESSION CONSTRUCTS, USING DIFFERENT INFLUENZA STRAINS AND PROTEINS:

## I. A/BEIJING/353/89

### A. V1Jneo-BJ-NP:

PROMOTER, SEQ. ID:20:

5' TCA CCG TCC TTA GAT C/ AA GCA GGG TTA ATA ATC
**CMVintA** **NP....**

ACT CAC TGA GTG ACA TCA AAA TC ATG GCG TCC CAA GGC ACC AAA CGG TCT TAT GAA CAG ATG GAA ACT GAT GGG GAA CGC CAG ATT

TERMINATOR, SEQ. ID:21:

5' GAG GGG CAA ACA ACA GAT GGC TGG CAA CTA GAA GGC ACA GCA GAT / ATT TTT TCC TTA ATT GTC GTA C...
**BGH** **NP....**

## II. A/TEXAS/36/91

### A. V1Jneo-TX-HA

PROMOTER, SEQ. ID:24:

5' CCT TAG ATC / GGA AAT AAA AAC AAC CAA AAT GAA
**CMVINTA** **HA....**

AGC AAA ACT ACT AGT CC...

TERMINATOR, SEQ. ID:25:

5' GCA GAT C/ CT TAT ATT TCT GAA ATT CTG GTC...
**BGH** **HA....**

TCA GAT...

## III. B/PANAMA/46/90

### A. V1Jneo-PA-HA

PROMOTER, SEQ. ID:26: (The first 1080 bases of this sequence is available on GENBANK as accession number M65171; the sequence obtained below is identical with the known sequence; the 3' sequence, SEQ. ID:27: below) has not been previously reported)

5'ACC GTC CTT AGA TC/ C AGA AGC AGA GCA TTT TCT AAT

**CMVintA** **HA....**

ATC CAC AAA ATG AAG GCA ATA ATT GTA CTA CTC ATG GTA GTA ACA TCC AAC GCA GAT CGA ATC TGC...

TERMINATOR, SEQ. ID:27:

5' GGC ACA GCA GAT C/ TT TCA ATA ACG TTT CTT TGT
**BGH** **HA....**

AAT GGT AAC...

EXAMPLE 9

Intradermal Injections of Influenza Genes:

The protocol for intradermal introduction of genes was the same as for intramuscular introduction: Three injections of 200μg each, three weeks apart, of V1-PR-NP. The spleens were harvested for the in vitro assay 55 days after the third injection, and restimulated with the nonapeptide nucleoprotein epitope 147-155, SEQ. ID:9:. Target cells (P815 cells, mouse mastocytoma, syngeneic with BALB/c mice H-$2^d$) were infected with the heterologous virus A/Victoria/73, and specific lysis using the spleen cells as the effector at effector:target ratios ranging between 5:1 and 40:1. Negative controls were carried out by measuring lysis of target cells which were not infected with influenza virus. Positive controls were carried out by measuring lysis of influenza virus infected target cells by spleen cells obtained from a mouse which was injected three times with 130 μg of V1-PR-NP and which survived a live influenza virus infection by strain A/HK/68.

Results: Specific lysis was achieved using the spleen cells from intradermally injected mice at all effector:target ratios. No specific lysis was seen when spleen cells obtained from uninjected mice, or mice injected with the vector V1 without the inserted PR-NP gene, were used as the effector cells. In addition, the specific lysis achieved using the intradermal delivery was comparable at all effector:target ratios to the results obtained using intramuscular delivery. Influenza virus lung titers were also measured in mice injected intradermally or intramusculary. The results, using 5 mice per group, 3 x 200 μg per dose three weeks apart, and challenge 3 weeks post last dose, were as follows:

| Vaccine | Mode of Delivery | Mouse Lung Titer* | |
|---|---|---|---|
| | | Day 5 | Day 7 |
| V1-PR-NP | Intradermal | 5.2 ± 0.2 | 4.1 ± 1** |
| V1 | Intradermal | 5.9 ± 1 | 6.6 ± 0.3 |
| V1-PR-NP | Intramuscular | 4.6 ± 0.4 | 4.5 ± 1.1** |
| None | -------------- | 6.2 ± 0.3 | 5.9 ± 0.3 |

* Mean log titer ± SEM.
** One mouse had no virus at all.

Finally, percent survival of mice was tested out to twenty eight days. By day twenty eight, of the mice receiving V1-NP-PR, 89% of the i.m. recipients and 50% of the id. recipients survived. None of the V1 vector and only 30% of the untreated mice survived. This experiment demonstrates that DNA encoding nucleoprotein from the A/PR/8/34 strain was able to induce CTL's that recognized the nucleoprotein from the hetereologous strain A/Victoria/73 and a protective immune response against the heterologous strain A/HK/68.

EXAMPLE 10

Polynucleotide vaccination in primates

1. Antibody to NP in Rhesus monkeys: Rhesus monkeys (006 NP, 009 NP or control 101; 021) were injected with 1 mg/site of RSV-NP i.m. in 3 sites on day 1. Injections of 1 mg each of RSV-LUX and CMV-int-LUX, constructs for the reporter gene firefly luciferase expression, were given at the same time into separate sites. Animals were reinjected on day 15 with the same amounts of DNA as before and also with 1 mg of pD5-CAT, a construct for the reporter gene chloramphenical acetyl transferase expression, in 1 site each. Muscle sites containing reporter genes were biopsied and assayed for reporter gene activity. Serum was collected 3, 5, 9, 11, 13, and 15 weeks after the first injection. The first positive sample for anti-NP antibody was collected at week 11 and positive samples were also collected on weeks 13 and 15. Anti-NP antibody was determined by ELISA. The results are shown in Figure 9.

2. Hemagglutination inhibiting (HI) antibody in rhesus monkeys: Monkeys were injected i.m. with V1J-PR-HA on day 1. Two animals each received 1 mg, 100 µg, or 10 µg DNA in each quadriceps muscle. Each injection was administered in a volume of 0.5 ml. Animals were bled prior to injection on day 1. All animals were reinjected with DNA on day 15, and blood was collected at 2-4 week intervals thereafter. Hemagglutination inhibition (HI) titers against A/PR/8/34 were positive at 5 weeks, 9 weeks and 12 weeks after the first injection of V1J-PR-HA DNA. Results are shown below in Table 10-I:

TABLE 10-I

| HI ANTIBODY TITER OF RHESUS MONKEYS RECEIVING V1J-PR-HA DNA | | | | | | |
|---|---|---|---|---|---|---|
| RHESUS # | DOSE | HI ANTIBODY TITER AT WEEK # | | | | |
| | | PRE | 3 WK | 5 WK | 9 WK | 12 WK |
| 88-010 | 1 MG | <10 | <10 | 320 | 320 | 320 |
| 88-0200 | | <10 | <10 | <10 | 40 | 40 |
| | | | | | | |
| 88-021 | 100 UG | <10 | <10 | <10 | 40 | 20 |
| 90-026 | | <10 | <10 | 20 | 20 | 40 |
| | | | | | | |
| 88-084 | 10 UG | <10 | 20 | 40 | 20 | 10 |
| 90-028 | | <10 | <10 | 20 | <10 | <10 |

EXAMPLE 11

Polynucleotide vaccine studies in ferrets

1. A study of polynucleotide vaccination in ferrets was initiated with the purpose of determining whether animals could be protected from influenza A infection by immunization with genes encoding either the HA (a surface protein capable of inducing strain-specific neutralizing antibody) or the interal protein NP, NSI, PBI, M (thought to induce a cell-mediated immune response that would be strain-independent). Animals were injected with DNA encoding the various influenza genes in our V1J-vector as shown:

TABLE 11-1

| Group | Construct | Dose | No. Animals Immunized | Chall. H1N1 | Chall. H3N2 |
|---|---|---|---|---|---|
| 1 | V1J-HA | 1000 mg | 16 | 8 | 8 |
| 2 | V1J-NP | 1000 mg | 16 | 8 | 8 |
| 3 | V1J-NP + NS1 + PB1 + PB2 + M | 2000 mg total | 16 | 8 | 8 |
| 4 | V1J-HA + NP + NS1 + PB1 + PB2 + M | 2000 mg total | 16 | 8 | 8 |
| 5 | V1J- | 1000 mg | 16 | 8 | 8 |
| 6 | None | None | 10 | 5 | 5 |
| Total Animals | | | 90 | 45 | 45 |

2. On days 22 and 43 postimmunization, serum was collected from the immunized animals and assayed for neutralizing (hemagglutination inhibiting-HI) antibodies and for antibodies to nucleoprotein (NP) by ELISA. Animals that had been injected with DNA expressed antibodies to the corresponding genes. These are reflected in the attached Figures 10, 11, and 16.

3. On Day 128, selected immunized animals were challenged with 1200 TCID50 of Influenza A/HK/68. This strain is heterologous to the A/PR/8/34 strain that was the source of the coding sequences used to immunize and therefore protection indicates immunity based on cell-mediated, strain-independent immune mechanisms. As shown in the attached Figure 12, a statistically significant reduction in viral shedding compared to controls was seen m animals immunized with DNA encoding internal proteins, confirming that polynucleotide immunization in ferrets is capable of eliciting an immune response and that such responses are protective. 4. A homologous challenge using A/PR/8/34 is similarly tested and the protective efficacy of neutralizing antibody induced by polynucleotide vaccination is demonstrated similarly.

EXAMPLE 12

PRODUCTION OF V1Jns

An Sfi I site was added to V1Jneo to facilitate integration studies. A commercially available 13 base pair Sfi I linker (New England BioLabs) was added at the Kpn I site within the BGH sequence of the vector. V1Jneo was linearized with Kpn I, gel purified, blunted by T4 DNA polymerase, and ligated to the blunt Sfi I linker. Clonal isolates were chosen by restriction mapping and verified by sequencing through the linker. The new vector was designated V1Jns (Figure 17). Expression of heterologous genes in V1Jns (with Sfi I) was comparable to expression of the same genes in V1Jneo (with Kpn I).

EXAMPLE 13

IMMUNOGENICITY

1. Humoral Immune Responses

Injection of DNA encoding influenza HA, NP and M1 has resulted in humoral immune responses in mice, ferrets or non-human primates (including African green monkeys and Rhesus monkeys). To date, PNVs containing HA genes cloned from the A/PR/34, B/Panama/90, A/Beijing/89, A/Texas/91, A/Hawaii/91, and A/Georgia/93 strains of influenza virus have been shown to generate antibodies.

a) Mice: Antibodies to NP and M1 were detected by ELISA in sera from mice after injection of DNA. Substantial antibody titers ($10^4$ - $10^6$) were generated with as low as 1 $\mu$g of NP DNA (A/PR/34) administered once (the smallest dose tested), arose as soon as 2 weeks after injection (the earliest time point tested), and have not decreased for at least 6 months after injection. These NP antibodies are not neutralizing and do not participate in protection. They do, however, demonstrate NP protein expression *in vivo* after DNA injection. In contrast, antibodies to HA do provide protective immunity against the homologous strain of influenza vtrus. Injection of HA DNA cloned from the A/PR/34 strain resulted in the production of neutralizing antibodies, as measured *in vitro* by a hemagglutination inhibition (HI) assay. HI titers ≧ 1280 were measured in many mice given three doses of 100 $\mu$g of HA DNA, and detectable titers were seen in some animals that had received a little as two doses of 0.1 $\mu$g. There was a dose-response relationship between HI titer and DNA dose, as well as HI titer and number of injections (Table 13-I):

Table 13-I

| Generation of Humoral Immune Responses in Mice | | | |
|---|---|---|---|
| dose (µg) | GMT HI | | |
| | # doses | | |
| | 1 | 2 | 3 |
| HA DNA (100) | 75 | 106 | 260 |
| HA DNA (10) | 37 | 69 | 86 |
| HA DNA (1) | <10[a] | 13 | 24 |
| HA DNA (0.1) | <10[b] | <10[a] | <10[a] |
| Control DNA (100) | <10[b] | <10[b] | <10[b] |
| uninjected | | <10[b] | |

Table 13-I: Female BALB/c mice (4-6 weeks) were injected with A/PR/34 HA DNA (V1JHA) at the indicated doses either once, twice or three times at three week intervals. Negative controls included mice injected with control DNA consisting of the vector without a gene insert (V1J) and naive, uninjected mice. Serum samples were collected at seven weeks post-dose one and analyzed for the presence of hemagglutination inhibition (HI) antibodies. The data is represented as geometric mean HI titer where n = 10.

[a] some of the mice tested positive for HI titer.
[b] all of the mice.

In every mouse tested, the presence of HI antibodies correlated with protection in a homologous challenge model. HI antibody responses in mice injected with HA DNA (A/PR/34) have remained essentially unchanged for at least six months. HA antibodies, as measured by ELISA, have also been generated in mice using HA DNA from A/Beijing/89, B/Panama/90, and A/Texas/91 strains of influenza virus.

Based on a report in the literature that demonstrated lower reporter gene expression in older mice after injection of DNA, the effect of age on humoral immune responses to HA was tested. Due to the lack of availability of senescent virgin female mice, retired breeders of approximately 10 months of age were used. Retired breeders and 4-6 week-old virgin mice were compared for their ability to generate antibodies to HA. The older mice were able to generate HA antibodies after injection of HA DNA at doses as low as 1 μg (the lowest dose tested), albeit lower in titer than in the younger mice (Table 13-II):

Table 13-II

| Effect of Age on Humoral Immune Responses | | | |
|---|---|---|---|
| inoculum | dose (μg) | GMT (4-6 wk) | GMT (10 mo) |
| HA DNA | 100 | 1034 | 110 |
| HA DNA | 10 | 338 | 68 |
| HA DNA | 1 | 80 | 20 |
| Control DNA | 100 | <5[a] | <5[a] |
| Uninjected | - | <5[a] | <5[a] |
| Flu | - | 538 | 36 |

Table 13-II: Female BALB/c mice (4-6 week virgins and 10-month retired breeders) were injected with A/PR/34 HA DNA (V1JHA) at the indicated doses three times at three week intervals. Negative controls included mice injected with control DNA (V1J) and naive, uninjected mice. For comparison, other mice were infected with a sublethal dose of influenza A/PR/34. Serum samples were collected at nine weeks post-dose one and analyzed for HI titer. Data is represented as geometric mean HI titer where n = 15.

[a] all mice tested negative for HI titer

However, this was not a result of the PNV itself, but rather a diminished capacity in the older mice to generate humoral immune responses in general since older mice also exhibited lower HI responses than younger mice after infection with live A/PR/34 virus. In fact, the HI antibodies appeared to be less depressed in DNA-vaccinated aged mice than in influenza-infected aged mice. Moreover, the retired breeders used in these studies were approximately 50% heavier than typical virgins of the same age, which based on studies of others using calorie-restricted diets in mice could have had a detrimental effect on the immune responses of these animals. For this and other reasons, the immune responses of these mice may not be representative. Nevertheless, age (up to at least 10 months) does not appear to have significantly reduced the ability of polynucleotide vaccination to induce humoral immune responses, even at doses of as low as 1 µg.

b) Ferrets: Humoral immune responses have been generated in ferrets injected with HA DNA from the A/PR/34, A/Beijing/89, A/Hawaii/91 and A/Georgia/93 strains of influenza virus. HI antibodies against A/PR/34 and ELISA antibodies against HAs from the other strains were elicited by the appropriate PNV. Sera from ferrets injected with A/Beijing/89, A/Hawaii/91, and A/Georgia/93 HA DNAs are found to have HI antibodies and neutralizing antibodies, since these animals were protected from virus challenge.

c) Non-Human Primates: (See also Example 10 above). Rhesus monkeys were immunized twice with HA DNA (A/PR/34) at doses of 10,100, and 1000 µg per leg. HI titers of up to 320 were measured in animals that had received 100 or 1000 µg doses, and one of the two 10 µg-dose monkeys had an HI of 80. So far, sera have been assayed out to 13 months; HI titers did not decline appreciably from 6-13 months (Table 13-III):

Table 13-III

| Generation of HI Antibodies in Rhesus Monkeys | | | | | |
|---|---|---|---|---|---|
| dose ($\mu g$) | pre | 1 mo | 2 mo | 5.5 mo | 13 mo |
| 2000 | <10 | 640 | 320 | 160 | 80 |
| 2000 | <10 | 40 | 40 | 20 | 20 |
| 200 | <10 | 80 | 40 | 40 | 80 |
| 200 | <10 | 80 | 80 | 40 | 20 |
| 20 | <10 | 40 | 20 | 20 | 20 |
| 20 | <10 | <10 | <10 | <10 | <10 |

Table 13-III: Rhesus monkeys (both male and female) ranging in size from 4.3 to 8.8 kg were injected with A/PR/34 HA DNA (V1JHA) at the indicated doses at 0 and 2 weeks. Serum samples were collected at the indicated times post-dose one and analyzed for HI titer. The data represent HI titers for individual animals.

In African green monkeys injected with a combination PNV containing 100 µg HA DNA (A/Beijing/89), evaluation of sera 4-6 weeks post-dose 1 showed a GMT of 29(8/9 responding). This compares favorably with responses to both licensed subvirion vaccine (GMT of 16, 5/6 responding) and licensed whole virion vaccine (GMT = 36, 6/6 responding) at the same time point (Figure 18). A marked booster effect of the second immunization was seen in animals receiving a 10-µg dose of HA DNA (GMT of 1.9 after 1 dose and 199 after two doses). Licensed whole virion vaccine demonstrated a similar boosting effect, whereas HI titers produced by the second dose of subvirion vaccine were only transient. To date, similar levels of HI antibodies have been measured out to 18 weeks in animals immunized with both 10 µg and 100 µg doses of HA DNA compared to the best licensed vaccine (whole virion). These results demonstrate that the PNV was as least as effective in generating neutralizing antibodies as whole virion vaccine and superior to subvirion vaccine. Purified subunit vaccines were not detectably immunogenic in mice; hence they were not tested in non-human primates. In this study, the PNV contained a 5-valent cocktail of DNAs encoding HA from A/Beijing/89, B/Panama/90 and A/Texas/91, and NP and M1 from A/PR/34, in order to resemble a candidate vaccine. We have also tested these animals for the generation of humoral immune responses against the other components of the vaccine and have detected antibodies to B/Panama/90 HA and A/PR/34 NP. In a separate experiment, both HI and neutralizing antibodies against A/Texas/91 HA were induced by injection of two doses of PCR-cloned HA DNA.

2. Cell-Mediated Immune Responses

See Example 3 above.

3. Generation of Immune Responses

a) Humoral Immunity: The events leading to the production of humoral and cell-mediated immune responses after injection of DNA have not yet been elucidated. To engender neutralizing antibodies (*e.g.*, against influenza virus HA), it is likely that cells must express the antigen on the plasma membrane or secrete it into the extracellular milieu. In addition, transfected cells should express HA with secondary, tertiary and quaternary structure similar to that in the virion. In a rosetting assay, cell surface expression of HA was demonstrated in RD cells (rhabdomyosarcoma; myoblast origin) transiently transfected with HA DNA. Red blood cells agglutinated to the surface of HA transfected cells but not mock-transfected cells, indicating that HA was not only expressed on the surface but also had retained the proper conformation for binding to sialic acid-containing proteins.

b) Cell-Mediated Immunity: The generation of cell-mediated immune responses (*e.g.*, against influenza virus NP) requires proteolytic processing and presentation of peptides derived therefrom in association with MHC class I. The nature of the antigen presenting cell leading to the generation of immune responses after injection of DNA is not yet known. Muscle cells express low levels of MHC class I and are not thought to express costimulatory molecules on their surfaces. Therefore, muscle cells are not generally considered to be antigen presenting cells. However, several lines of evidence suggest that muscle cells are involved in the generation of immune responses after i.m. injection of DNA. First, a limited survey of the tissues capable of internalizing naked plasmid DNA leading to protein expression *in situ* demonstrated that many cell types can express reporter genes when the plasmid is injected directly into the tissue, but substantially less efficiently than muscle cells. A complete analysis of the uptake of DNA by non-muscle cells after i.m. injection has not yet been reported, but it is likely that uptake would be even less efficient. Second, expression of reporter genes after i.m. injection of DNA has been demonstrated in skeletal and cardiac muscle cells in many different species. Third, although CTL responses can be generated after injection of DNA via other routes (iv. and id.), the best protective immune responses in mice were elicited after i.m. injection of DNA. Fourth, myoblasts and myocytes can be recognized and lysed by CTL *in vitro* and this lysis can by enhanced by pretreatment with γ-interferon, which upregulates MHC class I expression. Finally, transplantation of stably transfected, NP-expressing myoblasts into naive, syngeneic mice resulted in the generation of protective cell-mediated immune responses *in vivo* (Figure 20). Therefore, expression of antigens by muscle cells is sufficient to induce the protective immune responses seen after DNA injection. Furthermore, uptake and expression of DNA by non-muscle cells may not be required to account for the generation of protective immunity. From the standpoint of polynucleotides as vaccines, it would be potentially advantageous to limit DNA uptake to muscle cells. First, myocytes are terminally differentiated and do not divide. This could be important for reducing the possibility of integration of plasmid DNA into chromosomal DNA and maintaining a persistent expression of antigen, which could lead to long-lived immune responses. Second, myocytes are large, multinucleate cells that can be regenerated by fusion of myoblasts. This may help to explain why injection of DNA leads to protein expression that can potentially persist for long periods of time without evidence of cytolytic destruction by CTL.

EXAMPLE 14

Protection Studies

Immunization with DNA encoding influenza virus antigens provided protection from death and disease, and reduced viral burdens, in a variety of combinations of influenza strains, using two widely accepted animal models for human influenza infection (mice and ferrets).

1. Heterologous (heterotypic, group-common) protection is not provided efficiently by the licensed killed virus vaccine but was provided by DNA vaccination in animal models. This protection was demonstrated when DNA that encoded NP or M1 was injected into laboratory animals. The cross-reactive cell-mediated immune (CMI) response induced by vaccination with these DNAs provided a protective response.

a. Mice: BALB/c and C3H mice injected i.m. with DNA coding for NP from A/PR/34 were protected from death and from disease (assessed by reduction in body weight) when challenged by total respiratory tract infection with an $LD_{90}$ of A/Hong Kong/68 (H3N2), a heterotypic strain (H3 vs H1 for A/PR/34). Figure 21 shows the survival of BALB/c mice immunized 3 times with NP DNA (200 $\mu$g/dose) at 3-week intervals and challenged 3 weeks after the last immunization. Figure 22 shows the inhibition of weight loss after challenge in immunized mice compared with the severe weight loss experienced by control mice. Figure 23 shows the reduction in viral burden in the lung 7 days after after upper respiratory tract challenge of mice immunized with NP DNA compared with mice given control noncoding DNA. Mice immunized with NP DNA were completely protected from death, experienced reduced weight loss, and showed lower viral burden in the lungs when compared with control mice. The amount of NP DNA required to protect mice from death and weight loss was found to be ≦6.25 $\mu$g per injection when 3 injections of DNA were given (Figure 24). The protection produced by immunization with NP DNA was found to persist essentially unchanged for at least 3 months in immunized mice. The level of protection persisted to 6 months, but declined slightly between 3 and 6 months after the last immunization. However revaccination with a single injection of NP DNA at 22 weeks, 3 weeks prior to challenge at 25 weeks, restored full protection (Figure 25). Thus immunization of mice with NP DNA produced a long-lasting, boostable, heterologous protection. The ability to generate an anamnestic response upon revaccination of these animals suggests that immunological memory was induced by the NP DNA immunization.

b. Ferrets: Ferrets are a commonly used model for human influenza infection because they are susceptible to infection with a wide variety of human isolates of influenza virus. Virus replication in the ferret occurs predominantly in the nares and trachea and to a much lesser extent in the lung, in contrast to mice in which the viral burden in the lung is large. Infection in the ferret is followed most readily by titration of virus in nasal wash fluid. Ferrets immunized with NP DNA or M1 DNA, singly or in combination, from a strain recently obtained from humans (A/Beijing/89, H3N2), exhibited significantly reduced viral shedding on days 1-6 upon challenge with a field isolate, A/Georgia/93 (H3N2) (Figure 26). This field isolate exhibited antigenic drift from the A/Beijing/89 type strain such that the A/Beijing/89 licensed vaccine offered little or no protection of humans against disease caused by A/Georgia/93. Ferrets immunized with DNA encoding internal proteins of A/PR/34 exhibited significantly reduced nasal viral shedding on days 5 and 6 after infection with the homotypic strain, A/PR/34 (Figure 27). Reduction in viral shedding was seen after A/Georgia/93 challenge at both early and late time points, while late reduction in shedding was observed after A/PR/34 challenge; this may be due to a difference in virulence for ferrets between the two strains.

2. Homologous (homotypic, type-specific) protection was demonstrated readily in both mice and ferrets imunized with HA DNA.

a. Mice: BALB/c mice immunized with HA DNA (A/PR/34) were fully protected against challenge with an $LD_{90}$ of A/PR/34. Immunized mice experienced neither death (Figure 28) nor loss of more than 5% of body weight (Figure 29) after challenge, while 90-100% of control mice died and experienced severe weight loss. Titration of the dose of HA DNA required to achieve protection showed that three injections of 1 $\mu$g HA DNA was sufficient to achieve full protection (Figure 30).

b. Ferrets: Ferrets that had been immunized with DNA that coded for the HA from A/PR/34 had significantly lower viral shedding on days 1-6 after homologous challenge infection than ferrets given control DNA (Figure 31). Similarly, ferrets that had been immunized with A/Georgia/93 HA DNA had reduced viral shedding on days 1 and 3-7 after homologous infection (Figure 32). HI antibodies were present against the appropriate strains in sera from all of the immunized ferrets (*vide supra*). Thus immunization with HA DNA produces homologous protection.

3. Vaccine combinations: The ability of HA DNA to provide superior breadth of protection when combined with NP and M1 DNAs was examined in ferrets.

a. Breadth of protection against antigenic drift variants: The antigenic drift that occurred between the A/Beijing/89 and A/Beijing/92 strains was of sufficient magnitude that many humans immunized with the licensed vaccine containing the A/Beijing/89 strain were not protected against disease caused by the A/Beijing/92 variant. In North America, widespread disease was caused by A/Beijing/92-like field isolates, for example, A/Georgia/93. The North American field isolates are antigenically similar to the type strain, A/Beijing/92, but differ in their geographic site of isolation, and in their passage history in that they were passaged in mammalian cell culture rather than in eggs. In terms of the amino acid sequence of HA, however, the A/Beijing/92-like strains differed from the A/Beijing/89-like strains by only 11 point mutations (positions 133, 135, 145, 156, 157, 186, 190, 191, 193, 226, and 262) in the HA1 region. We therefore sought to determine whether combining the homotypic immune responses induced by HA DNA with cross-reactive CMI responses induced by NP and M1 DNA would provide a greater degree of protection against this antigenic drift variant. Immunization of ferrets with licensed vaccine containing the A/Beijing/89 strain, or with HA DNA from A/Beijing/89 or a Beijing-89-like field isolate (A/Hawaii/91) gave a reduction in viral shedding when ferrets were challenged with A/Georgia/93 (Figure 33). Ferrets that were immunized with a combination PNV containing NP, M1 and HA DNAs had significantly lower virus shedding than ferrets immunized with licensed product, or with HA DNA alone (Figure 34). In the case of the A/Hawaii/91 HA DNA combined with A/Beijing/89 NP and M1 DNAs, the resulting protection was not significantly different from the maximal protection provided by the homologous A/Georgia/93 HA DNA (Figure 35). Thus combining HA, NP and M1 DNA's gave improved protection against an antigenic drift variant compared with the licensed vaccine.

b. Effect of passage history of the vaccine antigen: Ferrets that had been immunized with a vaccine consisting of the HA DNA sequence derived from a U.S. field isolate that had been passed in MDCK cells in tissue culture (A/Hawaii/91) experienced less ($p = 0.021$ by two-way ANOVA) viral shedding than ferrets given the licensed killed virus vaccine containing the egg-passaged A/Beijing/89 strain, when challenged with the antigenically drifted strain A/Georgia/93 (Figure 33). In contrast, ferrets given HA DNA from A/Beijing/89 did not experience significantly different viral shedding ($p = 0.058$) after A/Georgia/93 challenge from ferrets given the licensed vaccine containing the identical virus. The egg and mammalian cell-grown strains differ by two point mutations in the HA1 region of HA (positions 186 and 193), both of which are located in antigenic site B, close to the apex of the HA monomer in a region thought to be important for the binding of HI and neutralizing antibodies. In some instances, the ability of some human influenza isolates to bind to chicken RBC is initially very low but is increased by successive passages in eggs, suggesting that the receptor binding region of the HA may undergo substantial selection by growth in avian cells. The effect of small sequence variations on the efficacy of HA-based influenza vaccines in laboratory animals underscores the potential importance of remaining as close as possible to the sequence of the wild-type virus in preparing such vaccines.

c. Nonhuman primates: Nonhuman primates are not commonly used for influenza challenge models due to their lack of a clinical response to infection. However, we have investigated the immunogenicity of the PNV vaccine combinations in nonhuman primates in comparison with the licensed killed virus vaccines. The antibody liters elicited by the combination PNV containing the HA and internal protein genes were at least equivalent to the licensed product in terms of HI antibody titer and duration of response (*vide supra*). African Green Monkeys that had been immunized with PNV responded to an HA PNV for an antigenic drift variant, showing that type-specific responses to PNV can be generated in previously immunized subjects. Monkeys that were immunized with PNV also responded to subsequent immunization with conventional killed-virus vaccine.

4. Conclusion: Polynucleotide vaccines against influenza are efficacious in laboratory animal models of influenza infection. Homologous protection can be achieved using DNA vectors encoding HA, most likely by an immunological mechanism analogous to that induced by the HA protein used in the current licensed influenza vaccine. Heterologous protection can be achieved against both antigenically shifted and drifted strains by also including DNA encoding conserved internal proteins of influenza. Combination of these approaches in a single immunization yields improved protection against antigenic drift variants in comparison with the currently licensed vaccine in the ferret model.

EXAMPLE 15

Vector V1R Preparation

In an effort to continue to optimize our basic vaccination vector, we prepared a derivative of V1Jns which was designated as V1R. The purpose for this vector construction was to obtain a minimum-sized vaccine vector, i.e., without unnecessary DNA sequences, which still retained the overall optimized heterologous gene expression characteristics and high plasmid yields that V1J and V1Jns afford. We determined from the literature as well as by experiment that (1) regions within the pUC backbone comprising the E. coli origin of replication could be removed without affecting plasmid yield from bacteria; (2) the 3'-region of the *kan*$^r$ gene following the kanamycin open reading frame could be removed if a bacterial terminator was inserted in its stead; and, (3) ~300 bp from the 3'- half of the BGH terminator could be removed without affecting its regulatory function (following the original KpnI restriction enzyme site within the BGH element).

V1R was constructed by using PCR to synthesize three segments of DNA from V1Jns representing the CMVintA promoter/BGH terminator, origin of replication, and kanamycin resistance elements, respectively. Restriction enzymes unique for each segment were added to each segment end using the PCR oligomers: SspI and XhoI for CMVintA/BGH; EcoRV and BamHI for the *kan* $^r$ gene; and, BclI and SalI for the *ori* $^r$. These enzyme sites were chosen because they allow directional ligation of each of the PCR-derived DNA segments with subsequent loss of each site: EcoRV and SspI leave blunt-ended DNAs which are compatible for ligation while BamHI and BclI leave complementary overhangs as do SalI and XhoI. After obtaining these segments by PCR each segment was digested with the appropriate restriction enzymes indicated above and then ligated together in a single reaction mixture containing all three DNA segments. The 5'-end of the *ori* $^r$ was designed to include the T2 rho independent terminator sequence that is normally found in this region so that it could provide termination information for the kanamycin resistance gene. The ligated product was confirmed by restriction enzyme digestion (>8 enzymes) as well as by DNA sequencing of the ligation junctions. DNA plasmid yields and heterologous expression using viral genes within V1 R appear similar to V1Jns. The net reduction in vector size achieved was 1346 bp (V1Jns = 4.86 kb; V1R = 3.52 kb), see figure 36, SEQ.ID:45:.

PCR oligomer sequences used to synthesize V1R (restriction enzyme sites are underlined and identified in brackets following sequence):

(1) 5'-GGT ACA AAT ATT GG CTA TTG GCC ATT GCA TAC G-3' [SspI], SEQ.ID:60:,
(2) 5'-CCA CAT CTC GAG GAA CCG GGT CAA TTC TTC AGC ACC-3' [XhoI], SEQ.ID:61:
(for CMVintA/BGH segment)
(3) 5'-GGT ACA GAT ATC GGA AAG CCA CGT TGT GTC TCA AAA TC-3'[EcoRV], SEQ.ID:62:
(4) 5'-CCA CAT GGA TCC G TAA TGC TCT GCC AGT GTT ACA ACC-3' [BamHI], SEQ.ID:63:
(for kanamycin resistance gene segment)
(5) 5'-GGT ACA TGA TCA CGT AGA AAA GAT CAA AGG ATC TTC TTG-3'[BclI], SEQ.ID:64:,
(6) 5'-CCA CAT GTC GAC CC GTA AAA AGG CCG CGT TGC TGG-3' [SalI], SEQ.ID:32:
(for E. coli origin of replication)

SEQUENCE LISTING

(1) GENERAL INFORMATION:

(i) APPLICANT:
(A) NAME: Merck & Co., Inc.
(B) STREET: P O Box 2000
(C) CITY: Rahway
(D) STATE: New Jersey
(E) COUNTRY: U.S.A.
(F) POSTAL CODE (ZIP): 07065

(i) APPLICANT:
(A) NAME: Vical Incorporated
(B) STREET: 9373 Towne Centre Drive, Suite 100
(C) CITY: San Diego
(D) STATE: California
(E) COUNTRY: U.S.A.
(F) POSTAL CODE (ZIP): 92121

(ii) TITLE OF INVENTION: Nucleic Acid Pharmaceuticals

(iii)NUMBER OF SEQUENCES: 64

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(v) PRIOR APPLICATION DATA:
(A) APPLICATION NUMBER: 08/032,383
(B) FILING DATE: 18.03.93

(v) PRIOR APPLICATION DATA:
(A) APPLICATION NUMBER: 08/089,985
(B) FILING DATE: 08.07.93

(2) INFORMATION FOR SEQUENCE ID NO: 1

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:1:

GTGTGCACCT CAAGCTGG 18

(2) INFORMATION FOR SEQ ID NO:2:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:2:

CCCTTTGAGA ATGTTGCACA TTC 23

(2) INFORMATION FOR SEQ ID NO:3:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:3:

GGTACAAGAT CTACCATGCT TCTAACCGAG GTC 33

(2) INFORMATION FOR SEQ ID NO:4:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: YES

(xi) SEQUENCE DESCRIPTION: SEQ ID NO:4:

CCACATAGAT CTTCACTTGA ACCGTTGCAT CTGCAC 36

(2) INFORMATION FOR SEQ ID NO:5:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:5:

CTATATAAGC AGAGCTCGTT TAG 23

(2) INFORMATION FOR SEQ ID NO:6:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: YES

(xi) SEQUENCE DESCRIPTION: SEQ ID NO:6:

GTAGCAAAGA TCTAAGGACG GTGACTGCAG 30

(2) INFORMATION FOR SEQ ID NO:7:

(i) SEQUENCE CHARACTERISTICS:
(A) LENGTH: 39 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:7:

GTATGTGTCT GAAAATGAGC GTGGAGATTG GGCTCGCAC 39

(2) INFORMATION FOR SEQ ID NO:8:

(i) SEQUENCE CHARACTERISTICS:
(A) LENGTH: 39 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: YES

(xi) SEQUENCE DESCRIPTION: SEQ ID NO:8:

GTGCGAGCCC AATCTCCACG CTCATTTTCA GACACATAC 39

(2) INFORMATION FOR SEQ ID NO:9:

(i) SEQUENCE CHARACTERISTICS:
(A) LENGTH: 9 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: internal

(xi) SEQUENCE DESCRIPTION: SEQ ID NO:9:

Thr Tyr Gln Arg Thr Arg Ala Leu Val
1               5

(2) INFORMATION FOR SEQ ID NO:10:

(i) SEQUENCE CHARACTERISTICS:
(A) LENGTH: 4432 base pairs
(B) TYPE: nucleic acid
(C) STRANDEDNESS: double
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:10:

```
TCGCGCGTTT CGGTGATGAC GGTGAAAACC TCTGACACAT GCAGCTCCCG GAGACGGTCA     60
CAGCTTGTCT GTAAGCGGAT GCCGGGAGCA GACAAGCCCG TCAGGGCGCG TCAGCGGGTG    120
TTGGCGGGTG TCGGGGCTGG CTTAACTATG CGGCATCAGA GCAGATTGTA CTGAGAGTGC    180
ACCATATGCG GTGTGAAATA CCGCACAGAT GCGTAAGGAG AAAATACCGC ATCAGATTGG    240
CTATTGGCCA TTGCATACGT TGTATCCATA TCATAATATG TACATTTATA TTGGCTCATG    300
TCCAACATTA CCGCCATGTT GACATTGATT ATTGACTAGT TATTAATAGT AATCAATTAC    360
GGGGTCATTA GTTCATAGCC CATATATGGA GTTCCGCGTT ACATAACTTA CGGTAAATGG    420
CCCGCCTGGC TGACCGCCCA ACGACCCCCG CCCATTGACG TCAATAATGA CGTATGTTCC    480
CATAGTAACG CCAATAGGGA CTTTCCATTG ACGTCAATGG GTGGAGTATT TACGGTAAAC    540
TGCCCACTTG GCAGTACATC AAGTGTATCA TATGCCAAGT ACGCCCCCTA TTGACGTCAA    600
TGACGGTAAA TGGCCCGCCT GGCATTATGC CCAGTACATG ACCTTATGGG ACTTTCCTAC    660
TTGGCAGTAC ATCTACGTAT TAGTCATCGC TATTACCATG GTGATGCGGT TTTGGCAGTA    720
CATCAATGGG CGTGGATAGC GGTTTGACTC ACGGGGATTT CCAAGTCTCC ACCCCATTGA    780
CGTCAATGGG AGTTTGTTTT GGCACCAAAA TCAACGGGAC TTTCCAAAAT GTCGTAACAA    840
CTCCGCCCCA TTGACGCAAA TGGGCGGTAG GCGTGTACGG TGGGAGGTCT ATATAAGCAG    900
AGCTCGTTTA GTGAACCGTC AGATCGCCTG GAGACGCCAT CCACGCTGTT TTGACCTCCA    960
TAGAAGACAC CGGGACCGAT CCAGCCTCCG CGGCCGGGAA CGGTGCATTG GAACGCGGAT   1020
TCCCCGTGCC AAGAGTGACG TAAGTACCGC CTATAGAGTC TATAGGCCCA CCCCCTTGGC   1080
TTCTTATGCA TGCTATACTG TTTTTGGCTT GGGGTCTATA CACCCCCGCT TCCTCATGTT   1140
ATAGGTGATG GTATAGCTTA GCCTATAGGT GTGGGTTATT GACCATTATT GACCACTCCC   1200
CTATTGGTGA CGATACTTTC CATTACTAAT CCATAACATG GCTCTTTGCC ACAACTCTCT   1260
TTATTGGCTA TATGCCAATA CACTGTCCTT CAGAGACTGA CACGGACTCT GTATTTTTAC   1320
AGGATGGGGT CTCATTTATT ATTTACAAAT TCACATATAC AACACCACCG TCCCCAGTGC   1380
CCGCAGTTTT TATTAAACAT AACGTGGGAT CTCCACGCGA ATCTCGGGTA CGTGTTCCGG   1440
ACATGGGCTC TTCTCCGGTA GCGGCGGAGC TTCTACATCC GAGCCCTGCT CCCATGCCTC   1500
CAGCGACTCA TGGTCGCTCG GCAGCTCCTT GCTCCTAACA GTGGAGGCCA GACTTAGGCA   1560
CAGCACGATG CCCACCACCA CCAGTGTGCC GCACAAGGCC GTGGCGGTAG GGTATGTGTC   1620
TGAAAATGAG CTCGGGGAGC GGGCTTGCAC CGCTGACGCA TTTGGAAGAC TTAAGGCAGC   1680
```

GGCAGAAGAA GATGCAGGCA GCTGAGTTGT TGTGTTCTGA TAAGAGTCAG AGGTAACTCC 1740
CGTTGCGGTG CTGTTAACGG TGGAGGGCAG TGTAGTCTGA GCAGTACTCG TTGCTGCCGC 1800
GCGCGCCACC AGACATAATA GCTGACAGAC TAACAGACTG TTCCTTTCCA TGGGTCTTTT 1860
CTGCAGTCAC CGTCCTTAGA TCTGCTGTGC CTTCTAGTTG CCAGCCATCT GTTGTTTGCC 1920
CCTCCCCCGT GCCTTCCTTG ACCCTGGAAG GTGCCACTCC CACTGTCCTT TCCTAATAAA 1980
ATGAGGAAAT TGCATCGCAT TGTCTGAGTA GGTGTCATTC TATTCTGGGG GGTGGGGTGG 2040
GGCAGCACAG CAAGGGGGAG GATTGGGAAG ACAATAGCAG GCATGCTGGG GATGCGGTGG 2100
GCTCTATGGG TACCCAGGTG CTGAAGAATT GACCCGGTTC CTCCTGGGCC AGAAAGAAGC 2160
AGGCACATCC CCTTCTCTGT GACACACCCT GTCCACGCCC CTGGTTCTTA GTTCCAGCCC 2220
CACTCATAGG ACACTCATAG CTCAGGAGGG CTCCGCCTTC AATCCCACCC GCTAAAGTAC 2280
TTGGAGCGGT CTCTCCCTCC CTCATCAGCC CACCAAACCA AACCTAGCCT CCAAGAGTGG 2340
GAAGAAATTA AAGCAAGATA GGCTATTAAG TGCAGAGGGA GAGAAAATGC CTCCAACATG 2400
TGAGGAAGTA ATGAGAGAAA TCATAGAATT TCTTCCGCTT CCTCGCTCAC TGACTCGCTG 2460
CGCTCGGTCG TTCGGCTGCG GCGAGCGGTA TCAGCTCACT CAAAGGCGGT AATACGGTTA 2520
TCCACAGAAT CAGGGGATAA CGCAGGAAAG AACATGTGAG CAAAAGGCCA GCAAAAGGCC 2580
AGGAACCGTA AAAAGGCCGC GTTGCTGGCG TTTTTCCATA GGCTCCGCCC CCCTGACGAG 2640
CATCACAAAA ATCGACGCTC AAGTCAGAGG TGGCGAAACC CGACAGGACT ATAAAGATAC 2700
CAGGCGTTTC CCCCTGGAAG CTCCCTCGTG CGCTCTCCTG TTCCGACCCT GGCGCTTACC 2760
GGATACCTGT CCGCCTTTCT CCCTTCGGGA AGCGTGGCGC TTTCTCAATG CTCACGCTGT 2820
AGGTATCTCA GTTCGGTGTA GGTCGTTCGC TCCAAGCTGG GCTGTGTGCA CGAACCCCCC 2880
GTTCAGCCCG ACCGCTGCGC CTTATCCGGT AACTATCGTC TTGAGTCCAA CCCGGTAAGA 2940
CACGACTTAT CGCCACTGGC AGCAGCCACT GGTAACAGGA TTAGCAGAGC GAGGTATGTA 3000
GGCGGTGCTA CAGAGTTCTT GAAGTGGTGG CCTAACTACG GCTACACTAG AAGGACAGTA 3060
TTTGGTATCT GCGCTCTGCT GAAGCCAGTT ACCTTCGGAA AAAGAGTTGG TAGCTCTTGA 3120
TCCGGCAAAC AAACCACCGC TGGTAGCGGT GGTTTTTTTG TTTGCAAGCA GCAGATTACG 3180
CGCAGAAAAA AAGGATCTCA AGAAGATCCT TTGATCTTTT CTACGGGGTC TGACGCTCAG 3240
TGGAACGAAA ACTCACGTTA AGGGATTTTG GTCATGAGAT TATCAAAAAG GATCTTCACC 3300
TAGATCCTTT TAAATTAAAA ATGAAGTTTT AAATCAATCT AAAGTATATA TGAGTAAACT 3360
TGGTCTGACA GTTACCAATG CTTAATCAGT GAGGCACCTA TCTCAGCGAT CTGTCTATTT 3420
CGTTCATCCA TAGTTGCCTG ACTCCCCGTC GTGTAGATAA CTACGATACG GGAGGGCTTA 3480
CCATCTGGCC CCAGTGCTGC AATGATACCG CGAGACCCAC GCTCACCGGC TCCAGATTTA 3540

```
TCAGCAATAA ACCAGCCAGC CGGAAGGGCC GAGCGCAGAA GTGGTCCTGC AACTTTATCC    3600
GCCTCCATCC AGTCTATTAA TTGTTGCCGG GAAGCTAGAG TAAGTAGTTC GCCAGTTAAT    3660
AGTTTGCGCA ACGTTGTTGC CATTGCTACA GGCATCGTGG TGTCACGCTC GTCGTTTGGT    3720
ATGGCTTCAT TCAGCTCCGG TTCCCAACGA TCAAGGCGAG TTACATGATC CCCCATGTTG    3780
TGCAAAAAAG CGGTTAGCTC CTTCGGTCCT CCGATCGTTG TCAGAAGTAA GTTGGCCGCA    3840
GTGTTATCAC TCATGGTTAT GGCAGCACTG CATAATTCTC TTACTGTCAT GCCATCCGTA    3900
AGATGCTTTT CTGTGACTGG TGAGTACTCA ACCAAGTCAT TCTGAGAATA GTGTATGCGG    3960
CGACCGAGTT GCTCTTGCCC GGCGTCAATA CGGGATAATA CCGCGCCACA TAGCAGAACT    4020
TTAAAAGTGC TCATCATTGG AAAACGTTCT TCGGGGCGAA AACTCTCAAG GATCTTACCG    4080
CTGTTGAGAT CCAGTTCGAT GTAACCCACT CGTGCACCCA ACTGATCTTC AGCATCTTTT    4140
ACTTTCACCA GCGTTTCTGG GTGAGCAAAA ACAGGAAGGC AAAATGCCGC AAAAAAGGGA    4200
ATAAGGGCGA CACGGAAATG TTGAATACTC ATACTCTTCC TTTTTCAATA TTATTGAAGC    4260
ATTTATCAGG GTTATTGTCT CATGAGCGGA TACATATTTG AATGTATTTA GAAAAATAAA    4320
CAAATAGGGG TTCCGCGCAC ATTTCCCCGA AAAGTGCCAC CTGACGTCTA AGAAACCATT    4380
ATTATCATGA CATTAACCTA TAAAAATAGG CGTATCACGA GGCCCTTTCG TC            4432
```

(2) INFORMATION FOR SEQ ID NO:11:

(i) SEQUENCE CHARACTERISTICS:
- (A) LENGTH: 2196 base pairs
- (B) TYPE: nucleic acid
- (C) STRANDEDNESS: double
- (D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:11:

```
ATTGGCTATT GGCCATTGCA TACGTTGTAT CCATATCATA ATATGTACAT TTATATTGGC      60
TCATGTCCAA CATTACCGCC ATGTTGACAT TGATTATTGA CTAGTTATTA ATAGTAATCA     120
ATTACGGGGT CATTAGTTCA TAGCCCATAT ATGGAGTTCC GCGTTACATA ACTTACGGTA     180
AATGGCCCGC CTGGCTGACC GCCCAACGAC CCCCGCCCAT TGACGTCAAT AATGACGTAT     240
GTTCCCATAG TAACGCCAAT AGGGACTTTC CATTGACGTC AATGGGTGGA GTATTTACGG     300
TAAACTGCCC ACTTGGCAGT ACATCAAGTG TATCATATGC CAAGTACGCC CCCTATTGAC     360
GTCAATGACG GTAAATGGCC CGCCTGGCAT TATGCCCAGT ACATGACCTT ATGGGACTTT     420
```

```
CCTACTTGGC AGTACATCTA CGTATTAGTC ATCGCTATTA CCATGGTGAT GCGGTTTTGG    480
CAGTACATCA ATGGGCGTGG ATAGCGGTTT GACTCACGGG GATTTCCAAG TCTCCACCCC    540
ATTGACGTCA ATGGGAGTTT GTTTTGGCAC CAAAATCAAC GGGACTTTCC AAAATGTCGT    600
AACAACTCCG CCCCATTGAC GCAAATGGGC GGTAGGCGTG TACGGTGGGA GGTCTATATA    660
AGCAGAGCTC GTTTAGTGAA CCGTCAGATC GCCTGGAGAC GCCATCCACG CTGTTTTGAC    720
CTCCATAGAA GACACCGGGA CCGATCCAGC CTCCGCGGCC GGGAACGGTG CATTGGAACG    780
CGGATTCCCC GTGCCAAGAG TGACGTAAGT ACCGCCTATA GAGTCTATAG GCCCACCCCC    840
TTGGCTTCTT ATGCATGCTA TACTGTTTTT GGCTTGGGGT CTATACACCC CCGCTTCCTC    900
ATGTTATAGG TGATGGTATA GCTTAGCCTA TAGGTGTGGG TTATTGACCA TTATTGACCA    960
CTCCCCTATT GGTGACGATA CTTTCCATTA CTAATCCATA ACATGGCTCT TTGCCACAAC   1020
TCTCTTTATT GGCTATATGC CAATACACTG TCCTTCAGAG ACTGACACGG ACTCTGTATT   1080
TTTACAGGAT GGGGTCTCAT TTATTATTTA CAAATTCACA TATACAACAC CACCGTCCCC   1140
AGTGCCCGCA GTTTTTATTA AACATAACGT GGGATCTCCA CGCGAATCTC GGGTACGTGT   1200
TCCGGACATG GGCTCTTCTC CGGTAGCGGC GGAGCTTCTA CATCCGAGCC CTGCTCCCAT   1260
GCCTCCAGCG ACTCATGGTC GCTCGGCAGC TCCTTGCTCC TAACAGTGGA GGCCAGACTT   1320
AGGCACAGCA CGATGCCCAC CACCACCAGT GTGCCGCACA AGGCCGTGGC GGTAGGGTAT   1380
GTGTCTGAAA ATGAGCTCGG GGAGCGGGCT TGCACCGCTG ACGCATTTGG AAGACTTAAG   1440
GCAGCGGCAG AAGAAGATGC AGGCAGCTGA GTTGTTGTGT TCTGATAAGA GTCAGAGGTA   1500
ACTCCCGTTG CGGTGCTGTT AACGGTGGAG GGCAGTGTAG TCTGAGCAGT ACTCGTTGCT   1560
GCCGCGCGCG CCACCAGACA TAATAGCTGA CAGACTAACA GACTGTTCCT TTCCATGGGT   1620
CTTTTCTGCA GTCACCGTCC TTAGATCTGC TGTGCCTTCT AGTTGCCAGC CATCTGTTGT   1680
TTGCCCCTCC CCCGTGCCTT CCTTGACCCT GGAAGGTGCC ACTCCCACTG TCCTTTCCTA   1740
ATAAAATGAG GAAATTGCAT CGCATTGTCT GAGTAGGTGT CATTCTATTC TGGGGGGTGG   1800
GGTGGGGCAG CACAGCAAGG GGGAGGATTG GGAAGACAAT AGCAGGCATG CTGGGGATGC   1860
GGTGGGCTCT ATGGGTACCC AGGTGCTGAA GAATTGACCC GGTTCCTCCT GGGCCAGAAA   1920
GAAGCAGGCA CATCCCCTTC TCTGTGACAC ACCCTGTCCA CGCCCCTGGT TCTTAGTTCC   1980
AGCCCCACTC ATAGGACACT CATAGCTCAG GAGGGCTCCG CCTTCAATCC CACCCGCTAA   2040
AGTACTTGGA GCGGTCTCTC CCTCCCTCAT CAGCCCACCA AACCAAACCT AGCCTCCAAG   2100
AGTGGGAAGA AATTAAAGCA AGATAGGCTA TTAAGTGCAG AGGGAGAGAA AATGCCTCCA   2160
ACATGTGAGG AAGTAATGAG AGAAATCATA GAATTC                             2196
```

(2) INFORMATION FOR SEQ ID NO:12:

(i) SEQUENCE CHARACTERISTICS:
(A) LENGTH: 71 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:12:

GTCACCGTCC TTAGATCAAT TCCAGCAAAA GCAGGGTAGA TAATCACTCA CTGAGTGACA 60

TCAAAATCAT G 71

(2) INFORMATION FOR SEQ ID NO:13:

(i) SEQUENCE CHARACTERISTICS:
(A) LENGTH: 117 base pairs
(B) TYPE: nucleic acid
(C) STRANDEDNESS: double
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:13:

ACCGTCCTTA GATCAGCTTG GCAAAAGCAG GCAAACCATT TGAATGGATG TCAATCCGAC 60

CTTACTTTTC TTAAAAGTGC CAGCACAAAA TGCTATAAGC ACAACTTTCC CTTATAC 117

(2) INFORMATION FOR SEQ ID NO:14:

(i) SEQUENCE CHARACTERISTICS:
(A) LENGTH: 136 base pairs
(B) TYPE: nucleic acid
(C) STRANDEDNESS: double
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:14:

GTCACCGTCC TTAGATCAAT TCCAGCAAAA GCAGGGTGAC AAAAACATAA TGGATCCAAA 60

CACTGTGTCA AGCTTTCAGG TAGATTGCTT TCTTTGGCAT GTCCGCAAAC GAGTTGCAGA 120

CCAAGAACTA GGTGAT 136

(2) INFORMATION FOR SEQ ID NO:15:

(i) SEQUENCE CHARACTERISTICS:
(A) LENGTH: 152 base pairs
(B) TYPE: nucleic acid
(C) STRANDEDNESS: double
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:15:

TCTGCAGTCA CCGTCCTTAG ATCAGCTTGG AGCAAAAGCA GGGGAAAATA AAAACAACCA 60

AAATGAAGGC AAACCTACTG GTCCTGTTAA GTGCACTTGC AGCTGCAGAT GCAGACACAA 120

TATGTATAGG CTACCATGCG AACAATTCAA CC 152

(2) INFORMATION FOR SEQ ID NO:16:

(i) SEQUENCE CHARACTERISTICS:
(A) LENGTH: 162 base pairs
(B) TYPE: nucleic acid
(C) STRANDEDNESS: double
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:16:

TTTTCTGCAG TCACCGTCCT TAGATCCCGA ATTCCAGCAA AAGCAGGTCA ATTATATTCA 60

ATATGGAAAG AATAAAAGAA CTAAGAAATC TAATGTCGCA GTCTGCCACC CCGGAGATAC 120

TCACAAAAAC CACCGTGGAC CATATGGCCA TAATCAAGAA GT 162

(2) INFORMATION FOR SEQ ID NO:17:

(i) SEQUENCE CHARACTERISTICS:
(A) LENGTH: 122 base pairs
(B) TYPE: nucleic acid
(C) STRANDEDNESS: double
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:17:

GTCACCGTCC TTAGATCTAC CATGAGTCTT CTAACCGAGG TCGAAACGTA CGTACTCTCT 60

ATCATCCCGT CAGGCCCCCT CAAAGCCGAG ATCGCACAGA GACTTGAAGA GTTGACGGAA 120

GA 122

(2) INFORMATION FOR SEQ ID NO:18:

(i) SEQUENCE CHARACTERISTICS:
(A) LENGTH: 4864 base pairs
(B) TYPE: nucleic acid
(C) STRANDEDNESS: double
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:18:

TCGCGCGTTT CGGTGATGAC GGTGAAAACC TCTGACACAT GCAGCTCCCG GAGACGGTCA 60

CAGCTTGTCT GTAAGCGGAT GCCGGGAGCA GACAAGCCCG TCAGGGCGCG TCAGCGGGTG 120

TTGGCGGGTG TCGGGGCTGG CTTAACTATG CGGCATCAGA GCAGATTGTA CTGAGAGTGC 180

ACCATATGCG GTGTGAAATA CCGCACAGAT GCGTAAGGAG AAAATACCGC ATCAGATTGG 240

CTATTGGCCA TTGCATACGT TGTATCCATA TCATAATATG TACATTTATA TTGGCTCATG 300

TCCAACATTA CCGCCATGTT GACATTGATT ATTGACTAGT TATTAATAGT AATCAATTAC 360

GGGGTCATTA GTTCATAGCC CATATATGGA GTTCCGCGTT ACATAACTTA CGGTAAATGG 420

CCCGCCTGGC TGACCGCCCA ACGACCCCCG CCCATTGACG TCAATAATGA CGTATGTTCC 480

CATAGTAACG CCAATAGGGA CTTTCCATTG ACGTCAATGG GTGGAGTATT TACGGTAAAC 540

TGCCCACTTG GCAGTACATC AAGTGTATCA TATGCCAAGT ACGCCCCCTA TTGACGTCAA 600

TGACGGTAAA TGGCCCGCCT GGCATTATGC CCAGTACATG ACCTTATGGG ACTTTCCTAC 660

TTGGCAGTAC ATCTACGTAT TAGTCATCGC TATTACCATG GTGATGCGGT TTTGGCAGTA 720

CATCAATGGG CGTGGATAGC GGTTTGACTC ACGGGGATTT CCAAGTCTCC ACCCCATTGA 780

CGTCAATGGG AGTTTGTTTT GGCACCAAAA TCAACGGGAC TTTCCAAAAT GTCGTAACAA 840

CTCCGCCCCA TTGACGCAAA TGGGCGGTAG GCGTGTACGG TGGGAGGTCT ATATAAGCAG 900

AGCTCGTTTA GTGAACCGTC AGATCGCCTG GAGACGCCAT CCACGCTGTT TTGACCTCCA 960
TAGAAGACAC CGGGACCGAT CCAGCCTCCG CGGCCGGGAA CGGTGCATTG GAACGCGGAT 1020
TCCCCGTGCC AAGAGTGACG TAAGTACCGC CTATAGAGTC TATAGGCCCA CCCCCTTGGC 1080
TTCTTATGCA TGCTATACTG TTTTTGGCTT GGGGTCTATA CACCCCCGCT TCCTCATGTT 1140
ATAGGTGATG GTATAGCTTA GCCTATAGGT GTGGGTTATT GACCATTATT GACCACTCCC 1200
CTATTGGTGA CGATACTTTC CATTACTAAT CCATAACATG GCTCTTTGCC ACAACTCTCT 1260
TTATTGGCTA TATGCCAATA CACTGTCCTT CAGAGACTGA CACGGACTCT GTATTTTTAC 1320
AGGATGGGGT CTCATTTATT ATTTACAAAT TCACATATAC AACACCACCG TCCCCAGTGC 1380
CCGCAGTTTT TATTAAACAT AACGTGGGAT CTCCACGCGA ATCTCGGGTA CGTGTTCCGG 1440
ACATGGGCTC TTCTCCGGTA GCGGCGGAGC TTCTACATCC GAGCCCTGCT CCCATGCCTC 1500
CAGCGACTCA TGGTCGCTCG GCAGCTCCTT GCTCCTAACA GTGGAGGCCA GACTTAGGCA 1560
CAGCACGATG CCCACCACCA CCAGTGTGCC GCACAAGGCC GTGGCGGTAG GGTATGTGTC 1620
TGAAAATGAG CTCGGGGAGC GGGCTTGCAC CGCTGACGCA TTTGGAAGAC TTAAGGCAGC 1680
GGCAGAAGAA GATGCAGGCA GCTGAGTTGT TGTGTTCTGA TAAGAGTCAG AGGTAACTCC 1740
CGTTGCGGTG CTGTTAACGG TGGAGGGCAG TGTAGTCTGA GCAGTACTCG TTGCTGCCGC 1800
GCGCGCCACC AGACATAATA GCTGACAGAC TAACAGACTG TTCCTTTCCA TGGGTCTTTT 1860
CTGCAGTCAC CGTCCTTAGA TCTGCTGTGC CTTCTAGTTG CCAGCCATCT GTTGTTTGCC 1920
CCTCCCCCGT GCCTTCCTTG ACCCTGGAAG GTGCCACTCC CACTGTCCTT TCCTAATAAA 1980
ATGAGGAAAT TGCATCGCAT TGTCTGAGTA GGTGTCATTC TATTCTGGGG GGTGGGGTGG 2040
GGCAGCACAG CAAGGGGGAG GATTGGGAAG ACAATAGCAG GCATGCTGGG GATGCGGTGG 2100
GCTCTATGGG TACCCAGGTG CTGAAGAATT GACCCGGTTC CTCCTGGGCC AGAAAGAAGC 2160
AGGCACATCC CCTTCTCTGT GACACACCCT GTCCACGCCC CTGGTTCTTA GTTCCAGCCC 2220
CACTCATAGG ACACTCATAG CTCAGGAGGG CTCCGCCTTC AATCCCACCC GCTAAAGTAC 2280
TTGGAGCGGT CTCTCCCTCC CTCATCAGCC CACCAAACCA AACCTAGCCT CCAAGAGTGG 2340
GAAGAAATTA AAGCAAGATA GGCTATTAAG TGCAGAGGGA GAGAAAATGC CTCCAACATG 2400
TGAGGAAGTA ATGAGAGAAA TCATAGAATT TCTTCCGCTT CCTCGCTCAC TGACTCGCTG 2460
CGCTCGGTCG TTCGGCTGCG GCGAGCGGTA TCAGCTCACT CAAAGGCGGT AATACGGTTA 2520
TCCACAGAAT CAGGGGATAA CGCAGGAAAG AACATGTGAG CAAAAGGCCA GCAAAAGGCC 2580
AGGAACCGTA AAAAGGCCGC GTTGCTGGCG TTTTTCCATA GGCTCCGCCC CCCTGACGAG 2640
CATCACAAAA ATCGACGCTC AAGTCAGAGG TGGCGAAACC CGACAGGACT ATAAAGATAC 2700
CAGGCGTTTC CCCCTGGAAG CTCCCTCGTG CGCTCTCCTG TTCCGACCCT GCCGCTTACC 2760

GGATACCTGT CCGCCTTTCT CCCTTCGGGA AGCGTGGCGC TTTCTCAATG CTCACGCTGT 2820

AGGTATCTCA GTTCGGTGTA GGTCGTTCGC TCCAAGCTGG GCTGTGTGCA CGAACCCCCC 2880

GTTCAGCCCG ACCGCTGCGC CTTATCCGGT AACTATCGTC TTGAGTCCAA CCCGGTAAGA 2940

CACGACTTAT CGCCACTGGC AGCAGCCACT GGTAACAGGA TTAGCAGAGC GAGGTATGTA 3000

GGCGGTGCTA CAGAGTTCTT GAAGTGGTGG CCTAACTACG GCTACACTAG AAGGACAGTA 3060

TTTGGTATCT GCGCTCTGCT GAAGCCAGTT ACCTTCGGAA AAAGAGTTGG TAGCTCTTGA 3120

TCCGGCAAAC AAACCACCGC TGGTAGCGGT GGTTTTTTTG TTTGCAAGCA GCAGATTACG 3180

CGCAGAAAAA AAGGATCTCA AGAAGATCCT TTGATCTTTT CTACGGGGTC TGACGCTCAG 3240

TGGAACGAAA ACTCACGTTA AGGGATTTTG GTCATGAGAT TATCAAAAAG GATCTTCACC 3300

TAGATCCTTT TAAATTAAAA ATGAAGTTTT AAATCAATCT AAAGTATATA TGAGTAAACT 3360

TGGTCTGACA GTTACCAATG CTTAATCAGT GAGGCACCTA TCTCAGCGAT CTGTCTATTT 3420

CGTTCATCCA TAGTTGCCTG ACTCCGGGGG GGGGGGGCGC TGAGGTCTGC CTCGTGAAGA 3480

AGGTGTTGCT GACTCATACC AGGCCTGAAT CGCCCCATCA TCCAGCCAGA AAGTGAGGGA 3540

GCCACGGTTG ATGAGAGCTT TGTTGTAGGT GGACCAGTTG GTGATTTTGA ACTTTTGCTT 3600

TGCCACGGAA CGGTCTGCGT TGTCGGGAAG ATGCGTGATC TGATCCTTCA ACTCAGCAAA 3660

AGTTCGATTT ATTCAACAAA GCCGCCGTCC CGTCAAGTCA GCGTAATGCT CTGCCAGTGT 3720

TACAACCAAT TAACCAATTC TGATTAGAAA AACTCATCGA GCATCAAATG AAACTGCAAT 3780

TTATTCATAT CAGGATTATC AATACCATAT TTTTGAAAAA GCCGTTTCTG TAATGAAGGA 3840

GAAAACTCAC CGAGGCAGTT CCATAGGATG GCAAGATCCT GGTATCGGTC TGCGATTCCG 3900

ACTCGTCCAA CATCAATACA ACCTATTAAT TTCCCCTCGT CAAAAATAAG GTTATCAAGT 3960

GAGAAATCAC CATGAGTGAC GACTGAATCC GGTGAGAATG GCAAAAGCTT ATGCATTTCT 4020

TTCCAGACTT GTTCAACAGG CCAGCCATTA CGCTCGTCAT CAAAATCACT CGCATCAACC 4080

AAACCGTTAT TCATTCGTGA TTGCGCCTGA GCGAGACGAA ATACGCGATC GCTGTTAAAA 4140

GGACAATTAC AAACAGGAAT CGAATGCAAC CGGCGCAGGA ACACTGCCAG CGCATCAACA 4200

ATATTTTCAC CTGAATCAGG ATATTCTTCT AATACCTGGA ATGCTGTTTT CCCGGGGATC 4260

GCAGTGGTGA GTAACCATGC ATCATCAGGA GTACGGATAA AATGCTTGAT GGTCGGAAGA 4320

GGCATAAATT CCGTCAGCCA GTTTAGTCTG ACCATCTCAT CTGTAACATC ATTGGCAACG 4380

CTACCTTTGC CATGTTTCAG AAACAACTCT GGCGCATCGG GCTTCCCATA CAATCGATAG 4440

ATTGTCGCAC CTGATTGCCC GACATTATCG CGAGCCCATT TATACCCATA TAAATCAGCA 4500

TCCATGTTGG AATTTAATCG CGGCCTCGAG CAAGACGTTT CCCGTTGAAT ATGGCTCATA 4560

ACACCCCTTG TATTACTGTT TATGTAAGCA GACAGTTTTA TTGTTCATGA TGATATATTT 4620

TTATCTTGTG CAATGTAACA TCAGAGATTT TGAGACACAA CGTGGCTTTC CCCCCCCCCC 4680

CATTATTGAA GCATTTATCA GGGTTATTGT CTCATGAGCG GATACATATT TGAATGTATT 4740

TAGAAAAATA AACAAATAGG GGTTCCGCGC ACATTTCCCC GAAAAGTGCC ACCTGACGTC 4800

TAAGAAACCA TTATTATCAT GACATTAACC TATAAAAATA GGCGTATCAC GAGGCCCTTT 4860

CGTC 4864

(2) INFORMATION FOR SEQ ID NO:19:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:19:

AGCAGAAGCA GAGCA 15

(2) INFORMATION FOR SEQ ID NO:20:

(i) SEQUENCE CHARACTERISTICS:
(A) LENGTH: 119 base pairs
(B) TYPE: nucleic acid
(C) STRANDEDNESS: double
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:20:

TCACCGTCCT TAGATCAAGC AGGGTTAATA ATCACTCACT GAGTGACATC AAAATCATGG 60

CGTCCCAAGG CACCAAACGG TCTTATGAAC AGATGGAAAC TGATGGGGAA CGCCAGATT 119

(2) INFORMATION FOR SEQ ID NO:21:

(i) SEQUENCE CHARACTERISTICS:
(A) LENGTH: 67 base pairs
(B) TYPE: nucleic acid
(C) STRANDEDNESS: double
(D) TOPOLOGY: both

(ii) MOLECULE TYPE: cDNA

(iii) HYPOTHETICAL: NO

(iv) ANTI-SENSE: YES

(xi) SEQUENCE DESCRIPTION: SEQ ID NO:21:

GAGGGGCAAA CAACAGATGG CTGGCAACTA GAAGGCACAG CAGATATTTT TTCCTTAATT 60

GTCGTAC 67

(2) INFORMATION FOR SEQ ID NO:22:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:22:

AGCAGAAGCA CGCAC 15

(2) INFORMATION FOR SEQ ID NO:23:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:23:

AGCAGAAGCA CAGCA 15

(2) INFORMATION FOR SEQ ID NO:24:

(i) SEQUENCE CHARACTERISTICS:
(A) LENGTH: 33 base pairs
(B) TYPE: nucleic acid
(C) STRANDEDNESS: double
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:24:

CCTTAGATCG GAAATAAAAA CAACCAAAAT GAA 33

(2) INFORMATION FOR SEQ ID NO:25:

(i) SEQUENCE CHARACTERISTICS:
(A) LENGTH: 36 base pairs
(B) TYPE: nucleic acid
(C) STRANDEDNESS: double
(D) TOPOLOGY: both

(ii) MOLECULE TYPE: cDNA

(iii) HYPOTHETICAL: NO

(iv) ANTI-SENSE: YES

(xi) SEQUENCE DESCRIPTION: SEQ ID NO:25:

GCAGATCCTT ATATTTCTGA AATTCTGGTC TCAGAT 36

(2) INFORMATION FOR SEQ ID NO:26:

(i) SEQUENCE CHARACTERISTICS:
(A) LENGTH: 102 base pairs
(B) TYPE: nucleic acid
(C) STRANDEDNESS: double
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:26:

ACCGTCCTTA GATCCAGAAG CAGAGCATTT TCTAATATCC ACAAAATGAA GGCAATAATT 60

GTACTACTCA TGGTAGTAAC ATCCAACGCA GATCGAATCT GC 102

(2) INFORMATION FOR SEQ ID NO:27:

(i) SEQUENCE CHARACTERISTICS:
(A) LENGTH: 42 base pairs
(B) TYPE: nucleic acid
(C) STRANDEDNESS: double
(D) TOPOLOGY: both

(ii) MOLECULE TYPE: cDNA

(iii) HYPOTHETICAL: NO

(iv) ANTI-SENSE: YES

(xi) SEQUENCE DESCRIPTION: SEQ ID NO:27:

GGCACAGCAG ATCTTTCAAT AACGTTTCTT TGTAATGGTA AC 42

(2) INFORMATION FOR SEQ ID NO:28:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:28:

CTAACAGACT GTTCCTTTCC ATG 23

(2) INFORMATION FOR SEQ ID NO:29:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: YES

(xi) SEQUENCE DESCRIPTION: SEQ ID NO:29:

GGAGTGGCAC CTTCCAGG 18

(2) INFORMATION FOR SEQ ID NO:30:

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:30:

AGCAAAAGCA GG 12

(2) INFORMATION FOR SEQ ID NO:31:

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:31:

AGCAGAAGCG GAGC 14

(2) INFORMATION FOR SEQ ID NO:32:

(i) SEQUENCE CHARACTERISTICS:
(A) LENGTH: 35 base pairs
(B) TYPE: nucleic acid
(C) STRANDEDNESS: both
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:32:

CCACATGTCG ACCCGTAAAA AGGCCGCGTT GCTGG 35

(2) INFORMATION FOR SEQ ID NO:33:

(i) SEQUENCE CHARACTERISTICS:
(A) LENGTH: 33 base pairs
(B) TYPE: nucleic acid
(C) STRANDEDNESS: both
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:33:

GGTACAACCA TGAAGACTAT CATTGCTTTG AGC 33

(2) INFORMATION FOR SEQ ID NO:34:

(i) SEQUENCE CHARACTERISTICS:
(A) LENGTH: 37 base pairs
(B) TYPE: nucleic acid
(C) STRANDEDNESS: both
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:34:

CCACATAGAT CTTCAAATGC AAATGTTGCA CCTAATG 37

(2) INFORMATION FOR SEQ ID NO:35:

(i) SEQUENCE CHARACTERISTICS:
(A) LENGTH: 38 base pairs
(B) TYPE: nucleic acid
(C) STRANDEDNESS: both
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:35:

GGTACAACCA TGAAAGCAAA ACTACTAGTC CTGTTATG 38

(2) INFORMATION FOR SEQ ID NO:36:

(i) SEQUENCE CHARACTERISTICS:
(A) LENGTH: 32 base pairs
(B) TYPE: nucleic acid
(C) STRANDEDNESS: both
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:36:

CCACATTCAG ATGCATATTC TACACTGCAA AG 32

(2) INFORMATION FOR SEQ ID NO:37:

(i) SEQUENCE CHARACTERISTICS:
(A) LENGTH: 36 base pairs
(B) TYPE: nucleic acid
(C) STRANDEDNESS: both
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:37:

GGTACAACCA TGAAGGCAAT AATTGTACTA CTCATG 36

(2) INFORMATION FOR SEQ ID NO:38:

(i) SEQUENCE CHARACTERISTICS:
(A) LENGTH: 36 base pairs
(B) TYPE: nucleic acid
(C) STRANDEDNESS: both
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:38:

CCACATTTAT AGACAGATGG AGCAAGAAAC ATTGTC 36

(2) INFORMATION FOR SEQ ID NO:39:

(i) SEQUENCE CHARACTERISTICS:
(A) LENGTH: 33 base pairs
(B) TYPE: nucleic acid
(C) STRANDEDNESS: both
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:39:

GGTACAAGAT CTACCATGCT TCTAACCGAG GTC 33

(2) INFORMATION FOR SEQ ID NO:40:

(i) SEQUENCE CHARACTERISTICS:
(A) LENGTH: 36 base pairs
(B) TYPE: nucleic acid
(C) STRANDEDNESS: both
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:40:

CCACATAGAT CTTCACTTGA ACCGTTGCAT CTGCAC 36

(2) INFORMATION FOR SEQ ID NO:41:

(i) SEQUENCE CHARACTERISTICS:
(A) LENGTH: 39 base pairs
(B) TYPE: nucleic acid
(C) STRANDEDNESS: both
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:41:

GGTACAGGAT CCACCATGTC CAACATGGAT ATTGACGGC 39

(2) INFORMATION FOR SEQ ID NO:42:

(i) SEQUENCE CHARACTERISTICS:
(A) LENGTH: 39 base pairs
(B) TYPE: nucleic acid
(C) STRANDEDNESS: both
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:42:

CCACATGGAT CCTTAATAAT CGAGGTCATC ATAATCCTC 39

(2) INFORMATION FOR SEQ ID NO:43:

(i) SEQUENCE CHARACTERISTICS:
(A) LENGTH: 42 base pairs
(B) TYPE: nucleic acid
(C) STRANDEDNESS: both
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:43:

GGTACAGGAT CCACCATGTC GCTGTTTGGA GACACAATTG CC 42

(2) INFORMATION FOR SEQ ID NO:44:

(i) SEQUENCE CHARACTERISTICS:
(A) LENGTH: 38 base pairs
(B) TYPE: nucleic acid
(C) STRANDEDNESS: both
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:44:

CCACATGGAT CCTTATAGGT ATTTCTTCAC AAGAGCTG 38

(2) INFORMATION FOR SEQ ID NO:45:

(i) SEQUENCE CHARACTERISTICS:
(A) LENGTH: 3553 base pairs
(B) TYPE: nucleic acid
(C) STRANDEDNESS: double
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:45:

GATATTGGCT ATTGGCCATT GCATACGTTG TATCCATATC ATAATATGTA CATTTATATT 60

GGCTCATGTC CAACATTACC GCCATGTTGA CATTGATTAT TGACTAGTTA TTAATAGTAA 120

TCAATTACGG GGTCATTAGT TCATAGCCCA TATATGGAGT TCCGCGTTAC ATAACTTACG 180
GTAAATGGCC CGCCTGGCTG ACCGCCCAAC GACCCCCGCC CATTGACGTC AATAATGACG 240
TATGTTCCCA TAGTAACGCC AATAGGGACT TTCCATTGAC GTCAATGGGT GGAGTATTTA 300
CGGTAAACTG CCCACTTGGC AGTACATCAA GTGTATCATA TGCCAAGTAC GCCCCCTATT 360
GACGTCAATG ACGGTAAATG GCCCGCCTGG CATTATGCCC AGTACATGAC CTTATGGGAC 420
TTTCCTACTT GGCAGTACAT CTACGTATTA GTCATCGCTA TTACCATGGT GATGCGGTTT 480
TGGCAGTACA TCAATGGGCG TGGATAGCGG TTTGACTCAC GGGGATTTCC AAGTCTCCAC 540
CCCATTGACG TCAATGGGAG TTTGTTTTGG CACCAAAATC AACGGGACTT TCCAAAATGT 600
CGTAACAACT CCGCCCCATT GACGCAAATG GGCGGTAGGC GTGTACGGTG GGAGGTCTAT 660
ATAAGCAGAG CTCGTTTAGT GAACCGTCAG ATCGCCTGGA GACGCCATCC ACGCTGTTTT 720
GACCTCCATA GAAGACACCG GGACCGATCC AGCCTCCGCG GCCGGGAACG GTGCATTGGA 780
ACGCGGATTC CCCGTGCCAA GAGTGACGTA AGTACCGCCT ATAGAGTCTA TAGGCCCACC 840
CCCTTGGCTT CTTATGCATG CTATACTGTT TTTGGCTTGG GGTCTATACA CCCCCGCTTC 900
CTCATGTTAT AGGTGATGGT ATAGCTTAGC CTATAGGTGT GGGTTATTGA CCATTATTGA 960
CCACTCCCCT ATTGGTGACG ATACTTTCCA TTACTAATCC ATAACATGGC TCTTTGCCAC 1020
AACTCTCTTT ATTGGCTATA TGCCAATACA CTGTCCTTCA GAGACTGACA CGGACTCTGT 1080
ATTTTTACAG GATGGGGTCT CATTTATTAT TTACAAATTC ACATATACAA CACCACCGTC 1140
CCCAGTGCCC GCAGTTTTTA TTAAACATGC TAACGTGGGA TCTCCACGCG AATCTCGGGT 1200
ACGTGTTCCG GACATGGGCT CTTCTCCGGT AGCGGCGGAG CTTCTACATC CGAGCCCTGC 1260
TCCCATGCCT CCAGCGACTC ATGGTCGCTC GGCAGCTCCT TGCTCCTAAC AGTGGAGGCC 1320
AGACTTAGGC ACAGCACGAT GCCCACCACC ACCAGTGTGC CGCACAAGGC CGTGGCGGTA 1380
GGGTATGTGT CTGAAAATGA GCTCGGGGAG CGGGCTTGCA CCGCTGACGC ATTTGGAAGA 1440
CTTAAGGCAG CGGCAGAAGA AGATGCAGGC AGCTGAGTTG TTGTGTTCTG ATAAGAGTCA 1500
GAGGTAACTC CCGTTGCGGT GCTGTTAACG GTGGAGGGCA GTGTAGTCTG AGCAGTACTC 1560
GTTGCTGCCG CGCGCGCCAC CAGACATAAT AGCTGACAGA CTAACAGACT GTTCCTTTCC 1620
ATGGGTCTTT TCTGCAGTCA CCGTCCTTAG ATCTGCTGTG CCTTCTAGTT GCCAGCCATC 1680
TGTTGTTTGC CCCTCCCCCG TGCCTTCCTT GACCCTGGAA GGTGCCACTC CCACTGTCCT 1740
TTCCTAATAA AATGAGGAAA TTGCATCGCA TTGTCTGAGT AGGTGTCATT CTATTCTGGG 1800
GGGTGGGGTG GGGCAGCACA GCAAGGGGGA GGATTGGGAA GACAATAGCA GGCATGCTGG 1860
GGATGCGGTG GGCTCTATGG GTACGGCCGC AGCGGCCGTA CCCAGGTGCT GAAGAATTGA 1920
CCCGGTTCCT CGACCCGTAA AAAGGCCGCG TTGCTGGCGT TTTTCCATAG GCTCCGCCCC 1980

CCTGACGAGC ATCACAAAAA TCGACGCTCA AGTCAGAGGT GGCGAAACCC GACAGGACTA 2040

TAAAGATACC AGGCGTTTCC CCCTGGAAGC TCCCTCGTGC GCTCTCCTGT TCCGACCCTG 2100

CCGCTTACCG GATACCTGTC CGCCTTTCTC CCTTCGGGAA GCGTGGCGCT TTCTCAATGC 2160

TCACGCTGTA GGTATCTCAG TTCGGTGTAG GTCGTTCGCT CCAAGCTGGG CTGTGTGCAC 2220

GAACCCCCCG TTCAGCCCGA CCGCTGCGCC TTATCCGGTA ACTATCGTCT TGAGTCCAAC 2280

CCGGTAAGAC ACGACTTATC GCCACTGGCA GCAGCCACTG GTAACAGGAT TAGCAGAGCG 2340

AGGTATGTAG GCGGTGCTAC AGAGTTCTTG AAGTGGTGGC CTAACTACGG CTACACTAGC 2400

TGAAGGACAG TATTTGGTAT CTGCGCTCTG CTGAAGCCAG TTACCTTCGG AAAAAGAGTT 2460

GGTAGCTCTT GATCCGGCAA ACAAACCACC GCTGGTAGCG GTGGTTTTTT TGTTTGCAAG 2520

CAGCAGATTA CGCGCAGAAA AAAAGGATCT CAAGAAGATC CTTTGATCTT TTCTACGTGA 2580

TCCCGTAATG CTCTGCCAGT GTTACAACCA ATTAACCAAT TCTGATTAGA AAAACTCATC 2640

GAGCATCAAA TGAAACTGCA ATTTATTCAT ATCAGGATTA TCAATACCAT ATTTTTGAAA 2700

AAGCCGTTTC TGTAATGAAG GAGAAAACTC ACCGAGGCAG TTCCATAGGA TGGCAAGATC 2760

CTGGTATCGG TCTGCGATTC CGACTCGTCC AACATCAATA CAACCTATTA ATTTCCCCTC 2820

GTCAAAAATA AGGTTATCAA GTGAGAAATC ACCATGAGTG ACGACTGAAT CCGGTGAGAA 2880

TGGCAAAAGC TTATGCATTT CTTTCCAGAC TTGTTCAACA GGCCAGCCAT TACGCTCGTC 2940

ATCAAAATCA CTCGCATCAA CCAAACCGTT ATTCATTCGT GATTGCGCCT GAGCGAGACG 3000

AAATACGCGA TCGCTGTTAA AAGGACAATT ACAAACAGGA ATCGAATGCA ACCGGCGCAG 3060

GAACACTGCC AGCGCATCAA CAATATTTTC ACCTGAATCA GGATATTCTT CTAATACCTG 3120

GAATGCTGTT TTCCCGGGGA TCGCAGTGGT GAGTAACCAT GCATCATCAG GAGTACGGAT 3180

AAAATGCTTG ATGGTCGGAA GAGGCATAAA TTCCGTCAGC CAGTTTAGTC TGACCATCTC 3240

ATCTGTAACA TCATTGGCAA CGCTACCTTT GCCATGTTTC AGAAACAACT CTGGCGCATC 3300

GGGCTTCCCA TACAATCGAT AGATTGTCGC ACCTGATTGC CCGACATTAT CGCGAGCCCA 3360

TTTATACCCA TATAAATCAG CATCCATGTT GGAATTTAAT CGCGGCCTCG AGCAAGACGT 3420

TTCCCGTTGA ATATGGCTCA TAACACCCCT TGTATTACTG TTTATGTAAG CAGACAGTTT 3480

TATTGTTCAT GATGATATAT TTTTATCTTG TGCAATGTAA CATCAGAGAT TTTGAGACAC 3540

AACGTGGCTT TCC 3553

(2) INFORMATION FOR SEQ ID NO:46:

(i) SEQUENCE CHARACTERISTICS:

(A) LENGTH: 72 base pairs

(B) TYPE: nucleic acid

(C) STRANDEDNESS: both

(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:46:

TCACCGTCCT TAGATCGGTA CAACCATGAA GACTATCATT GCTTTGAGCT ACATTTTATG 60

TCTGGTTTTC GC 72

(2) INFORMATION FOR SEQ ID NO:47:

(i) SEQUENCE CHARACTERISTICS:
(A) LENGTH: 111 base pairs
(B) TYPE: nucleic acid
(C) STRANDEDNESS: both
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:47:

TCATGCTTTT TGCTTTGTGT TGTTTTGCTG GGGTTCATCA TGTGGGCCTG CCAAAAAGGC 60

AACATTAGGT GCAACATTTG CATTTGAAGA TCTATGTGGG ATCTGCTGTG C 111

(2) INFORMATION FOR SEQ ID NO:48:

(i) SEQUENCE CHARACTERISTICS:
(A) LENGTH: 63 base pairs
(B) TYPE: nucleic acid
(C) STRANDEDNESS: both
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:48:

TTAGATCGGA ACATGAAAGC AAAACTACTA GTCCTGTTAT GTGCATTTAC AGCTACATAT 60

GCA 63

(2) INFORMATION FOR SEQ ID NO:49:

(i) SEQUENCE CHARACTERISTICS:
(A) LENGTH: 102 base pairs

(B) TYPE: nucleic acid
(C) STRANDEDNESS: double
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:49:

CTGGTGCTTT TGGTCTCCCT GGGGGCAATC AGCTTCTGGA TGTGTTCTAA TGGGTCTTTG 60

CAGTGTAGAA TATGCATCTG AATGTGGGAT CTGCTGTGCC TT 102

(2) INFORMATION FOR SEQ ID NO:50:

(i) SEQUENCE CHARACTERISTICS:
(A) LENGTH: 108 base pairs
(B) TYPE: nucleic acid
(C) STRANDEDNESS: both
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:50:

CCTTAGATCG GTACAACCAT GAAGGCAATA ATTGTACTAC TCATGGTAGT AACATCCAAC 60

GCAGATCGAA TCTGCACTGG GATAACATCT TCAAACTCAC CTCATGTG 108

(2) INFORMATION FOR SEQ ID NO:51:

(i) SEQUENCE CHARACTERISTICS:
(A) LENGTH: 102 base pairs
(B) TYPE: nucleic acid
(C) STRANDEDNESS: both
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:51:

TTGGCTGTAA CATTGATGAT AGCTATTTTT ATTGTTTATA TGGTCTCCAG AGACAATGTT 60

TCTTGCTCCA TCTGTCTATA AATGTGGGAT CTGCTGTGCC TT 102

(2) INFORMATION FOR SEQ ID NO:52:

(i) SEQUENCE CHARACTERISTICS:
(A) LENGTH: 84 base pairs
(B) TYPE: nucleic acid
(C) STRANDEDNESS: both
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:52:

GTCCTTAGAT CCACCATGGC GTCCCAAGGC ACCAAACGGT CTTATGAACA GATGGAAACT 60

GATGGGGAAC GCCAGAATGC AACT 84

(2) INFORMATION FOR SEQ ID NO:53:

(i) SEQUENCE CHARACTERISTICS:
(A) LENGTH: 108 base pairs
(B) TYPE: nucleic acid
(C) STRANDEDNESS: both
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:53:

GAAAAGGCAA CGAACCCGAT CGTGCCCTCT TTTGACATGA GTAATGAAGG ATCTTATTTC 60

TTCGGAGACA ATGCAGAAGA GTACGACAAT TAAGGATCTG CTGTGCCT 108

(2) INFORMATION FOR SEQ ID NO:54

(i) SEQUENCE CHARACTERISTICS:
(A) LENGTH: 132 base pairs
(B) TYPE: nucleic acid
(C) STRANDEDNESS: both
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:54:

CTTAGATCCA GATCTACCAT GAGTCTTCTA ACCGAGGTCG AAACGTATGT TCTCTCTATC 60

GTTCCATCAG GCCCCCTCAA AGCCGAAATC GCGCAGAGAC TTGAAGATGT CTTTGCTGGG 120

AAAAACACAG AT 132

(2) INFORMATION FOR SEQ ID NO:55:

(i) SEQUENCE CHARACTERISTICS:
(A) LENGTH: 129 base pairs
(B) TYPE: nucleic acid
(C) STRANDEDNESS: both
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:55:

GGGACTCATC CTAGCTCCAG TACTGGTCTA AAAGATGATC TTCTTGAAAA TTTGCAGACC 60

TATCAGAAAC GAATGGGGGT GCAGATGCAA CGGTTCAAGT GAAGATCTAT GTGGGATCTG 120

CTGTGCCTT 129

(2) INFORMATION FOR SEQ ID NO:56:

(i) SEQUENCE CHARACTERISTICS:
(A) LENGTH: 81 base pairs
(B) TYPE: nucleic acid
(C) STRANDEDNESS: both
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:56:

CTTAGATCCA CCATGTCCAA CATGGATATT GACGGTATCA ACACTGGGAC AATTGACAAA 60

ACACCGGAAG AAATAACTTC T 81

(2) INFORMATION FOR SEQ ID NO:57:

(i) SEQUENCE CHARACTERISTICS:
(A) LENGTH: 96 base pairs
(B) TYPE: nucleic acid
(C) STRANDEDNESS: both
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:57:

GTTGAAATTC CAATTAAGCA GACCATCCCC AATTTCTTCT TTGGGAGGGA CACAGCAGAG 60

GATTATGATG ACCTCGATTA TTAAGGATCT GCTGTG 96

(2) INFORMATION FOR SEQ ID NO:58:

(i) SEQUENCE CHARACTERISTICS:
(A) LENGTH: 96 base pairs
(B) TYPE: nucleic acid
(C) STRANDEDNESS: both
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:58:

CTTAGATCCA CCATGTCGCT GTTTGGAGAC ACAATTGCCT ACCTGCTTTC ATTGACAGAA 60

GATGGAGAAG GCAAAGCAGA ACTAGCAGAA AAATTA 96

(2) INFORMATION FOR SEQ ID NO:59:

(i) SEQUENCE CHARACTERISTICS:
(A) LENGTH: 123 base pairs
(B) TYPE: nucleic acid
(C) STRANDEDNESS: both
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:59:

AGATCTCTTG GGGCAAGTCA AGAGAATGGG GAAGGAATTG CAAAGGATGT GATGGAAGTG 60

CTAAAGCAGA GCTCTATGGG AAATTCAGCT CTTGTGAAGA AATACCTATA AGGATCTGCT 120

GTG 123

(2) INFORMATION FOR SEQ ID NO:60:

(i) SEQUENCE CHARACTERISTICS:
(A) LENGTH: 33 base pairs
(B) TYPE: nucleic acid

(C) STRANDEDNESS: both
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:60:

GGTACAAATA TTGGCTATTG GCCATTGCAT ACG 33

(2) INFORMATION FOR SEQ ID NO:61:

(i) SEQUENCE CHARACTERISTICS:
(A) LENGTH: 36 base pairs
(B) TYPE: nucleic acid
(C) STRANDEDNESS: both
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:61:

CCACATCTCG AGGAACCGGG TCAATTCTTC AGCACC 36

(2) INFORMATION FOR SEQ ID NO:62:

(i) SEQUENCE CHARACTERISTICS:
(A) LENGTH: 38 base pairs
(B) TYPE: nucleic acid
(C) STRANDEDNESS: both
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:62:

GGTACAGATA TCGGAAAGCC ACGTTGTGTC TCAAAATC 38

(2) INFORMATION FOR SEQ ID NO:63:

(i) SEQUENCE CHARACTERISTICS:
(A) LENGTH: 37 base pairs
(B) TYPE: nucleic acid
(C) STRANDEDNESS: both
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:63:

CCACATGGAT CCGTAATGCT CTGCCAGTGT TACAACC 37

(2) INFORMATION FOR SEQ ID NO:64:

(i) SEQUENCE CHARACTERISTICS:
(A) LENGTH: 39 base pairs
(B) TYPE: nucleic acid
(C) STRANDEDNESS: both
(D) TOPOLOGY: both

(ii) MOLECULE TYPE: cDNA

(iii) HYPOTHETICAL: NO

(iv) ANTI-SENSE: NO

(xi) SEQUENCE DESCRIPTION: SEQ ID NO:64:

GGTACATGAT CACGTAGAAA AGATCAAAGG ATCTTCTTG 39

## Claims

1. A DNA construct comprising nucleic acid encoding an influenza virus gene, wherein said DNA construct is capable of inducing the expression of an antigenic influenza virus gene product which induces an influenza virus specific immune response upon introduction of said DNA construct into animal tissues in vivo and resultant uptake of the DNA construct by cells which express the encoded influenza gene.

2. The DNA construct of Claim 1 wherein the influenza virus gene encodes nucleoprotein, hemagglutinin, polymerase, matrix, or non-structural human influenza virus gene products.

3. A DNA construct as claimed in Claim 1 or Claim 2 wherein the nucleic acid encoding an influenza virus gene is operatively linked to one or more control sequences which, when said DNA construct is introduced into a living tissue, direct the transcription initiation and subsequent translation of the gene.

4. A DNA construct as claimed in Claim 3 wherein the control sequences comprise a promoter for RNA polymerase transcription and a transcriptional terminator at the end of the influenza virus gene coding sequence.

5. A DNA construct as claimed in Claim 4 wherein the promoter for RNA polymerase transcription is selected from the Rous sarcoma virus long terminal repeat and the cytomegalovirus promoter with the intron A sequence.

6. A DNA construct as claimed in Claim 4 or 5 wherein the transcriptional terminator is the bovine growth hormone terminator.

7. A polynucleotide vaccine comprising a DNA construct as claimed in any one of Claims 1 to 6.

8. A polynucleotide vaccine comprising a DNA construct which induces neutralizing antibody against human influenza virus, influenza virus specific cytotoxic lymphocytes, or protective immune responses upon introduction of said DNA pharmaceutical into animal tissues in vivo, wherein the animal is a vertebrate, and the polynucleotide vaccine encodes an influenza virus gene which is expressed upon introduction into said verterbrates' tissues in vivo.

**9.** A polynucleotide vaccine as claimed in Claim 7 or Claim 8 which contains a DNA construct selected from one or more of:
a) pnRSV-PR-NP,
b) V1-PR-NP,
c) V1J-PR-NP, the 5' end of which is SEQ. ID:12:,
d) V1J-PR-PB1, the 5' end of which is SEQ. ID:13:,
e) V1J-PR-NS, the 5' end of which is SEQ. ID:14:,
f) V1J-PR-HA, the 5' end of which is SEQ. ID:15:,
g) V1J-PR-PB2, the 5' end of which is SEQ. ID:16:,
h) V1J-PR-M1, the 5, end of which is SEQ. ID:17:,
i) V1Jneo-BJ-NP, the 5, end of which is SEQ. ID:20: and
the 3' end of which is SEQ. ID:21:,
j) V1Jneo-TX-NP, the 5' end of which is SEQ. ID:24 and
the 3' end of which is SEQ. ID:25: and
k) V1Jneo-PA-HA, the 5' end of which is SEQ. ID:26: and
the 3' end of which is SEQ. ID:27:
l) V1Jns-GA-HA (A/Georgia/03/93), construct size 6.56 Kb,
the 5' end of which is SEQ.ID:46: and
the 3' end of which is SEQ. ID:47:,
m) V1Jns-TX-HA (A/Texas/36/91), construct size 6.56 Kb,
the 5, end of which is SEQ.ID:48: and
the 3' end of which is SEQ. ID:49:,
n) V1Jns-PA-HA (B/Panama/45/90), construct size 6.61 Kb,
the 5' end of which is SEQ.ID:50: and
the 3' end of which is SEQ. ID:51:,
o) V1Jns-BJ-NP (A/Beijing/353/89), construct size 6.42 Kb,
the 5' end of which is SEQ.ID:52: and
the 3' end of which is SEQ. ID:53:,
p) V1Jns-BJ-M1 (A/Beijing/353/89), construct size 5.62 Kb,
the 5' end of which is SEQ.ID:54: and
the 3' end of which is SEQ. ID:55:,
q) V1Jns-PA-NP (B/Panama/45/90), construct size 6.54 Kb,
the 5' end of which is SEQ.ID:56: and
the 3' end of which is SEQ. ID:57:, and
r) V1Jns-PA-M1 (B/Panama/45/90), construct size 5.61 Kb,
the 5' end of which is SEQ.ID:58: and
the 3' end of which is SEQ. ID:59:.

**10.** The expression vector: V1J, SEQ.ID:10:,
V1Jneo, SEQ.ID:18:,
V1Jns,
V1JR, SEQ:ID:45:.

**11.** The use of an isolated human influenza virus gene operatively linked to one or more control sequences for the manufacture of a medicament for use in immunization against infection by human influenza virus.

**12.** The use of a DNA construct as claimed in any one of Claims 1-6 for the manufacture of a medicament for use in immunisation against infection by human influenza virus.

**13.** The use of a polynucleotide vaccine as claimed in any one of Claims 7-9 for the manufacture of a medicament for use in immunisation against infection by human influenza virus.

**14.** The use of a DNA construct as claimed in any one of Claims 1-6 for the manufacture of a medicament for use in immunisation against infection by human influenza virus wherein said medicament is adapted for direct administration of the DNA construct into tissue in vivo.

**15.** The use as claimed in Claim 14 wherein said medicament is adapted for administration of the DNA construct either as naked DNA in a physiologically acceptable solution without a carrier or as a mixture of DNA and a liposome, or as a mixture with an adjuvant or a transfection facilitating agent.

**16.** A method for using an influenza virus gene to induce immune responses in vivo which comprises:
a) isolating the gene,
b) linking the gene to regulatory sequences such that the gene is operatively linked to control sequences which, when introduced into a living tissue direct the transcription initiation and subsequent translation of the gene, and
c) introducing the gene into a living tissue.

**17.** The method of Claim 16 which further comprises boosting induced immune responses by introducing influenza virus gene on multiple occasions.

**18.** The method of Claim 16 wherein the influenza virus gene encodes a human influenza virus nucleoprotein, hemagglutinin, matrix, nonstructural, or polymerase gene product.

**19.** The method of Claim 18 wherein the human influenza virus gene encodes the nucleoprotein, basic polymerase1, nonstructural protein1, hemagglutinin, matrix1, or basic polymerase2 of one or more of the human influenza virus isolates A/PR/8/34, A/Beijing/353/89, A/Texas/36/91, A/Georgia/03/93, and B/Panama/45/90.

**20.** A method for inducing immune responses against infection or disease caused by strains of influenza virus which comprises introducing into a vertebrate a nucleic acid which encodes a conserved influenza virus epitope specific to a first influenza virus strain such that the induced immune response protects not only against infection or disease by the first influenza virus strain but also protects against infection or disease by strains that are different to said first strain.

**21.** The method of any of Claims 16-20 wherein the organism being treated by the method is a human.

**22.** The DNA:
a) pnRSV-PR-NP,
b) V1-PR-NP,
c) V1J-PR-NP, the 5' end of which is SEQ. ID: 12:,
d) V1J-PR-PB1, the 5' end of which is SEQ. ID:13:,
e) V1J-PR-NS, the 5' end of which is SEQ. ID: 14:,
f) V1J-PR-HA, the 5' end of which is SEQ. ID: 15:,
g) V1J-PR-PB2, the 5' end of which is SEQ. ID: 16:,
h) V1J-PR-M1, the 5' end of which is SEQ. ID:17:,
i) V1Jneo-BJ-NP, the 5' end of which is SEQ. ID:20: and the 3' end of which is SEQ. ID:21:,
j) V1Jneo-TX-NP, the 5' end of which is SEQ. ID:24 and the 3' end of which is SEQ. ID:25: and
k) V1Jneo-PA-HA, the 5' end of which is SEQ. ID:26: and the 3' end of which is SEQ. ID:27:
l) V1Jns-GA-HA (A/Georgia/03/93), construct size 6.56 Kb, the 5' end of which is SEQ.ID:46: and the 3' end of which is SEQ. ID:47:,
m) V1Jns-TX-HA (A/Texas/36/91), construct size 6.56 Kb, the 5' end of which is SEQ.ID:48: and the 3' end of which is SEQ. ID:49:,
n) V1Jns-PA-HA (B/Panama/45/90), construct size 6.61 Kb, the 5' end of which is SEQ.ID:50: and the 3' end of which is SEQ. ID:51:,

o) V1Jns-BJ-NP (A/Beijing/353/89), construct size 6.42 Kb,
the 5' end of which is SEQ.ID:52: and
the 3' end of which is SEQ. ID:53:,
p) V1Jns-BJ-M1 (A/Beijing/353/89), construct size 5.62 Kb,
the 5' end of which is SEQ.ID:54: and
the 3' end of which is SEQ. ID:55:,
q) V1Jns-PA-NP (B/Panama/45/90), construct size 6.54 Kb,
the 5' end of which is SEQ.ID:56: and
the 3' end of which is SEQ. ID:57:, and
r) V1Jns-PA-M1 (B/Panama/45/90), construct size 5.61 Kb,
the 5' end of which is SEQ.ID:58: and
the 3' end of which is SEQ. ID:59:,

**23.** A pharmaceutical composition which comprises at least one DNA construct as claimed in any one of Claims 1 to 6.

**24.** A composition of nucleic acid constructs encoding influenza virus genes from both A-type and B-type human influenza viruses.

**25.** The composition of Claim 23 or Claim 24 which comprises DNA constructs encoding the hemagglutinin gene of at least three strains of influenza virus, the nucleoprotein gene of at least two strains of influenza virus, and the matrix protein gene of at least two strains of influenza virus.

**26.** The composition of Claim 23 or Claim 24 wherein said influenza virus genes are derived from influenza viruses of the H1N1, H2N2, and H3N2 and B strains of influenza virus.

**27.** The composition of Claim 23 or Claim 24 comprising:
a) V1Jns-GA-HA (A/Georgia/03/93), construct size 6.56 Kb,
the 5' end of which is SEQ.ID:46: and
the 3' end of which is SEQ. ID:47:,
b) V1Jns-TX-HA (A/Texas/36/91), construct size 6.56 Kb,
the 5' end of which is SEQ.ID;48: and
the 3' end of which is SEQ. ID:49:,
c) V1Jns-PA-HA (B/Panama/45/90), construct size 6.61 Kb,
the 5' end of which is SEQ.ID:50: and
the 3' end of which is SEQ. ID:51:,
d) V1Jns-BJ-NP (A/Beijing/353/89), construct size 6.42 Kb,
the 5' end of which is SEQ.ID:52: and
the 3' end of which is SEQ. ID:53:,
e) V1Jns-BJ-M1 (A/Beijing/353/89), construct size 5.62 Kb,
the 5' end of which is SEQ.ID:54: and
the 3' end of which is SEQ. ID:55:,
f) V1Jns-PA-NP (B/Panama/45/90), construct size 6.54 Kb,
the 5' end of which is SEQ.ID:56: and
the 3' end of which is SEQ. ID:57:, and
g) V1Jns-PA-M1 (B/Panama/45/90), construct size 5.61 Kb,
the 5' end of which is SEQ.ID:58: and
the 3' end of which is SEQ. ID:59:.

FIGURE 1

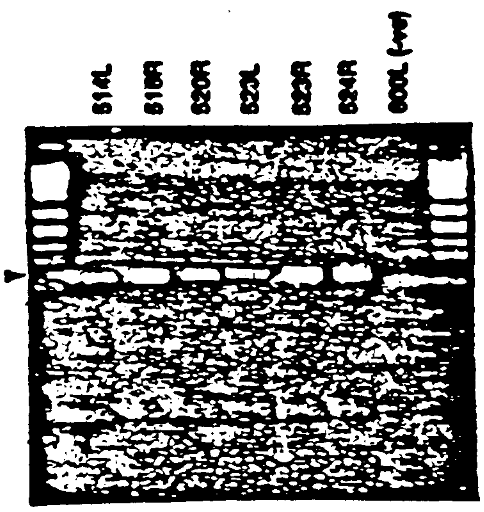

Figure 2

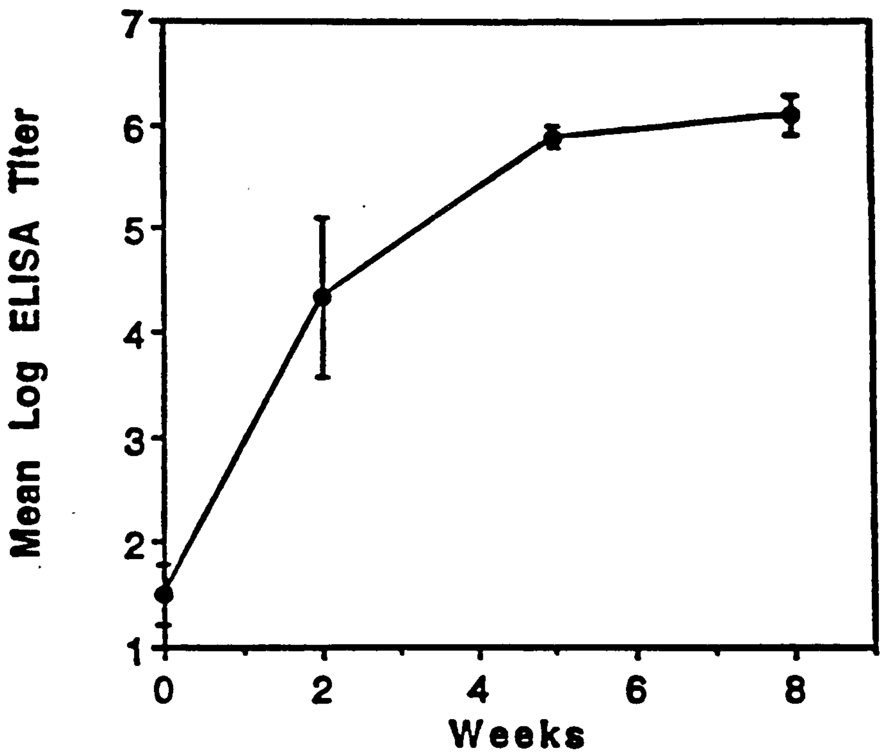

Figure 3

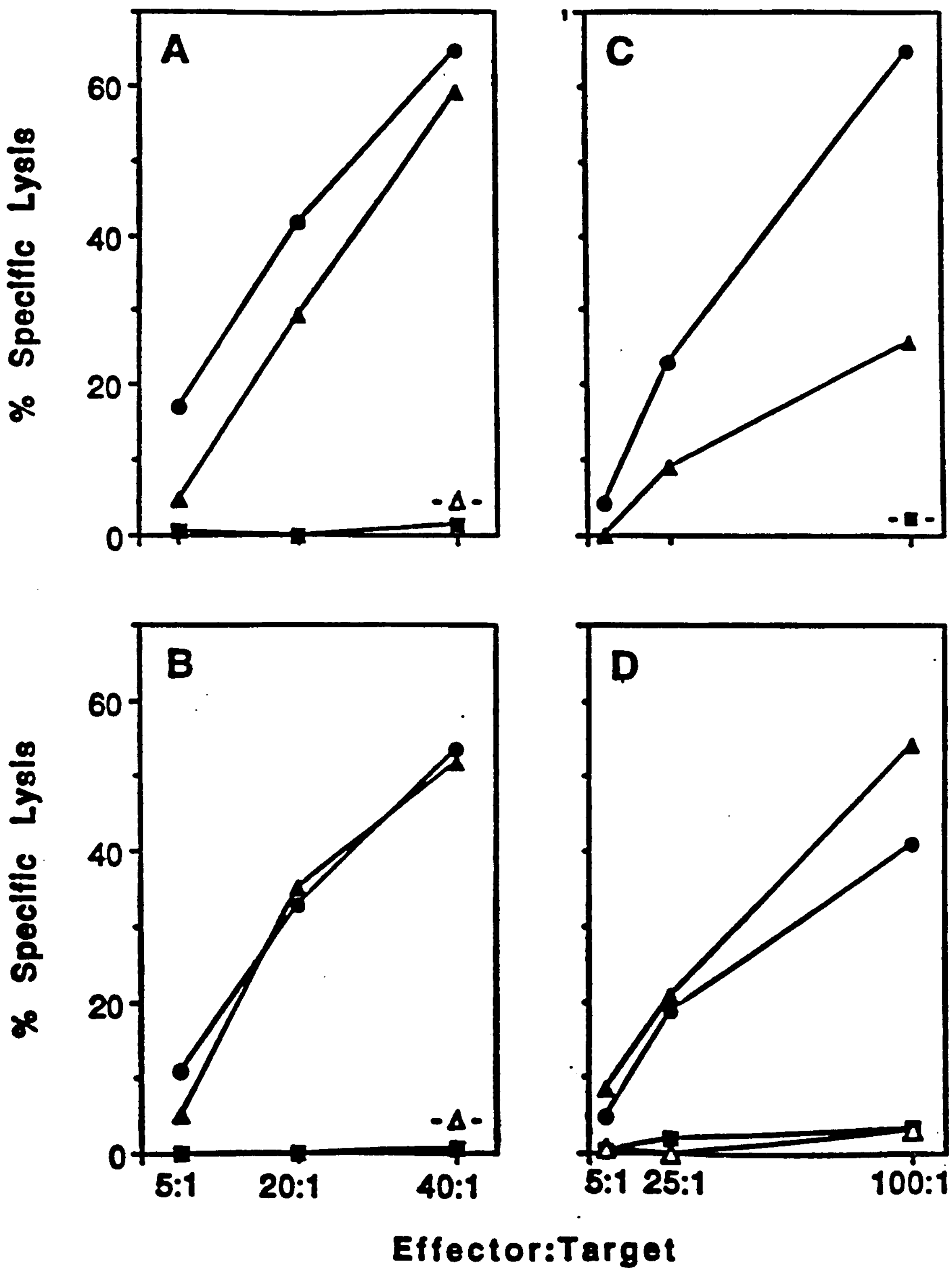
A
B
C
D
% Specific Lysis
% Specific Lysis
60
40
20
0
5:1
20:1
40:1
5:1
25:1
100:1
Effector:Target

Figure 4

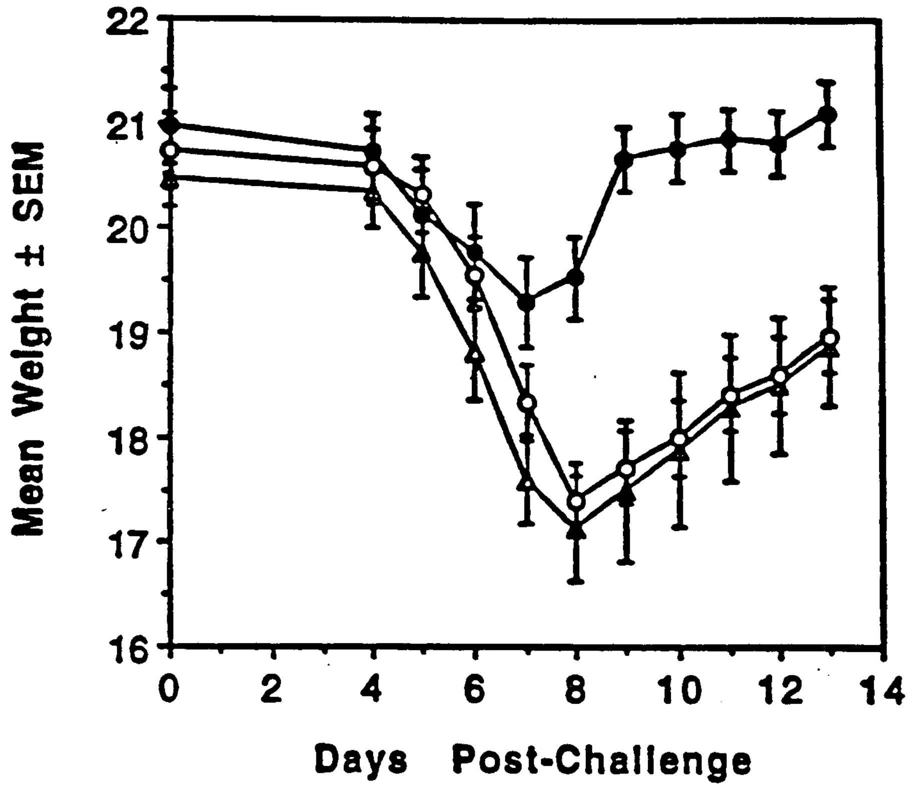

22
21
20
19
18
17
16
0
2
4
6
8
10
12
14
Mean Weight ± SEM
Days Post-Challenge

Figure 5

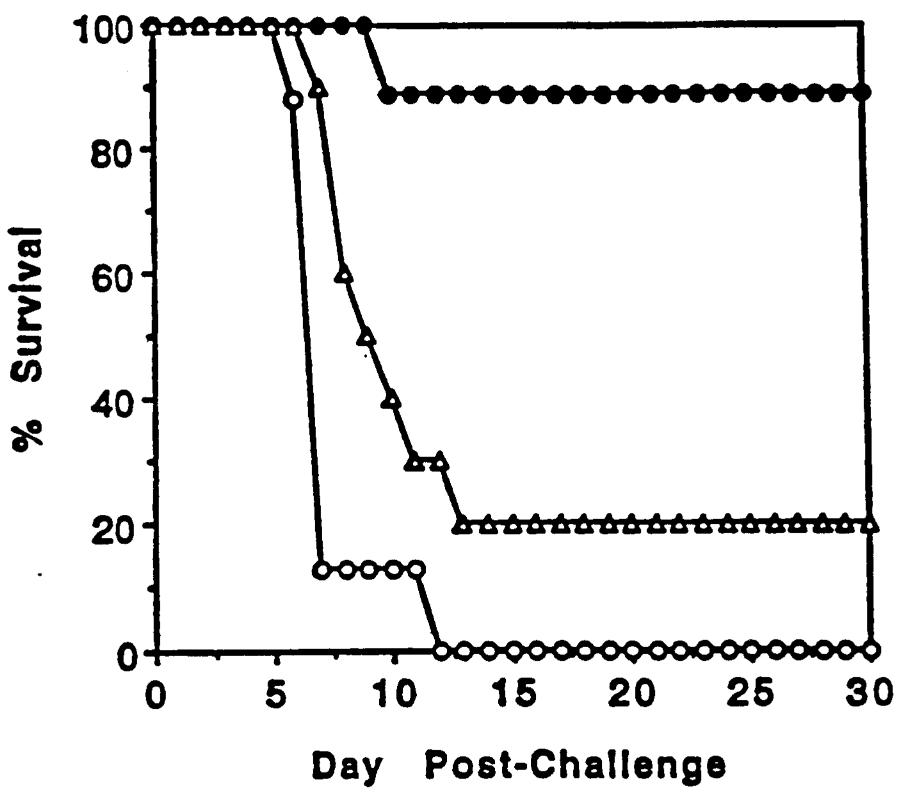

Figure 6: V1J.Sequence, SEQ. ID:10:

1 TCGCGCGTTT CGGTGATGAC GGTGAAAACC TCTGACACAT GCAGCTCCCG

51 GAGACGGTCA CAGCTTGTCT GTAAGCGGAT GCCGGGAGCA GACAAGCCCG

101 TCAGGGCGCG TCAGCGGGTG TTGGCGGGTG TCGGGGCTGG CTTAACTATG

151 CGGCATCAGA GCAGATTGTA CTGAGAGTGC ACCATATGCG GTGTGAAATA

201 CCGCACAGAT GCGTAAGGAG AAAATACCGC ATCAGATTGG CTATTGGCCA

251 TTGCATACGT TGTATCCATA TCATAATATG TACATTTATA TTGGCTCATG

301 TCCAACATTA CCGCCATGTT GACATTGATT ATTGACTAGT TATTAATAGT

351 AATCAATTAC GGGGTCATTA GTTCATAGCC CATATATGGA GTTCCGCGTT

401 ACATAACTTA CGGTAAATGG CCCGCCTGGC TGACCGCCCA ACGACCCCCG

451 CCCATTGACG TCAATAATGA CGTATGTTCC CATAGTAACG CCAATAGGGA

501 CTTTCCATTG ACGTCAATGG GTGGAGTATT TACGGTAAAC TGCCCACTTG

551 GCAGTACATC AAGTGTATCA TATGCCAAGT ACGCCCCCTA TTGACGTCAA

601 TGACGGTAAA TGGCCCGCCT GGCATTATGC CCAGTACATG ACCTTATGGG

651 ACTTTCCTAC TTGGCAGTAC ATCTACGTAT TAGTCATCGC TATTACCATG

701 GTGATGCGGT TTTGGCAGTA CATCAATGGG CGTGGATAGC GGTTTGACTC

751 ACGGGGATTT CCAAGTCTCC ACCCCATTGA CGTCAATGGG AGTTTGTTTT

801 GGCACCAAAA TCAACGGGAC TTTCCAAAAT GTCGTAACAA CTCCGCCCCA

851 TTGACGCAAA TGGGCGGTAG GCGTGTACGG TGGGAGGTCT ATATAAGCAG

901 AGCTCGTTTA GTGAACCGTC AGATCGCCTG GAGACGCCAT CCACGCTGTT

951 TTGACCTCCA TAGAAGACAC CGGGACCGAT CCAGCCTCCG CGGCCGGGAA

1001 CGGTGCATTG GAACGCGGAT TCCCCGTGCC AAGAGTGACG TAAGTACCGC

1051 CTATAGAGTC TATAGGCCCA CCCCCTTGGC TTCTTATGCA TGCTATACTG

1101 TTTTTGGCTT GGGGTCTATA CACCCCCGCT TCCTCATGTT ATAGGTGATG

1151 GTATAGCTTA GCCTATAGGT GTGGGTTATT GACCATTATT GACCACTCCC

1201 CTATTGGTGA CGATACTTTC CATTACTAAT CCATAACATG GCTCTTTGCC

Figure 6 (continued, p2/4)

1251 ACAACTCTCT TTATTGGCTA TATGCCAATA CACTGTCCTT CAGAGACTGA

1301 CACGGACTCT GTATTTTTAC AGGATGGGGT CTCATTTATT ATTTACAAAT

1351 TCACATATAC AACACCACCG TCCCCAGTGC CCGCAGTTTT TATTAAACAT

1401 AACGTGGGAT CTCCACGCGA ATCTCGGGTA CGTGTTCCGG ACATGGGCTC

1451 TTCTCCGGTA GCGGCGGAGC TTCTACATCC GAGCCCTGCT CCCATGCCTC

1501 CAGCGACTCA TGGTCGCTCG GCAGCTCCTT GCTCCTAACA GTGGAGGCCA

1551 GACTTAGGCA CAGCACGATG CCCACCACCA CCAGTGTGCC GCACAAGGCC

1601 GTGGCGGTAG GGTATGTGTC TGAAAATGAG CTCGGGGAGC GGGCTTGCAC

1651 CGCTGACGCA TTTGGAAGAC TTAAGGCAGC GGCAGAAGAA GATGCAGGCA

1701 GCTGAGTTGT TGTGTTCTGA TAAGAGTCAG AGGTAACTCC CGTTGCGGTG

1751 CTGTTAACGG TGGAGGGCAG TGTAGTCTGA GCAGTACTCG TTGCTGCCGC

1801 GCGCGCCACC AGACATAATA GCTGACAGAC TAACAGACTG TTCCTTTCCA

1851 TGGGTCTTTT CTGCAGTCAC CGTCCTTAG ATCTGCTGTG CCTTCTAGTT

1901 GCCAGCCATC TGTTGTTTGC CCCTCCCCCG TGCCTTCCTT GACCCTGGAA

1951 GGTGCCACTC CCACTGTCCT TTCCTAATAA AATGAGGAAA TTGCATCGCA

2001 TTGTCTGAGT AGGTGTCATT CTATTCTGGG GGGTGGGGTG GGGCAGCACA

2051 GCAAGGGGGA GGATTGGGAA GACAATAGCA GGCATGCTGG GGATGCGGTG

2101 GGCTCTATGG GTACCCAGGT GCTGAAGAAT TGACCCGGTT CCTCCTGGGC

2151 CAGAAAGAAG CAGGCACATC CCCTTCTCTG TGACACACCC TGTCCACGCC

2201 CCTGGTTCTT AGTTCCAGCC CCACTCATAG GACACTCATA GCTCAGGAGG

2251 GCTCCGCCTT CAATCCCACC CGCTAAAGTA CTTGGAGCGG TCTCTCCCTC

2301 CCTCATCAGC CCACCAAACC AAACCTAGCC TCCAAGAGTG GGAAGAAATT

2351 AAAGCAAGAT AGGCTATTAA GTGCAGAGGG AGAGAAAATG CCTCCAACAT

2401 GTGAGGAAGT AATGAGAGAA ATCATAGAAT TTCTTCCGCT TCCTCGCTCA

2451 CTGACTCGCT GCGCTCGGTC GTTCGGCTGC GGCGAGCGGT ATCAGCTCAC

Figure 6 (continued, p3/4)

2501 TCAAAGGCGG TAATACGGTT ATCCACAGAA TCAGGGGATA ACGCAGGAAA

2551 GAACATGTGA GCAAAAGGCC AGCAAAAGGC CAGGAACCGT AAAAAGGCCG

2601 CGTTGCTGGC GTTTTTCCAT AGGCTCCGCC CCCCTGACGA GCATCACAAA

2651 AATCGACGCT CAAGTCAGAG GTGGCGAAAC CCGACAGGAC TATAAAGATA

2701 CCAGGCGTTT CCCCCTGGAA GCTCCCTCGT GCGCTCTCCT GTTCCGACCC

2751 TGCCGCTTAC CGGATACCTG TCCGCCTTTC TCCCTTCGGG AAGCGTGGCG

2801 CTTTCTCAAT GCTCACGCTG TAGGTATCTC AGTTCGGTGT AGGTCGTTCG

2851 CTCCAAGCTG GGCTGTGTGC ACGAACCCCC CGTTCAGCCC GACCGCTGCG

2901 CCTTATCCGG TAACTATCGT CTTGAGTCCA ACCCGGTAAG ACACGACTTA

2951 TCGCCACTGG CAGCAGCCAC TGGTAACAGG ATTAGCAGAG CGAGGTATGT

3001 AGGCGGTGCT ACAGAGTTCT TGAAGTGGTG GCCTAACTAC GGCTACACTA

3051 GAAGGACAGT ATTTGGTATC TGCGCTCTGC TGAAGCCAGT TACCTTCGGA

3101 AAAAGAGTTG GTAGCTCTTG ATCCGGCAAA CAAACCACCG CTGGTAGCGG

3151 TGGTTTTTTT GTTTGCAAGC AGCAGATTAC GCGCAGAAAA AAAGGATCTC

3201 AAGAAGATCC TTTGATCTTT TCTACGGGGT CTGACGCTCA GTGGAACGAA

3251 AACTCACGTT AAGGGATTTT GGTCATGAGA TTATCAAAAA GGATCTTCAC

3301 CTAGATCCTT TTAAATTAAA AATGAAGTTT TAAATCAATC TAAAGTATAT

3351 ATGAGTAAAC TTGGTCTGAC AGTTACCAAT GCTTAATCAG TGAGGCACCT

3401 ATCTCAGCGA TCTGTCTATT TCGTTCATCC ATAGTTGCCT GACTCCCCGT

3451 CGTGTAGATA ACTACGATAC GGGAGGGCTT ACCATCTGGC CCCAGTGCTG

3501 CAATGATACC GCGAGACCCA CGCTCACCGG CTCCAGATTT ATCAGCAATA

3551 AACCAGCCAG CCGGAAGGGC CGAGCGCAGA AGTGGTCCTG CAACTTTATC

3601 CGCCTCCATC CAGTCTATTA ATTGTTGCCG GGAAGCTAGA GTAAGTAGTT

3651 CGCCAGTTAA TAGTTTGCGC AACGTTGTTG CCATTGCTAC AGGCATCGTG

3701 GTGTCACGCT CGTCGTTTGG TATGGCTTCA TTCAGCTCCG GTTCCCAACG

Figure 6 (continued, p4/4)

3751 ATCAAGGCGA GTTACATGAT CCCCCATGTT GTGCAAAAAA GCGGTTAGCT

3801 CCTTCGGTCC TCCGATCGTT GTCAGAAGTA AGTTGGCCGC AGTGTTATCA

3851 CTCATGGTTA TGGCAGCACT GCATAATTCT CTTACTGTCA TGCCATCCGT

3901 AAGATGCTTT TCTGTGACTG GTGAGTACTC AACCAAGTCA TTCTGAGAAT

3951 AGTGTATGCG GCGACCGAGT TGCTCTTGCC CGGCGTCAAT ACGGGATAAT

4001 ACCGCGCCAC ATAGCAGAAC TTTAAAAGTG CTCATCATTG GAAAACGTTC

4051 TTCGGGGCGA AAACTCTCAA GGATCTTACC GCTGTTGAGA TCCAGTTCGA

4101 TGTAACCCAC TCGTGCACCC AACTGATCTT CAGCATCTTT TACTTTCACC

4151 AGCGTTTCTG GGTGAGCAAA AACAGGAAGG CAAAATGCCG CAAAAAAGGG

4201 AATAAGGGCG ACACGGAAAT GTTGAATACT CATACTCTTC CTTTTTCAAT

4251 ATTATTGAAG CATTTATCAG GGTTATTGTC TCATGAGCGG ATACATATTT

4301 GAATGTATTT AGAAAAATAA ACAAATAGGG GTTCCGCGCA CATTTCCCCG

4351 AAAAGTGCCA CCTGACGTCT AAGAAACCAT TATTATCATG ACATTAACCT

4401 ATAAAAATAG GCGTATCACG AGGCCCTTTC GTC

Figure 7: V1Jneo Sequence, SEQ. ID:18:

1 TCGCGCGTTT CGGTGATGAC GGTGAAAACC TCTGACACAT GCAGCTCCCG

51 GAGACGGTCA CAGCTTGTCT GTAAGCGGAT GCCGGGAGCA GACAAGCCCG

101 TCAGGGCGCG TCAGCGGGTG TTGGCGGGTG TCGGGGCTGG CTTAACTATG

151 CGGCATCAGA GCAGATTGTA CTGAGAGTGC ACCATATGCG GTGTGAAATA

201 CCGCACAGAT GCGTAAGGAG AAAATACCGC ATCAGATTGG CTATTGGCCA

251 TTGCATACGT TGTATCCATA TCATAATATG TACATTTATA TTGGCTCATG

301 TCCAACATTA CCGCCATGTT GACATTGATT ATTGACTAGT TATTAATAGT

351 AATCAATTAC GGGGTCATTA GTTCATAGCC CATATATGGA GTTCCGCGTT

401 ACATAACTTA CGGTAAATGG CCCGCCTGGC TGACCGCCCA ACGACCCCCG

451 CCCATTGACG TCAATAATGA CGTATGTTCC CATAGTAACG CCAATAGGGA

501 CTTTCCATTG ACGTCAATGG GTGGAGTATT TACGGTAAAC TGCCCACTTG

551 GCAGTACATC AAGTGTATCA TATGCCAAGT ACGCCCCCTA TTGACGTCAA

601 TGACGGTAAA TGGCCCGCCT GGCATTATGC CCAGTACATG ACCTTATGGG

651 ACTTTCCTAC TTGGCAGTAC ATCTACGTAT TAGTCATCGC TATTACCATG

701 GTGATGCGGT TTTGGCAGTA CATCAATGGG CGTGGATAGC GGTTTGACTC

751 ACGGGGATTT CCAAGTCTCC ACCCCATTGA CGTCAATGGG AGTTTGTTTT

801 GGCACCAAAA TCAACGGGAC TTTCCAAAAT GTCGTAACAA CTCCGCCCCA

851 TTGACGCAAA TGGGCGGTAG GCGTGTACGG TGGGAGGTCT ATATAAGCAG

901 AGCTCGTTTA GTGAACCGTC AGATCGCCTG GAGACGCCAT CCACGCTGTT

951 TTGACCTCCA TAGAAGACAC CGGGACCGAT CCAGCCTCCG CGGCCGGGAA

1001 CGGTGCATTG GAACGCGGAT TCCCCGTGCC AAGAGTGACG TAAGTACCGC

1051 CTATAGAGTC TATAGGCCCA CCCCCTTGGC TTCTTATGCA TGCTATACTG

1101 TTTTTGGCTT GGGGTCTATA CACCCCCGCT TCCTCATGTT ATAGGTGATG

1151 GTATAGCTTA GCCTATAGGT GTGGGTTATT GACCATTATT GACCACTCCC

Figure 7 (continued, p2/4)

1201 CTATTGGTGA CGATACTTTC CATTACTAAT CCATAACATG GCTCTTTGCC
1251 ACAACTCTCT TTATTGGCTA TATGCCAATA CACTGTCCTT CAGAGACTGA
1301 CACGGACTCT GTATTTTTAC AGGATGGGGT CTCATTTATT ATTTACAAAT
1351 TCACATATAC AACACCACCG TCCCCAGTGC CCGCAGTTTT TATTAAACAT
1401 AACGTGGGAT CTCCACGCGA ATCTCGGGTA CGTGTTCCGG ACATGGGCTC
1451 TTCTCCGGTA GCGGCGGAGC TTCTACATCC GAGCCCTGCT CCCATGCCTC
1501 CAGCGACTCA TGGTCGCTCG GCAGCTCCTT GCTCCTAACA GTGGAGGCCA
1551 GACTTAGGCA CAGCACGATG CCCACCACCA CCAGTGTGCC GCACAAGGCC
1601 GTGGCGGTAG GGTATGTGTC TGAAAATGAG CTCGGGGAGC GGGCTTGCAC
1651 CGCTGACGCA TTTGGAAGAC TTAAGGCAGC GGCAGAAGAA GATGCAGGCA
1701 GCTGAGTTGT TGTGTTCTGA TAAGAGTCAG AGGTAACTCC CGTTGCGGTG
1751 CTGTTAACGG TGGAGGGCAG TGTAGTCTGA GCAGTACTCG TTGCTGCCGC
1801 GCGCGCCACC AGACATAATA GCTGACAGAC TAACAGACTG TTCCTTTCCA
1851 TGGGTCTTTT CTGCAGTCAC CGTCCTTAG ATCTGCTGTG CCTTCTAGTT
1901 GCCAGCCATC TGTTGTTTGC CCCTCCCCCG TGCCTTCCTT GACCCTGGAA
1951 GGTGCCACTC CCACTGTCCT TTCCTAATAA AATGAGGAAA TTGCATCGCA
2001 TTGTCTGAGT AGGTGTCATT CTATTCTGGG GGGTGGGGTG GGGCAGCACA
2051 GCAAGGGGGA GGATTGGGAA GACAATAGCA GGCATGCTGG GGATGCGGTG
2101 GGCTCTATGG GTACCCAGGT GCTGAAGAAT TGACCCGGTT CCTCCTGGGC
2151 CAGAAAGAAG CAGGCACATC CCCTTCTCTG TGACACACCC TGTCCACGCC
2201 CCTGGTTCTT AGTTCCAGCC CCACTCATAG GACACTCATA GCTCAGGAGG
2251 GCTCCGCCTT CAATCCCACC CGCTAAAGTA CTTGGAGCGG TCTCTCCCTC
2301 CCTCATCAGC CCACCAAACC AAACCTAGCC TCCAAGAGTG GGAAGAAATT
2351 AAAGCAAGAT AGGCTATTAA GTGCAGAGGG AGAGAAAATG CCTCCAACAT
2401 GTGAGGAAGT AATGAGAGAA ATCATAGAAT TTCTTCCGCT TCCTCGCTCA

Figure 7 (continued, p3/4)

2451 CTGACTCGCT GCGCTCGGTC GTTCGGCTGC GGCGAGCGGT ATCAGCTCAC

2501 TCAAAGGCGG TAATACGGTT ATCCACAGAA TCAGGGGATA ACGCAGGAAA

2551 GAACATGTGA GCAAAAGGCC AGCAAAAGGC CAGGAACCGT AAAAAGGCCG

2601 CGTTGCTGGC GTTTTTCCAT AGGCTCCGCC CCCCTGACGA GCATCACAAA

2651 AATCGACGCT CAAGTCAGAG GTGGCGAAAC CCGACAGGAC TATAAAGATA

2701 CCAGGCGTTT CCCCCTGGAA GCTCCCTCGT GCGCTCTCCT GTTCCGACCC

2751 TGCCGCTTAC CGGATACCTG TCCGCCTTTC TCCCTTCGGG AAGCGTGGCG

2801 CTTTCTCAAT GCTCACGCTG TAGGTATCTC AGTTCGGTGT AGGTCGTTCG

2851 CTCCAAGCTG GGCTGTGTGC ACGAACCCCC CGTTCAGCCC GACCGCTGCG

2901 CCTTATCCGG TAACTATCGT CTTGAGTCCA ACCCGGTAAG ACACGACTTA

2951 TCGCCACTGG CAGCAGCCAC TGGTAACAGG ATTAGCAGAG CGAGGTATGT

3001 AGGCGGTGCT ACAGAGTTCT TGAAGTGGTG GCCTAACTAC GGCTACACTA

3051 GAAGGACAGT ATTTGGTATC TGCGCTCTGC TGAAGCCAGT TACCTTCGGA

3101 AAAAGAGTTG GTAGCTCTTG ATCCGGCAAA CAAACCACCG CTGGTAGCGG

3151 TGGTTTTTTT GTTTGCAAGC AGCAGATTAC GCGCAGAAAA AAAGGATCTC

3201 AAGAAGATCC TTTGATCTTT TCTACGGGGT CTGACGCTCA GTGGAACGAA

3251 AACTCACGTT AAGGGATTTT GGTCATGAGA TTATCAAAAA GGATCTTCAC

3301 CTAGATCCTT TTAAATTAAA AATGAAGTTT TAAATCAATC TAAAGTATAT

3351 ATGAGTAAAC TTGGTCTGAC AGTTACCAAT GCTTAATCAG TGAGGCACCT

3401 ATCTCAGCGA TCTGTCTATT TCGTTCATCC ATAGTTGCCT GACTCCGGGG

3451 GGGGGGGGCG CTGAGGTCTG CCTCGTGAAG AAGGTGTTGC TGACTCATAC

3501 CAGGCCTGAA TCGCCCCATC ATCCAGCCAG AAAGTGAGGG AGCCACGGTT

3551 GATGAGAGCT TTGTTGTAGG TGGACCAGTT GGTGATTTTG AACTTTTGCT

3601 TTGCCACGGA ACGGTCTGCG TTGTCGGGAA GATGCGTGAT CTGATCCTTC

3651 AACTCAGCAA AAGTTCGATT TATTCAACAA AGCCGCCGTC CCGTCAAGTC

Figure 7 (continued, p4/4)

3701 AGCGTAATGC TCTGCCAGTG TTACAACCAA TTAACCAATT CTGATTAGAA

3751 AAACTCATCG AGCATCAAAT GAAACTGCAA TTTATTCATA TCAGGATTAT

3801 CAATACCATA TTTTTGAAAA AGCCGTTTCT GTAATGAAGG AGAAAACTCA

3851 CCGAGGCAGT TCCATAGGAT GGCAAGATCC TGGTATCGGT CTGCGATTCC

3901 GACTCGTCCA ACATCAATAC AACCTATTAA TTTCCCCTCG TCAAAAATAA

3951 GGTTATCAAG TGAGAAATCA CCATGAGTGA CGACTGAATC CGGTGAGAAT

4001 GGCAAAAGCT TATGCATTTC TTTCCAGACT TGTTCAACAG GCCAGCCATT

4051 ACGCTCGTCA TCAAAATCAC TCGCATCAAC CAAACCGTTA TTCATTCGTG

4101 ATTGCGCCTG AGCGAGACGA AATACGCGAT CGCTGTTAAA AGGACAATTA

4151 CAAACAGGAA TCGAATGCAA CCGGCGCAGG AACACTGCCA GCGCATCAAC

4201 AATATTTTCA CCTGAATCAG GATATTCTTC TAATACCTGG AATGCTGTTT

4251 TCCCGGGGAT CGCAGTGGTG AGTAACCATG CATCATCAGG AGTACGGATA

4301 AAATGCTTGA TGGTCGGAAG AGGCATAAAT TCCGTCAGCC AGTTTAGTCT

4351 GACCATCTCA TCTGTAACAT CATTGGCAAC GCTACCTTTG CCATGTTTCA

4401 GAAACAACTC TGGCGCATCG GGCTTCCCAT ACAATCGATA GATTGTCGCA

4451 CCTGATTGCC CGACATTATC GCGAGCCCAT TTATACCCAT ATAAATCAGC

4501 ATCCATGTTG GAATTTAATC GCGGCCTCGA GCAAGACGTT TCCCGTTGAA

4551 TATGGCTCAT AACACCCCTT GTATTACTGT TTATGTAAGC AGACAGTTTT

4601 ATTGTTCATG ATGATATATT TTTATCTTGT GCAATGTAAC ATCAGAGATT

4651 TTGAGACACA ACGTGGCTTT CCCCCCCCCC CCATTATTGA AGCATTTATC

4701 AGGGTTATTG TCTCATGAGC GGATACATAT TTGAATGTAT TTAGAAAAAT

4751 AAACAAATAG GGGTTCCGCG CACATTTCCC CGAAAAGTGC CACCTGACGT

4801 CTAAGAAACC ATTATTATCA TGACATTAAC CTATAAAAAT AGGCGTATCA

4851 CGAGGCCCTT TCGTC

Figure 8: CMVintaBGH Sequence, SEQ. ID:11:

1 ATTGGCTATT GGCCATTGCA TACGTTGTAT CCATATCATA ATATGTACAT

51 TTATATTGGC TCATGTCCAA CATTACCGCC ATGTTGACAT TGATTATTGA

101 CTAGTTATTA ATAGTAATCA ATTACGGGGT CATTAGTTCA TAGCCCATAT

151 ATGGAGTTCC GCGTTACATA ACTTACGGTA AATGGCCCGC CTGGCTGACC

201 GCCCAACGAC CCCCGCCCAT TGACGTCAAT AATGACGTAT GTTCCCATAG

251 TAACGCCAAT AGGGACTTTC CATTGACGTC AATGGGTGGA GTATTTACGG

301 TAAACTGCCC ACTTGGCAGT ACATCAAGTG TATCATATGC CAAGTACGCC

351 CCCTATTGAC GTCAATGACG GTAAATGGCC CGCCTGGCAT TATGCCCAGT

401 ACATGACCTT ATGGGACTTT CCTACTTGGC AGTACATCTA CGTATTAGTC

451 ATCGCTATTA CCATGGTGAT GCGGTTTTGG CAGTACATCA ATGGGCGTGG

501 ATAGCGGTTT GACTCACGGG GATTTCCAAG TCTCCACCCC ATTGACGTCA

551 ATGGGAGTTT GTTTTGGCAC CAAAATCAAC GGGACTTTCC AAAATGTCGT

601 AACAACTCCG CCCCATTGAC GCAAATGGGC GGTAGGCGTG TACGGTGGGA

651 GGTCTATATA AGCAGAGCTC GTTTAGTGAA CCGTCAGATC GCCTGGAGAC

701 GCCATCCACG CTGTTTTGAC CTCCATAGAA GACACCGGGA CCGATCCAGC

751 CTCCGCGGCC GGGAACGGTG CATTGGAACG CGGATTCCCC GTGCCAAGAG

801 TGACGTAAGT ACCGCCTATA GAGTCTATAG GCCCACCCCC TTGGCTTCTT

851 ATGCATGCTA TACTGTTTTT GGCTTGGGGT CTATACACCC CCGCTTCCTC

901 ATGTTATAGG TGATGGTATA GCTTAGCCTA TAGGTGTGGG TTATTGACCA

951 TTATTGACCA CTCCCCTATT GGTGACGATA CTTTCCATTA CTAATCCATA

1001 ACATGGCTCT TTGCCACAAC TCTCTTTATT GGCTATATGC CAATACACTG

1051 TCCTTCAGAG ACTGACACGG ACTCTGTATT TTTACAGGAT GGGGTCTCAT

1101 TTATTATTTA CAAATTCACA TATACAACAC CACCGTCCCC AGTGCCCGCA

1151 GTTTTTATTA AACATAACGT GGGATCTCCA CGCGAATCTC GGGTACGTGT

1201 TCCGGACATG GGCTCTTCTC CGGTAGCGGC GGAGCTTCTA CATCCGAGCC

Figure 8 (continued, p2/2)

1251 CTGCTCCCAT GCCTCCAGCG ACTCATGGTC GCTCGGCAGC TCCTTGCTCC

1301 TAACAGTGGA GGCCAGACTT AGGCACAGCA CGATGCCCAC CACCACCAGT

1351 GTGCCGCACA AGGCCGTGGC GGTAGGGTAT GTGTCTGAAA ATGAGCTCGG

1401 GGAGCGGGCT TGCACCGCTG ACGCATTTGG AAGACTTAAG GCAGCGGCAG

1451 AAGAAGATGC AGGCAGCTGA GTTGTTGTGT TCTGATAAGA GTCAGAGGTA

1501 ACTCCCGTTG CGGTGCTGTT AACGGTGGAG GGCAGTGTAG TCTGAGCAGT

1551 ACTCGTTGCT GCCGCGCGCG CCACCAGACA TAATAGCTGA CAGACTAACA

1601 GACTGTTCCT TTCCATGGGT CTTTTCTGCA GTCACCGTCC TTAGATCTG

1651 CTGTGCCTTC TAGTTGCCAG CCATCTGTTG TTTGCCCCTC CCCCGTGCCT

1701 TCCTTGACCC TGGAAGGTGC CACTCCCACT GTCCTTTCCT AATAAAATGA

1751 GGAAATTGCA TCGCATTGTC TGAGTAGGTG TCATTCTATT CTGGGGGGTG

1801 GGGTGGGGCA GCACAGCAAG GGGGAGGATT GGGAAGACAA TAGCAGGCAT

1851 GCTGGGGATG CGGTGGGCTC TATGGGTACC CAGGTGCTGA AGAATTGACC

1901 CGGTTCCTCC TGGGCCAGAA AGAAGCAGGC ACATCCCCTT CTCTGTGACA

1951 CACCCTGTCC ACGCCCCTGG TTCTTAGTTC CAGCCCCACT CATAGGACAC

2001 TCATAGCTCA GGAGGGCTCC GCCTTCAATC CCACCCGCTA AAGTACTTGG

2051 AGCGGTCTCT CCCTCCCTCA TCAGCCCACC AAACCAAACC TAGCCTCCAA

2101 GAGTGGGAAG AAATTAAAGC AAGATAGGCT ATTAAGTGCA GAGGGAGAGA

2151 AAATGCCTCC AACATGTGAG GAAGTAATGA GAGAAATCAT AGAATTC

FIGURE 9

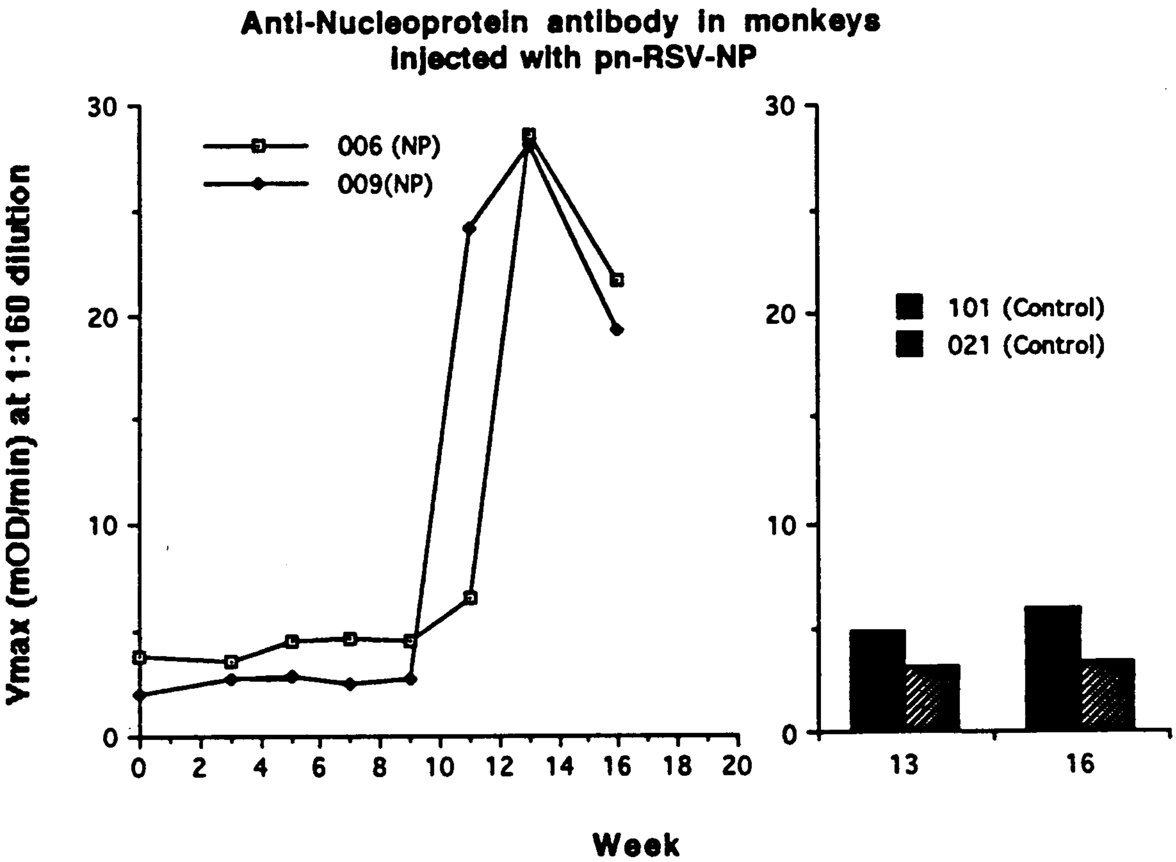
Anti-Nucleoprotein antibody in monkeys injected with pn-RSV-NP
Vmax (mOD/min) at 1:160 dilution
006 (NP)
009(NP)
101 (Control)
021 (Control)
0
10
20
30
0
2
4
6
8
10
12
14
16
18
20
13
16
Week

FIGURE 10

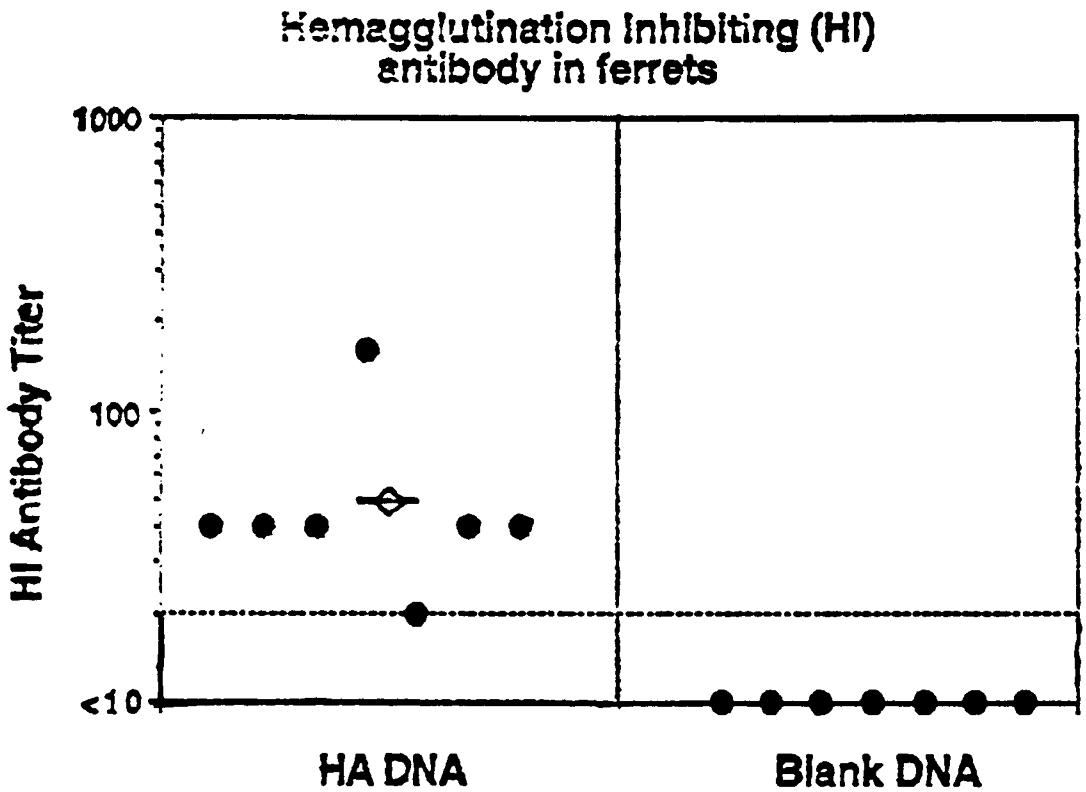
Hemagglutination Inhibiting (HI)
antibody in ferrets
1000
100
<10
HI Antibody Titer
HA DNA
Blank DNA

FIGURE 11

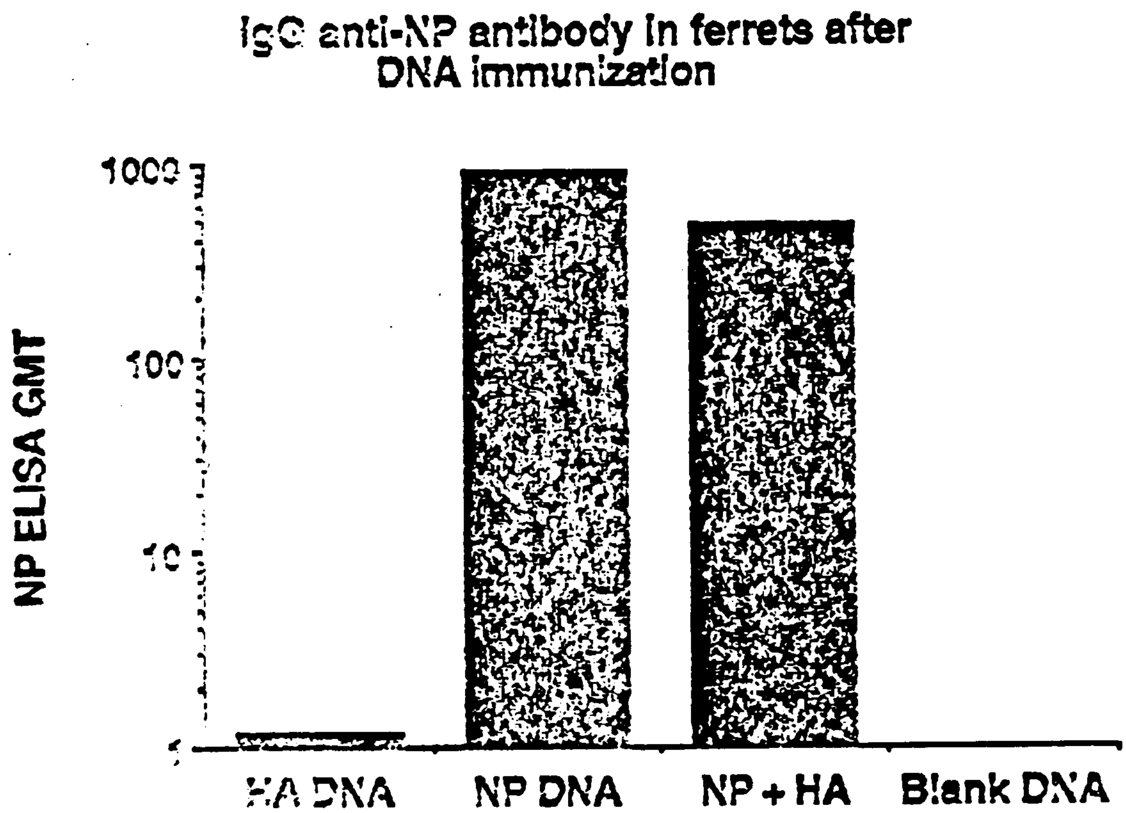
IgG anti-NP antibody in ferrets after DNA immunization
NP ELISA GMT
1000
100
10
1
HA DNA
NP DNA
NP + HA
Blank DNA

FIGURE 12

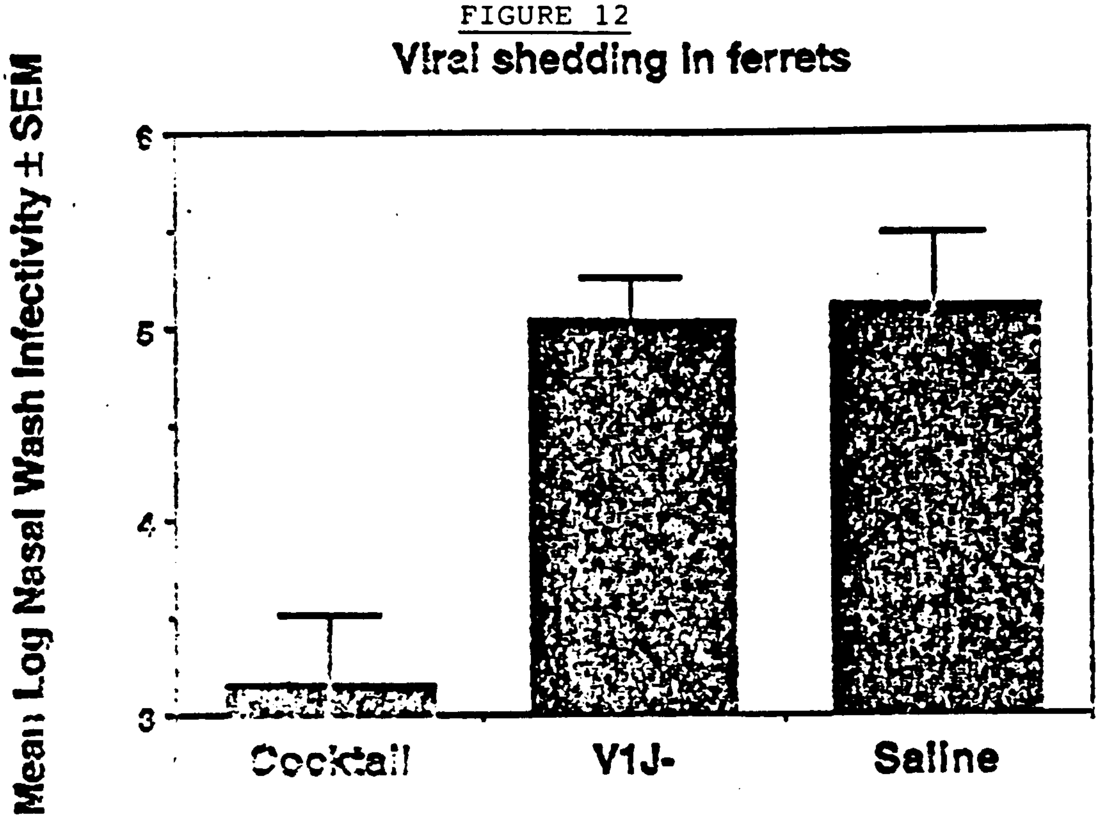
Viral shedding in ferrets
Mean Log Nasal Wash Infectivity ± SEM
6
5
4
3
Cocktail
V1J-
Saline

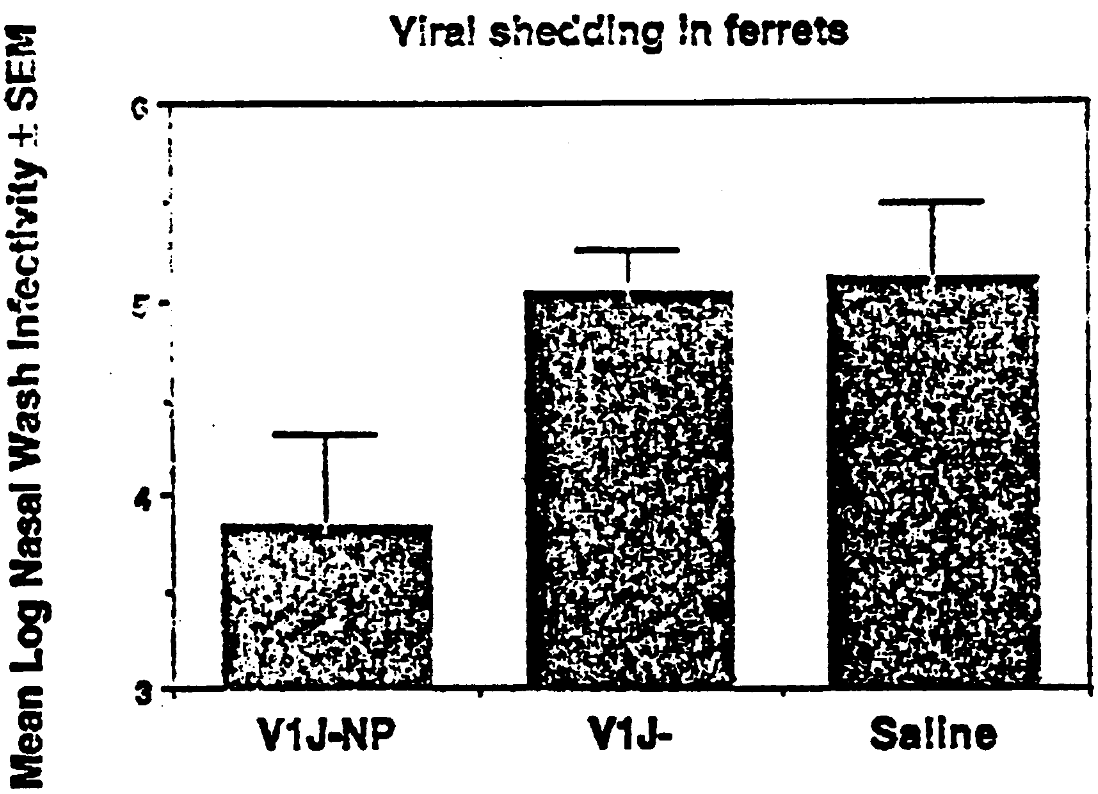
Viral shedding in ferrets
Mean Log Nasal Wash Infectivity ± SEM
6
5
4
3
V1J-NP
V1J-
Saline

FIGURE 13

# PLASMID DNA CONSTRUCTS

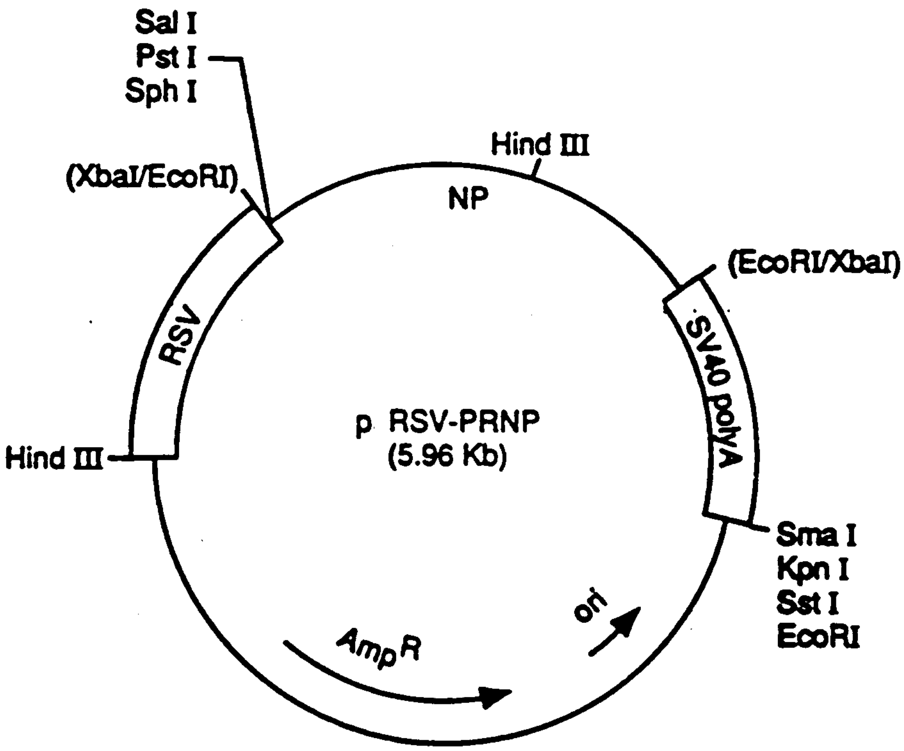


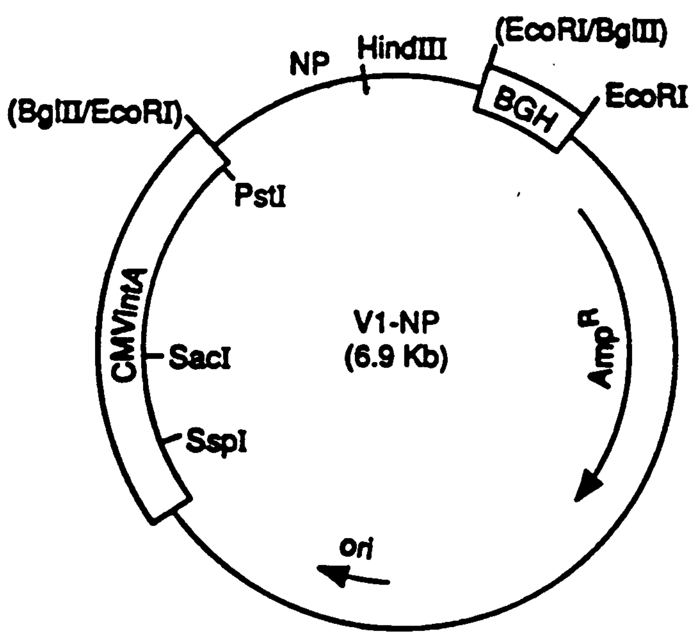

FIGURE 14

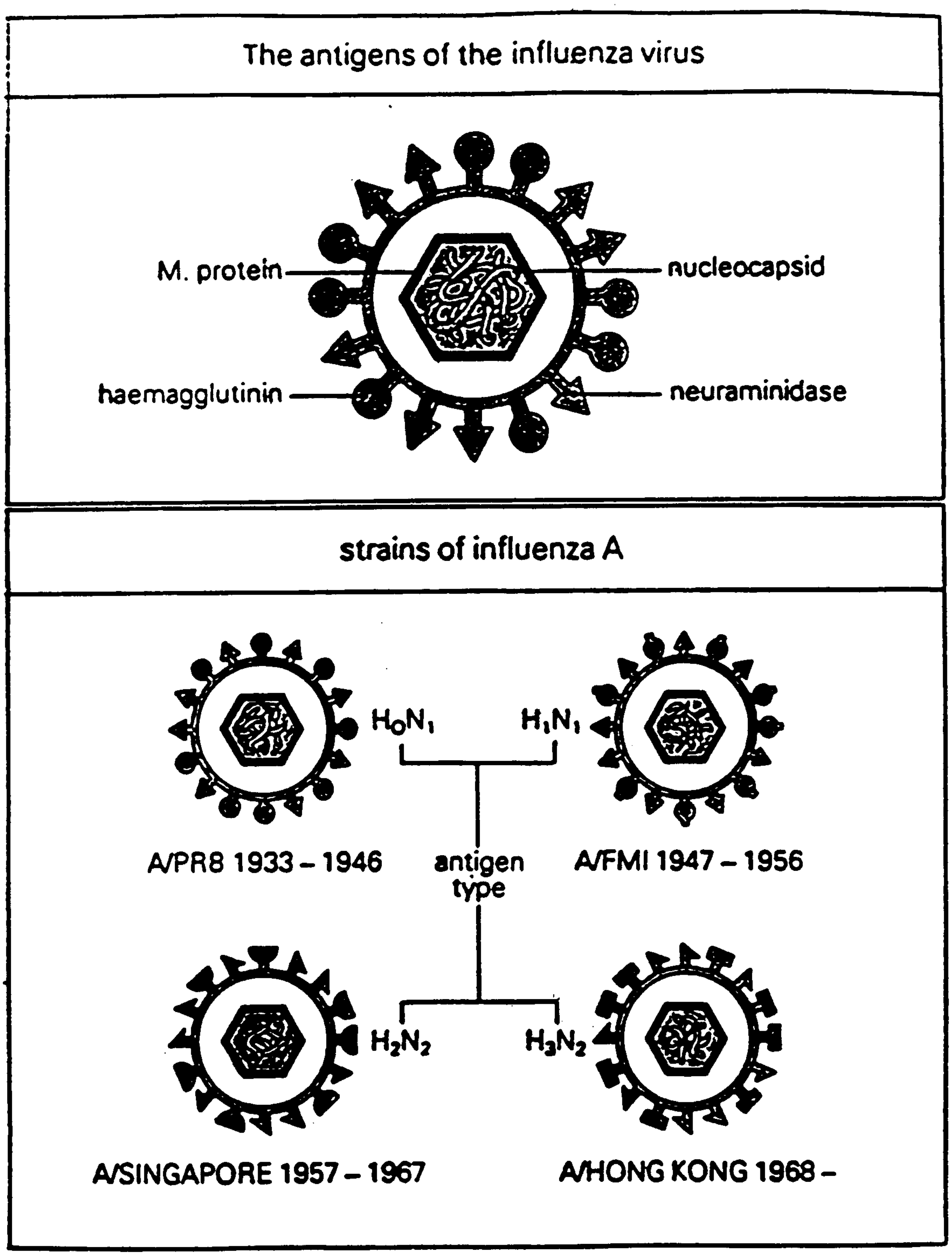
The antigens of the influenza virus
M. protein
nucleocapsid
haemagglutinin
neuraminidase
strains of influenza A
$H_0N_1$
$H_1N_1$
A/PR8 1933 – 1946
antigen type
A/FMI 1947 – 1956
$H_2N_2$
$H_3N_2$
A/SINGAPORE 1957 – 1967
A/HONG KONG 1968 –

FIGURE 15

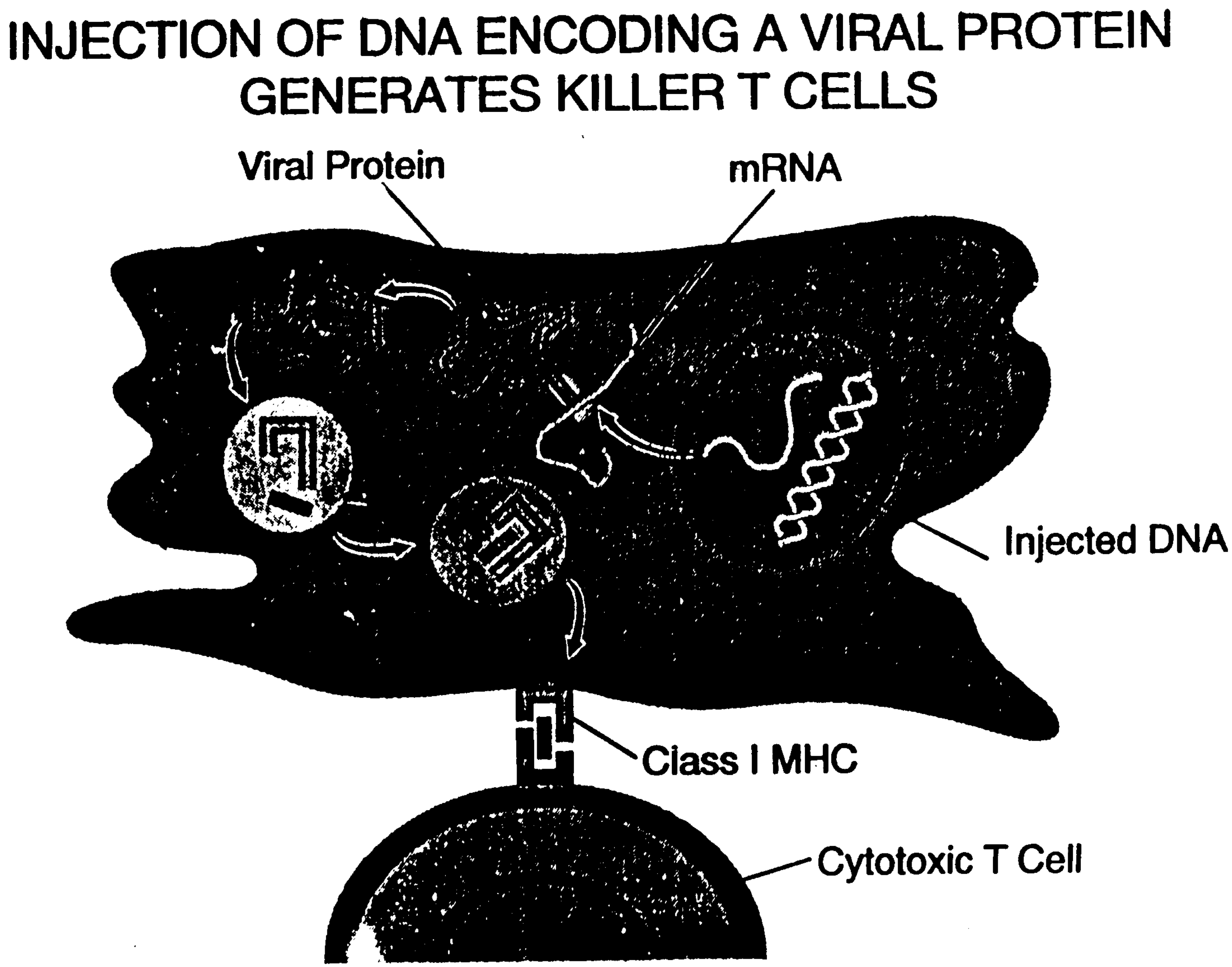
INJECTION OF DNA ENCODING A VIRAL PROTEIN
GENERATES KILLER T CELLS
Viral Protein
mRNA
Injected DNA
Class I MHC
Cytotoxic T Cell

Figure 16

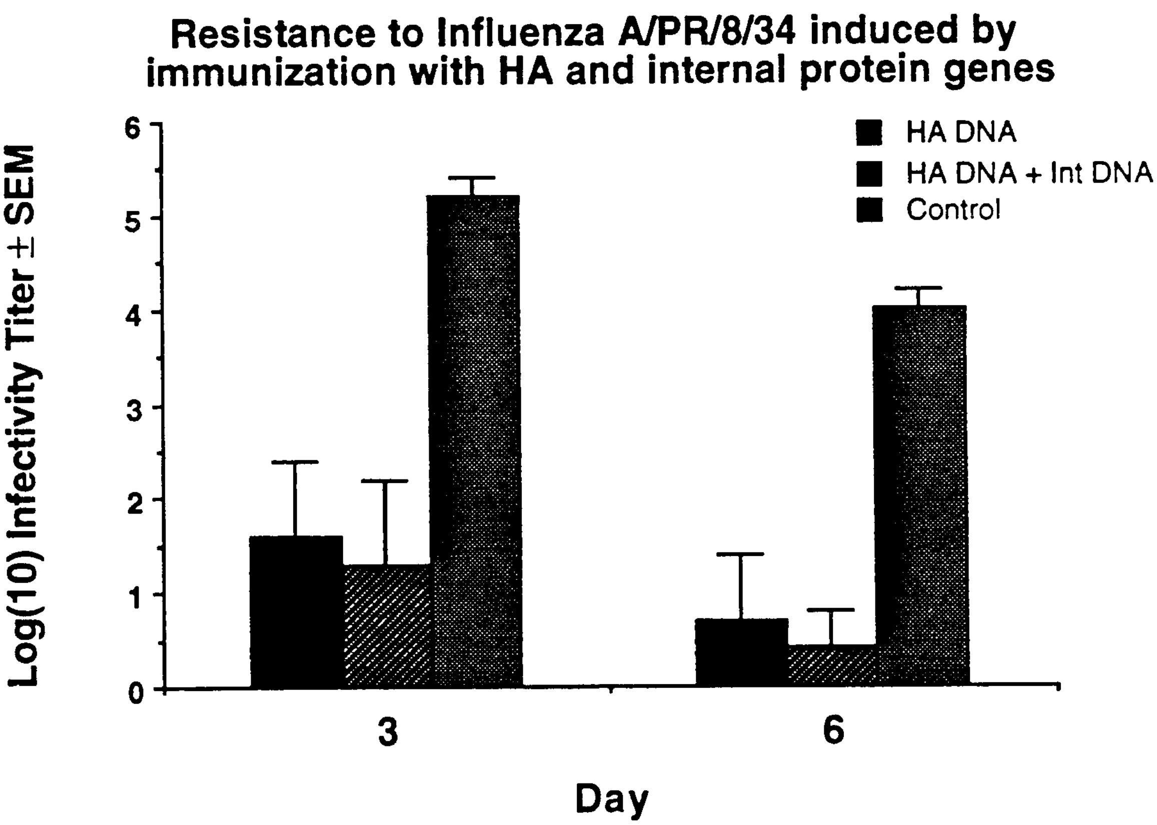
Resistance to Influenza A/PR/8/34 induced by immunization with HA and internal protein genes
Log(10) Infectivity Titer ± SEM
0
1
2
3
4
5
6
HA DNA
HA DNA + Int DNA
Control
3
6
Day

FIGURE 17

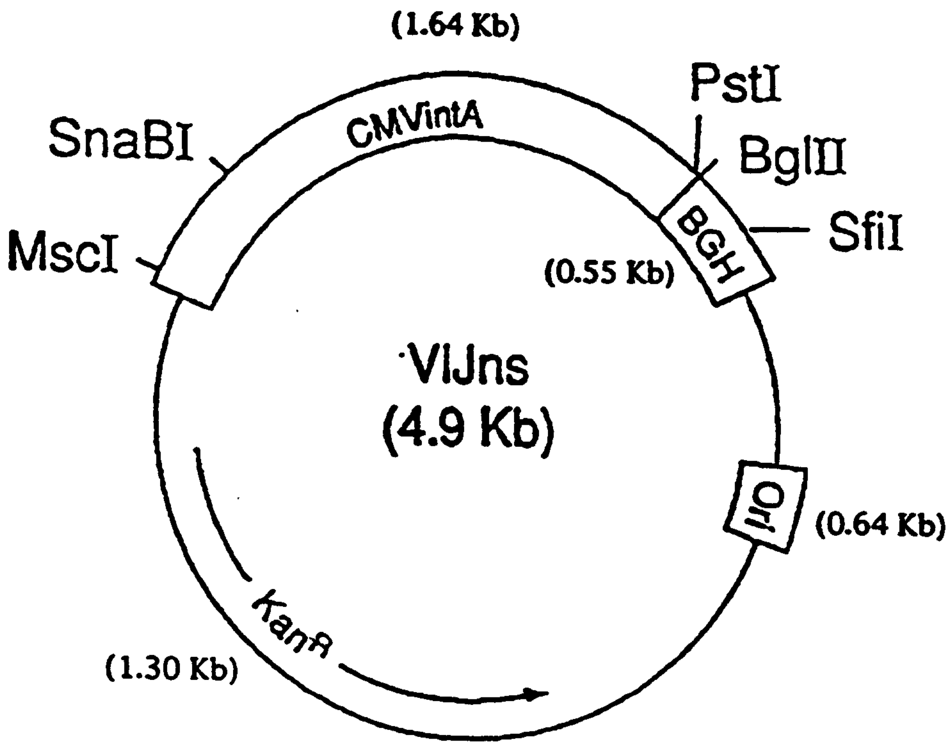
(1.64 Kb)
PstI
SnaBI
CMVintA
BglII
BGH
SfiI
MscI
(0.55 Kb)
VIJns
(4.9 Kb)
Ori
(0.64 Kb)
KanR
(1.30 Kb)

Figure 18

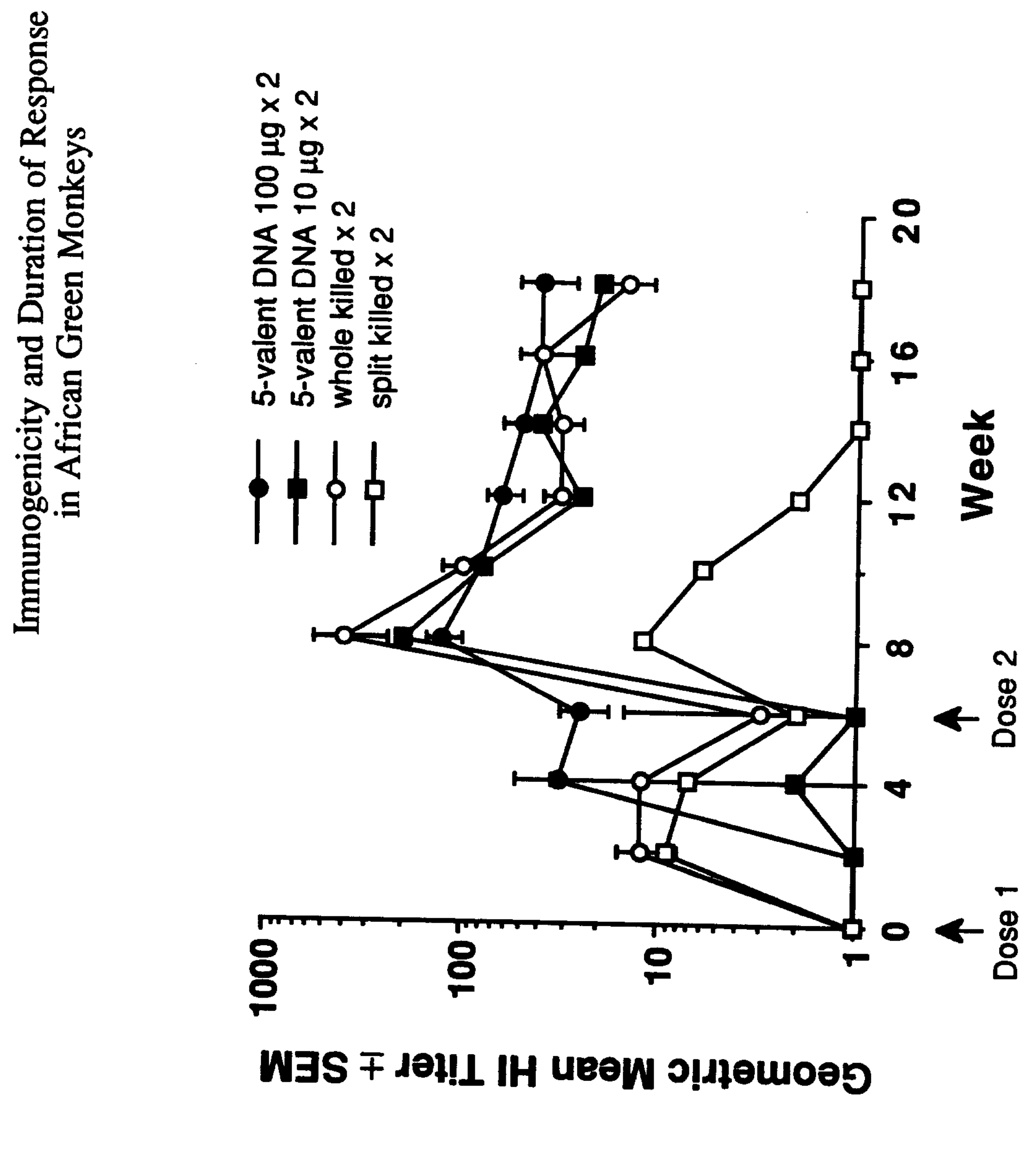
Immunogenicity and Duration of Response
in African Green Monkeys
5-valent DNA 100 µg x 2
5-valent DNA 10 µg x 2
whole killed x 2
split killed x 2
Geometric Mean HI Titer ± SEM
1000
100
10
1
0
4
8
12
16
20
Week
Dose 1
Dose 2

Figure 19

Cytotoxic T Cells 6 Months After Immunization With NP DNA

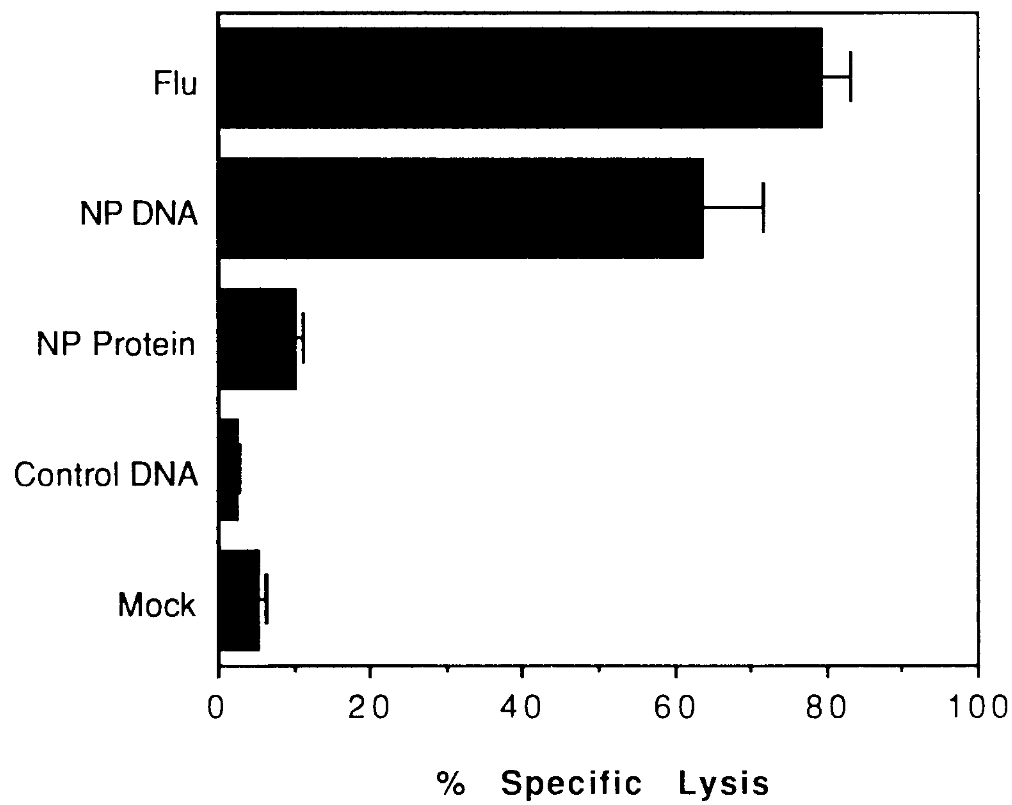

Figure 20

Expression of NP by Muscle Cells In Vivo is Sufficient for Generation of CTL

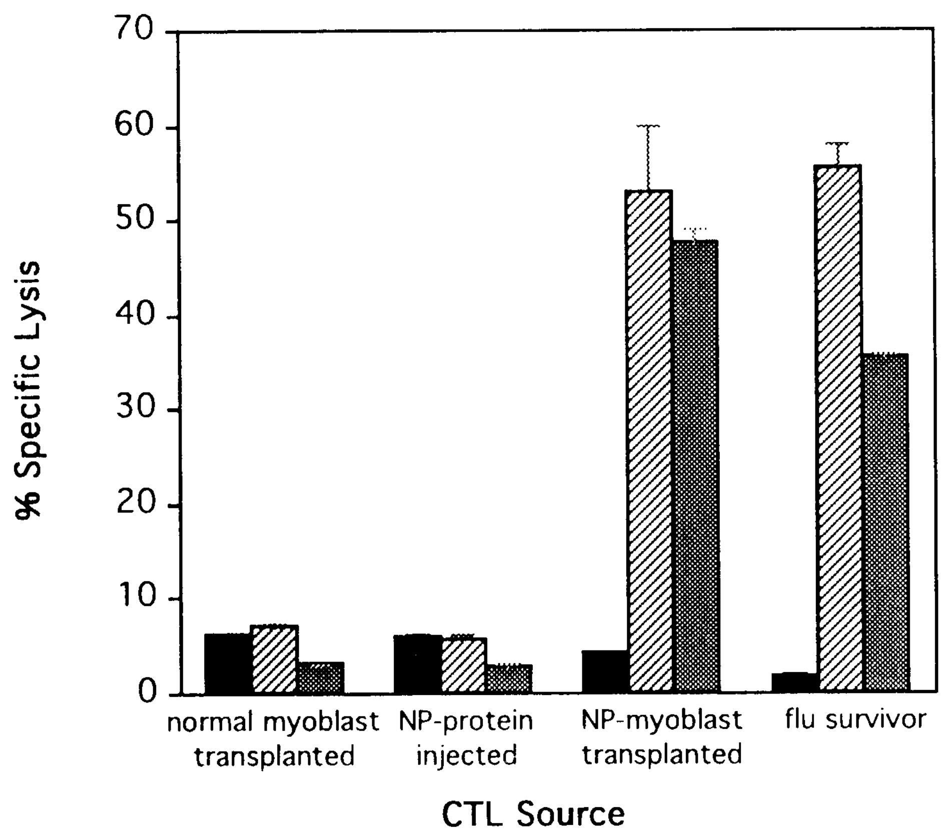

Figure 21

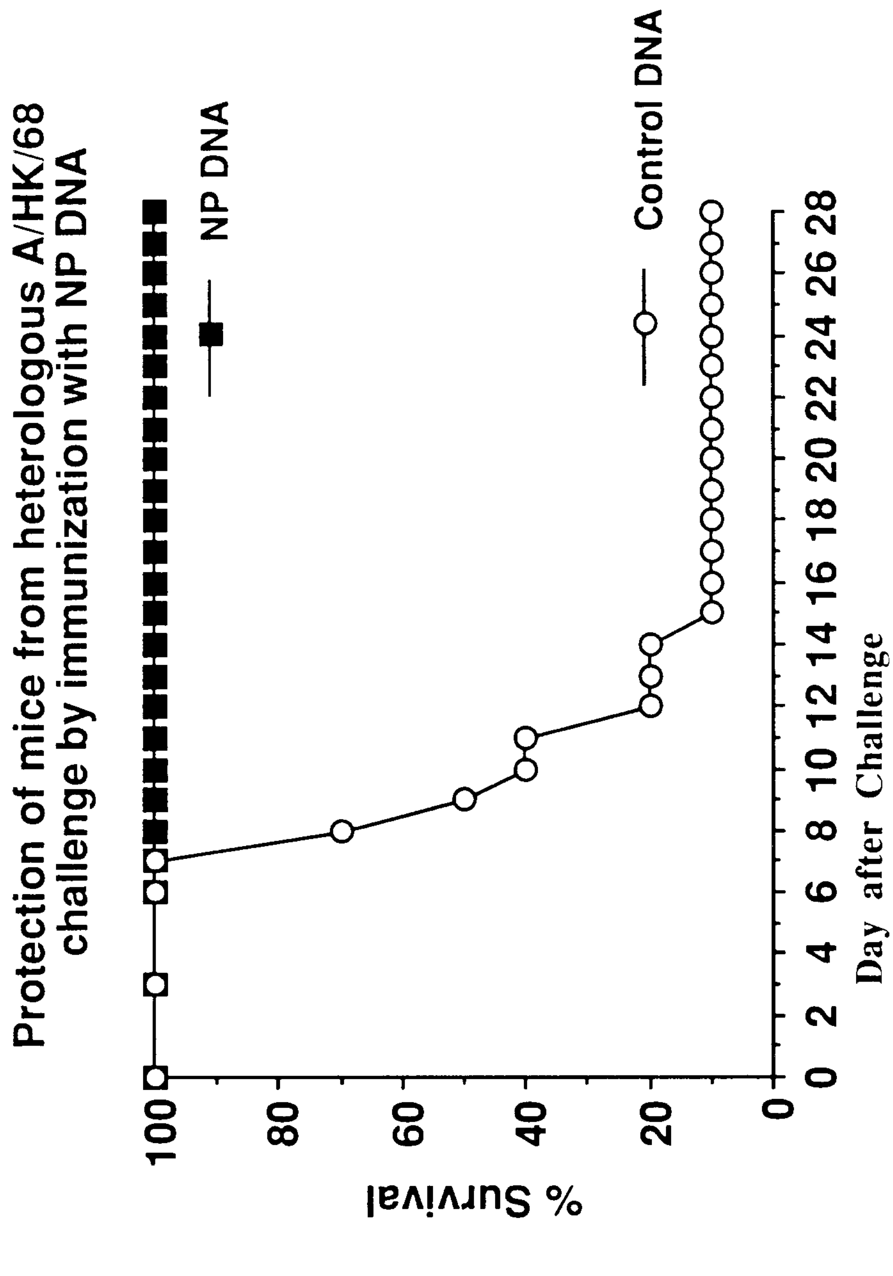
Protection of mice from heterologous A/HK/68
challenge by immunization with NP DNA
NP DNA
Control DNA
% Survival
100
80
60
40
20
0
0 2 4 6 8 10 12 14 16 18 20 22 24 26 28
Day after Challenge

Figure 22

Protection from weight loss during heterologous challenge with A/HK/68 by immunization with NP DNA

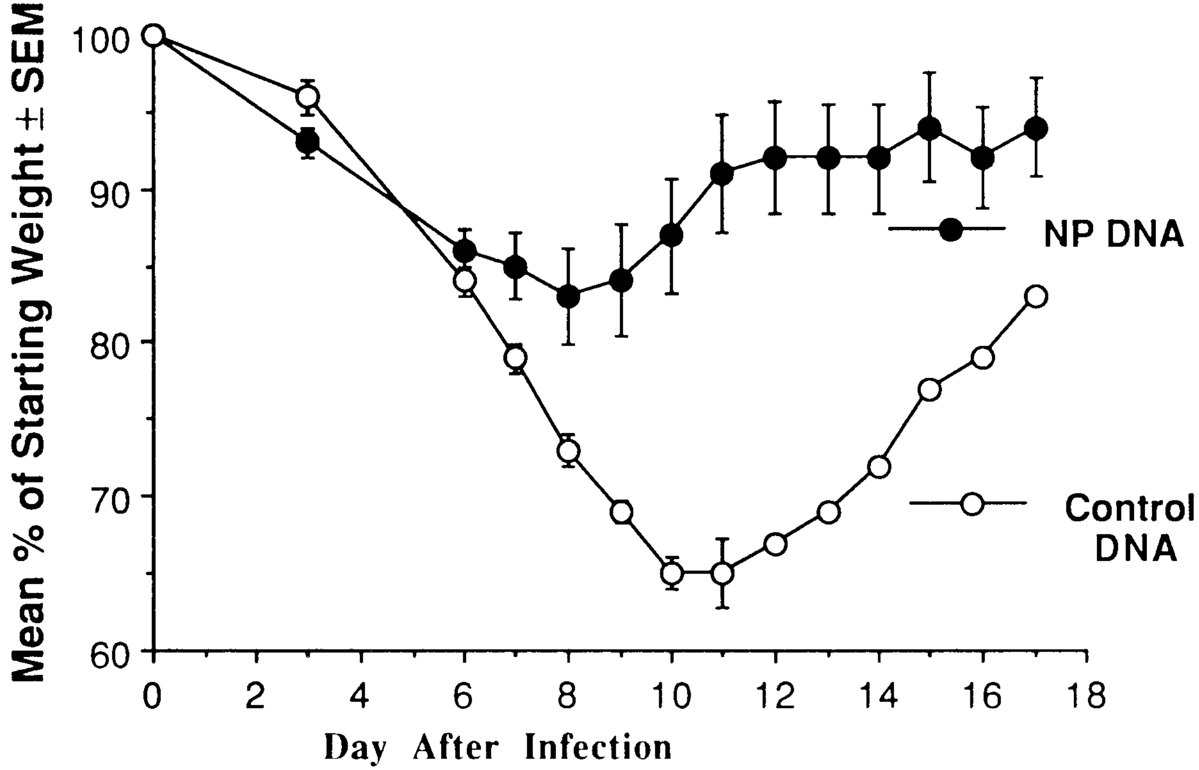

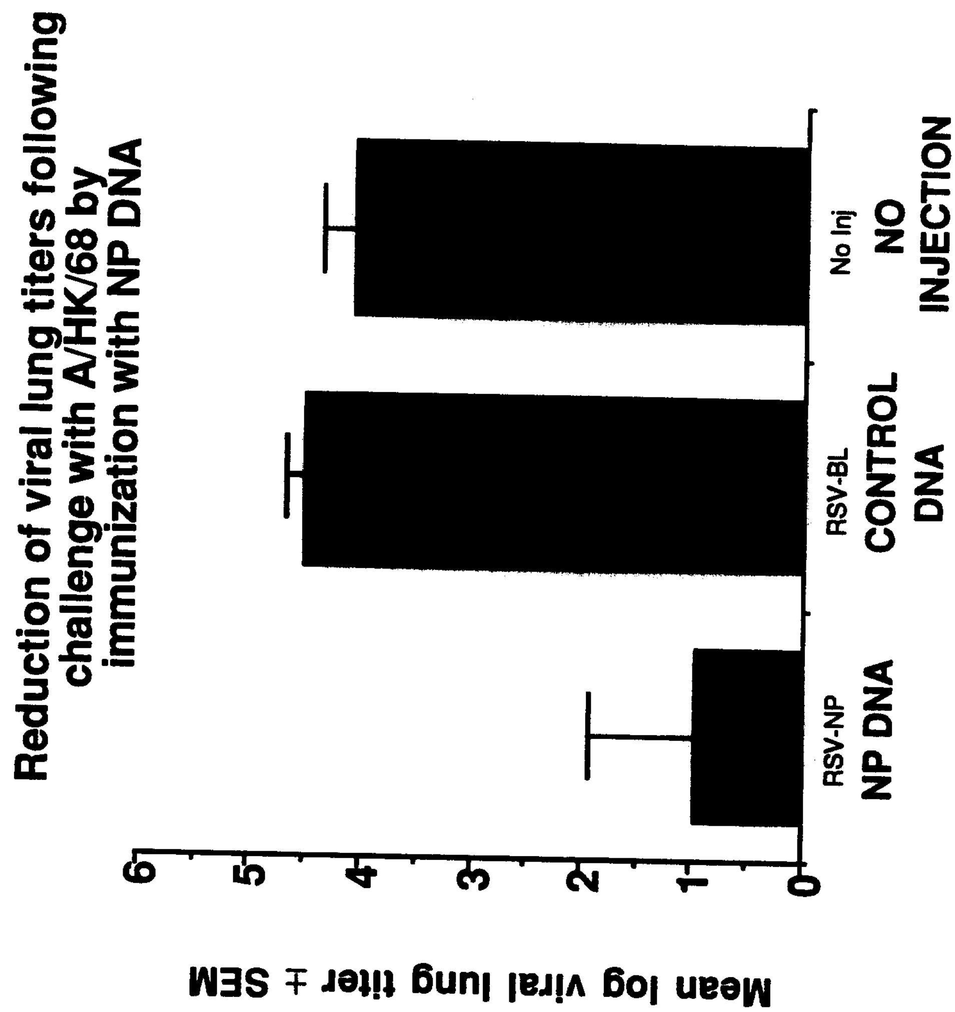
Reduction of viral lung titers following challenge with A/HK/68 by immunization with NP DNA
Mean log viral lung titer ± SEM
0
1
2
3
4
5
6
RSV-NP
NP DNA
RSV-BL
CONTROL DNA
No Inj
NO INJECTION

Figure 23

Figure 24

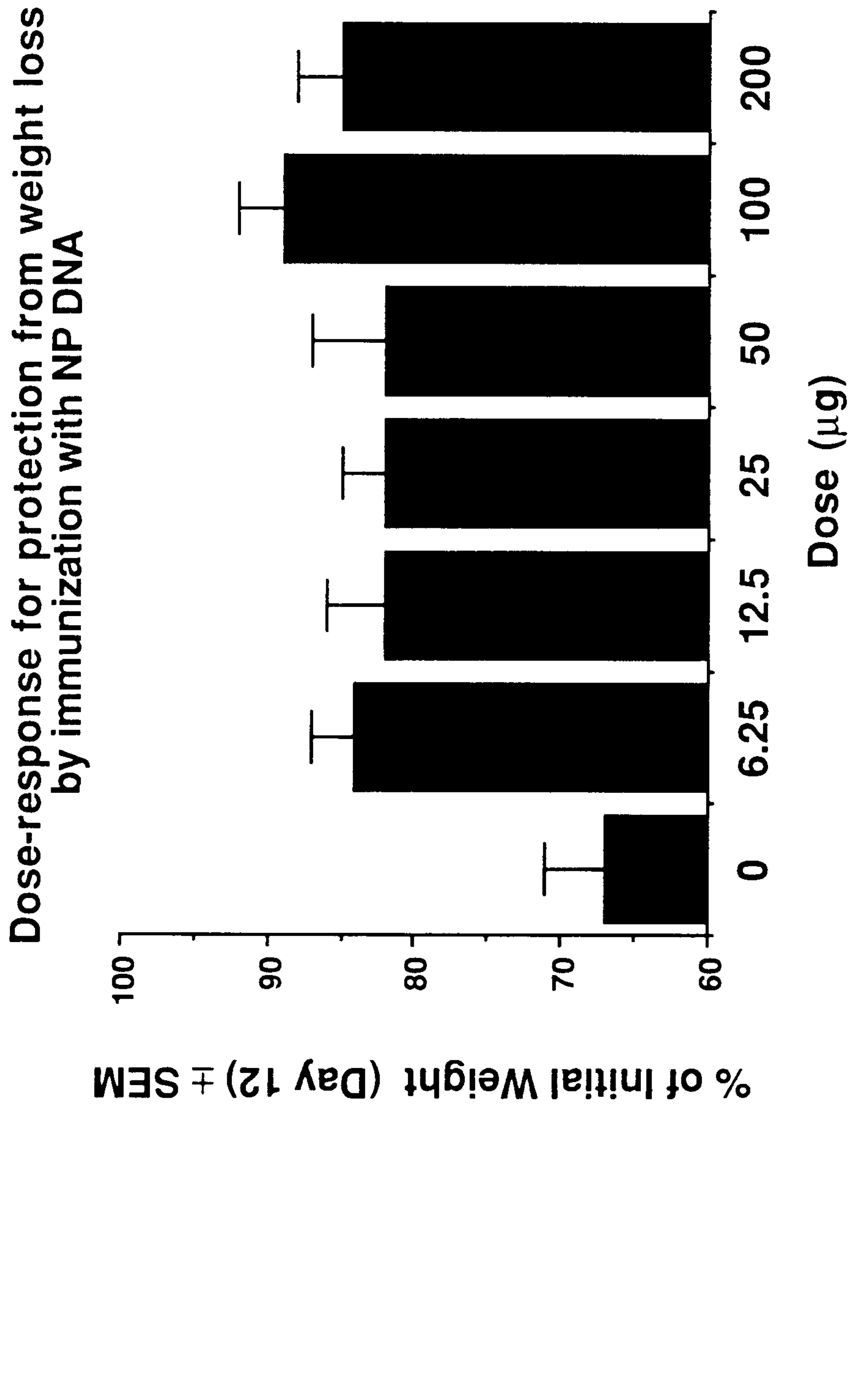
Dose-response for protection from weight loss
by immunization with NP DNA
% of Initial Weight (Day 12) ± SEM
100
90
80
70
60
0
6.25
12.5
25
50
100
200
Dose (μg)

Figure 25

**Duration of protection of mice against heterologous challenge with A/HK/68 after immunization with NP DNA (Group Weights)**

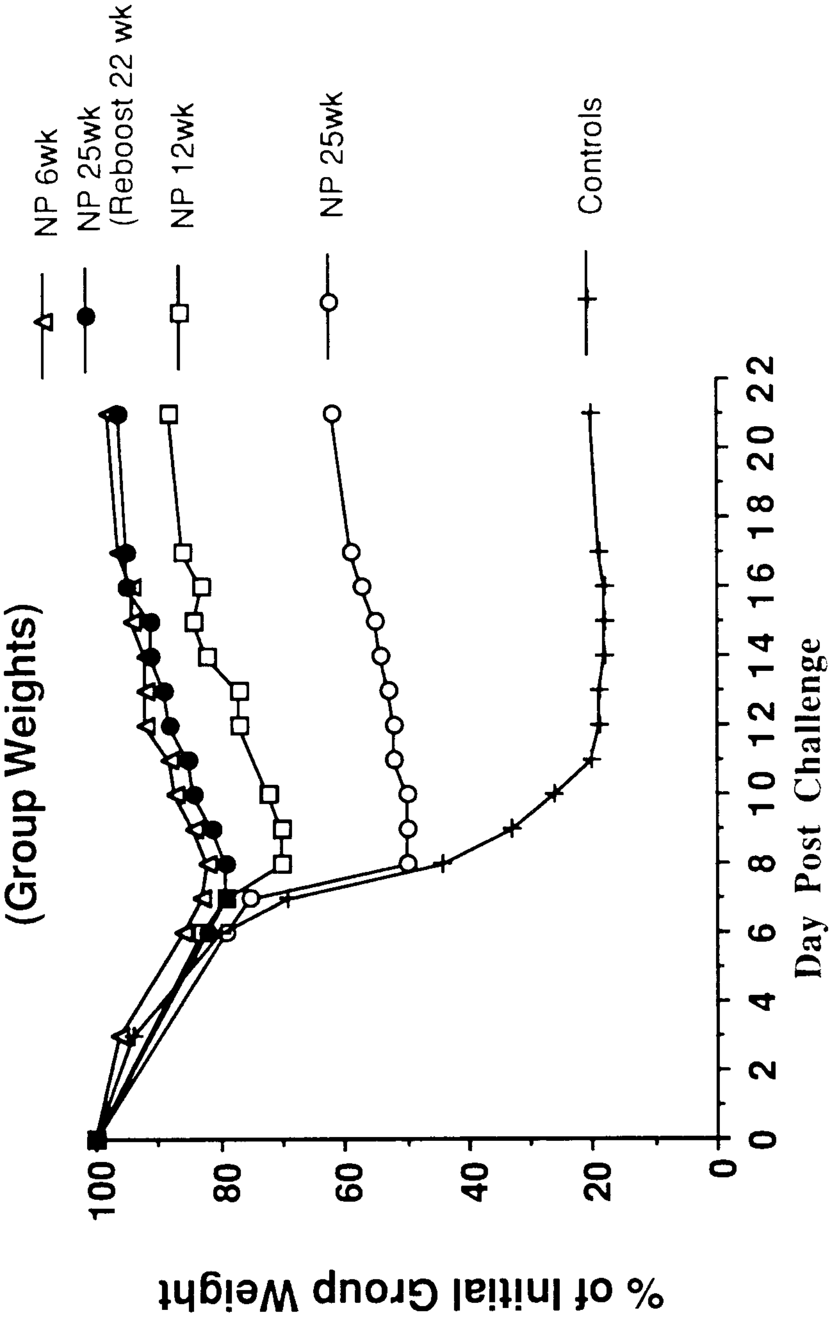

Figure 26

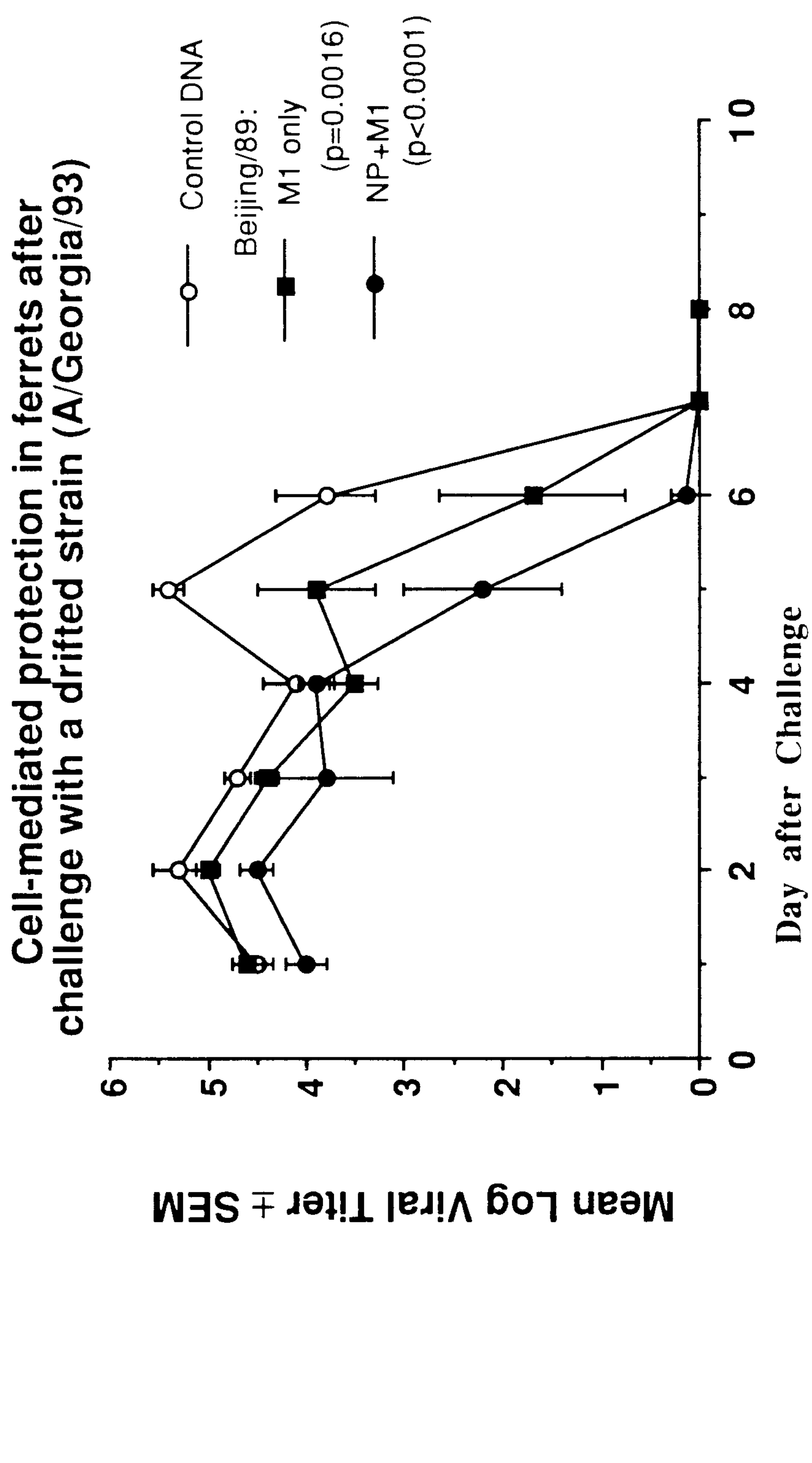
Cell-mediated protection in ferrets after
challenge with a drifted strain (A/Georgia/93)
Control DNA
Beijing/89:
M1 only
(p=0.0016)
NP+M1
(p<0.0001)
Mean Log Viral Titer ± SEM
Day after Challenge
0
1
2
3
4
5
6
0
2
4
6
8
10

Figure 27

Protection of ferrets from homologous challenge by internal protein DNA vaccination

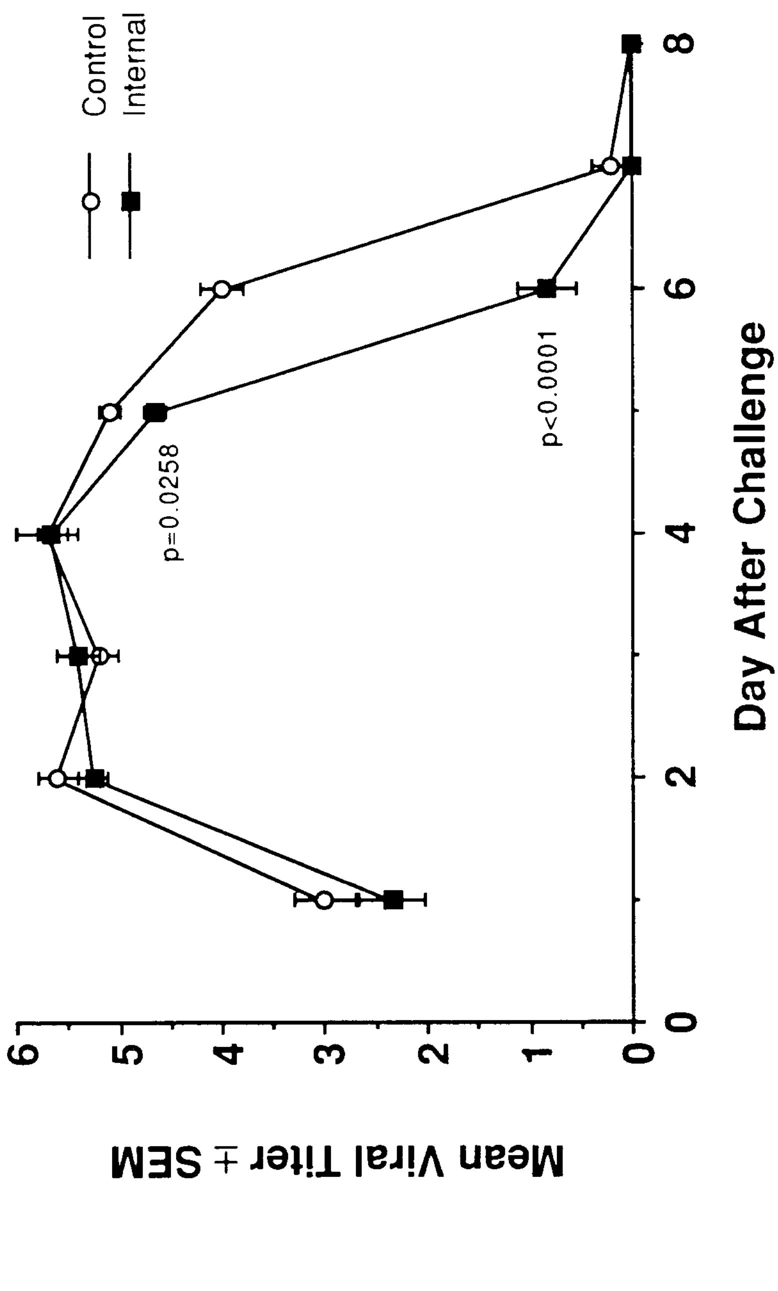

Figure 28

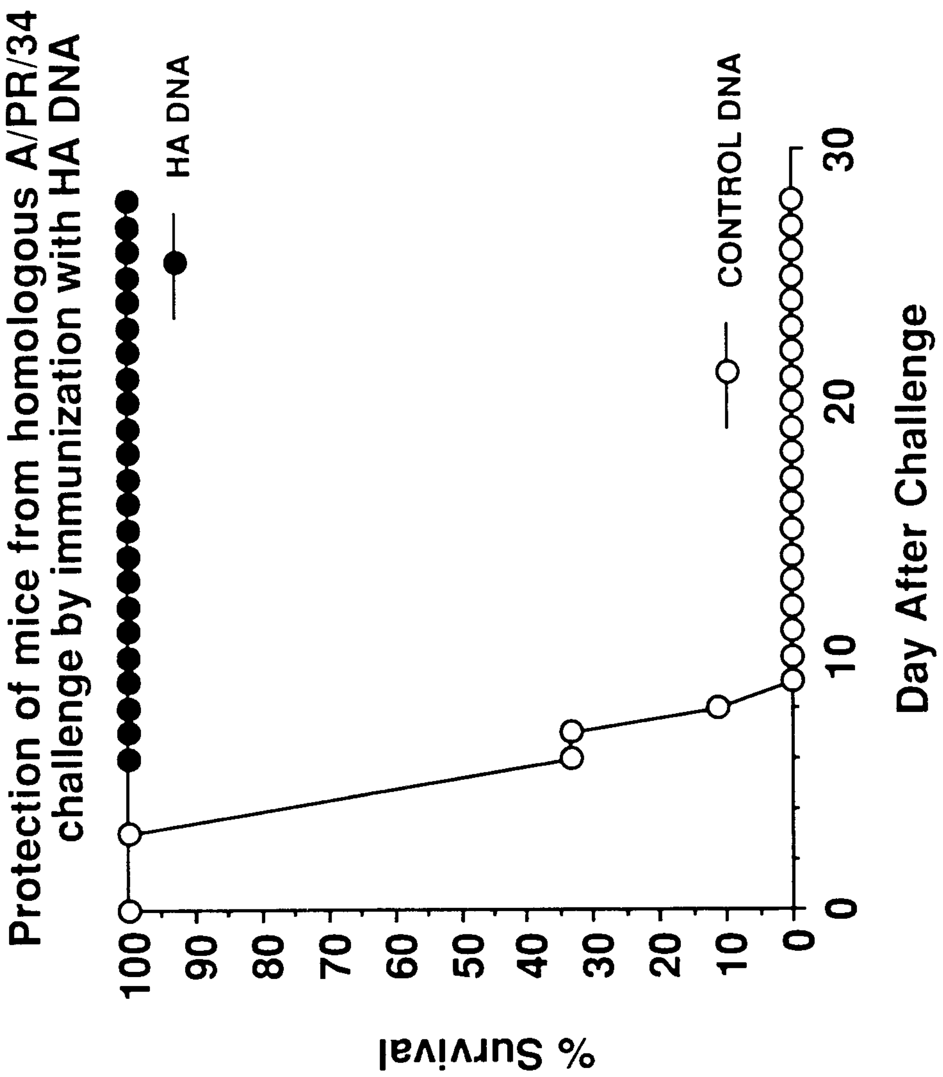
Protection of mice from homologous A/PR/34
challenge by immunization with HA DNA
HA DNA
CONTROL DNA
% Survival
100
90
80
70
60
50
40
30
20
10
0
0
10
20
30
Day After Challenge

Figure 29

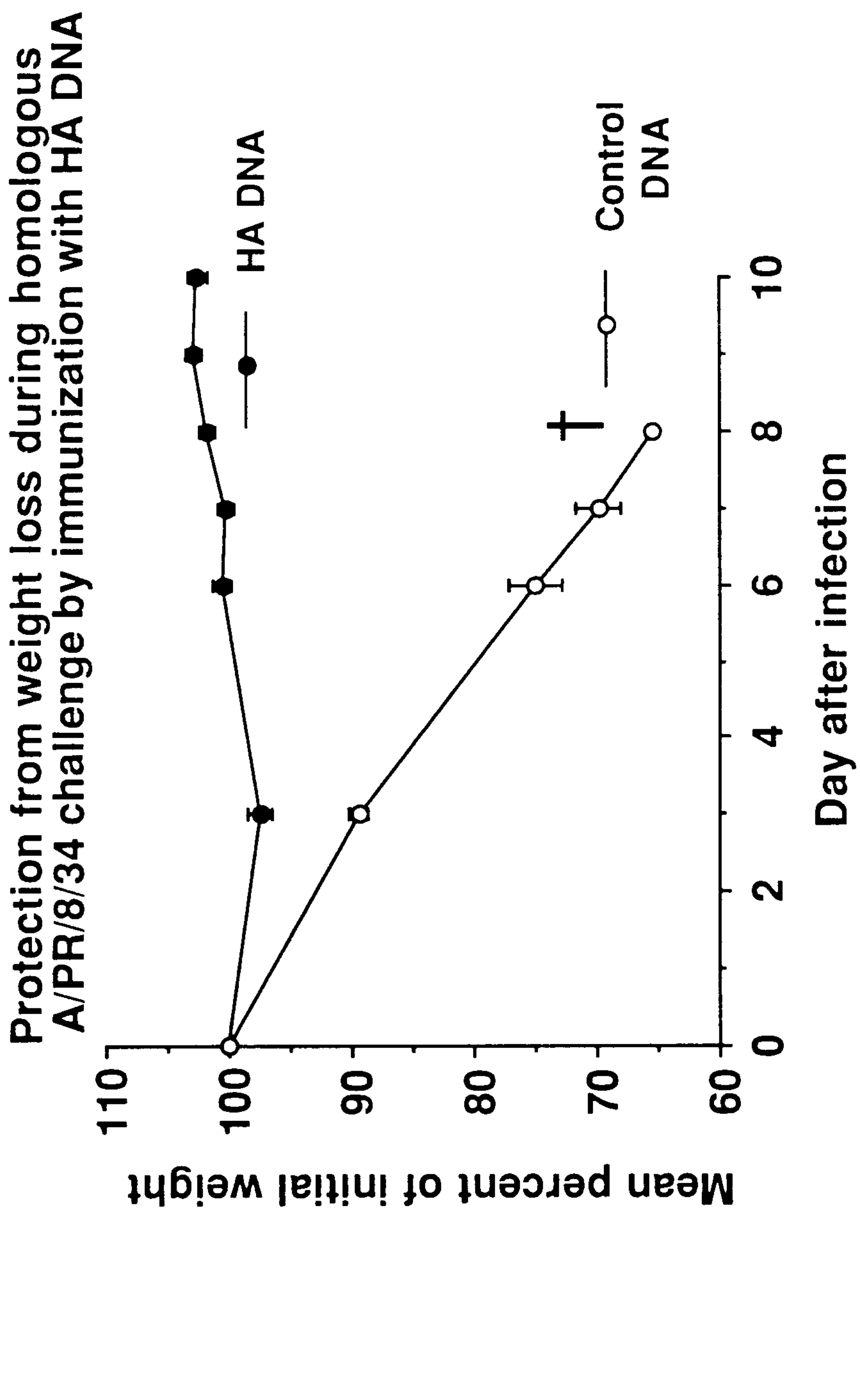
Protection from weight loss during homologous
A/PR/8/34 challenge by immunization with HA DNA
Mean percent of initial weight
110
100
90
80
70
60
0
2
4
6
8
10
Day after infection
HA DNA
Control
DNA

Figure 30

TITRATION OF HA DNA AGAINST HOMOLOGOUS CHALLENGE (A/PR/34)

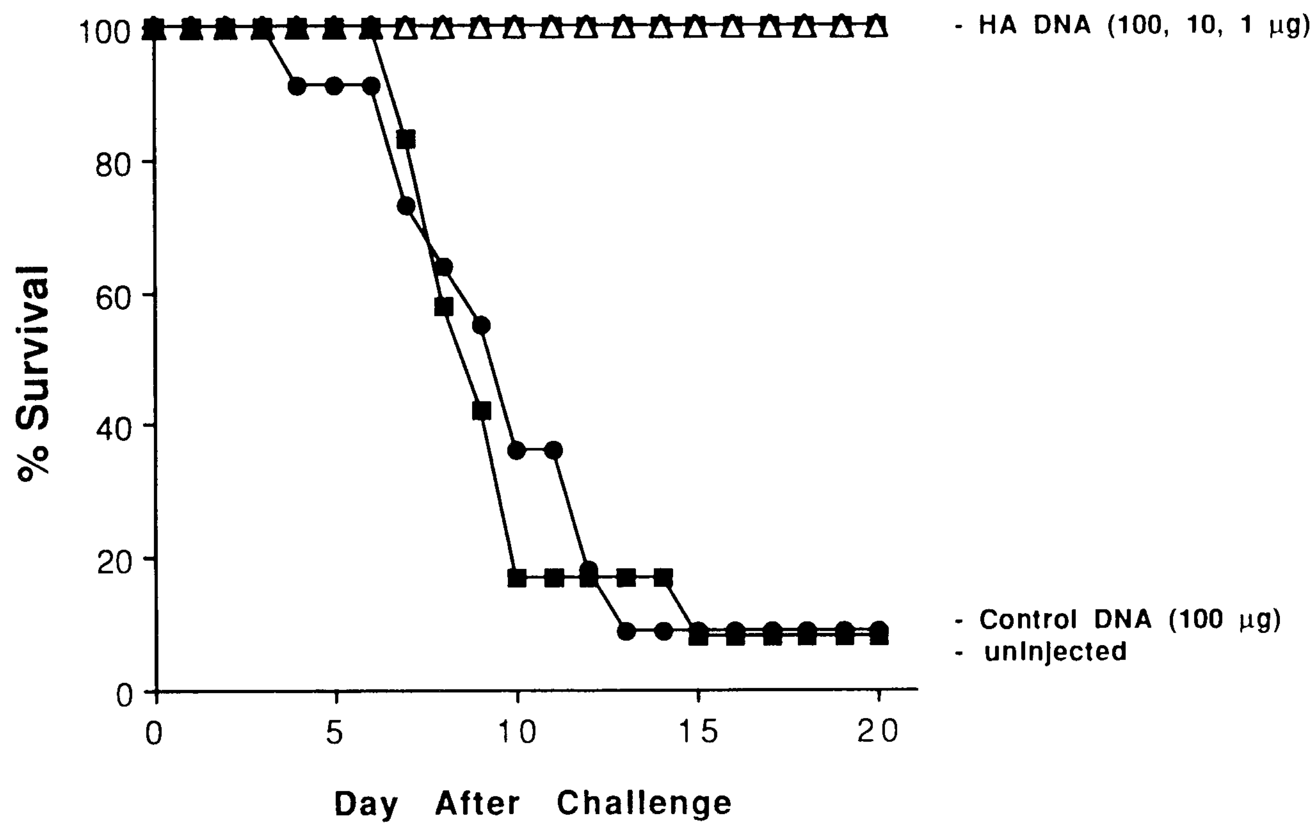

Figure 31

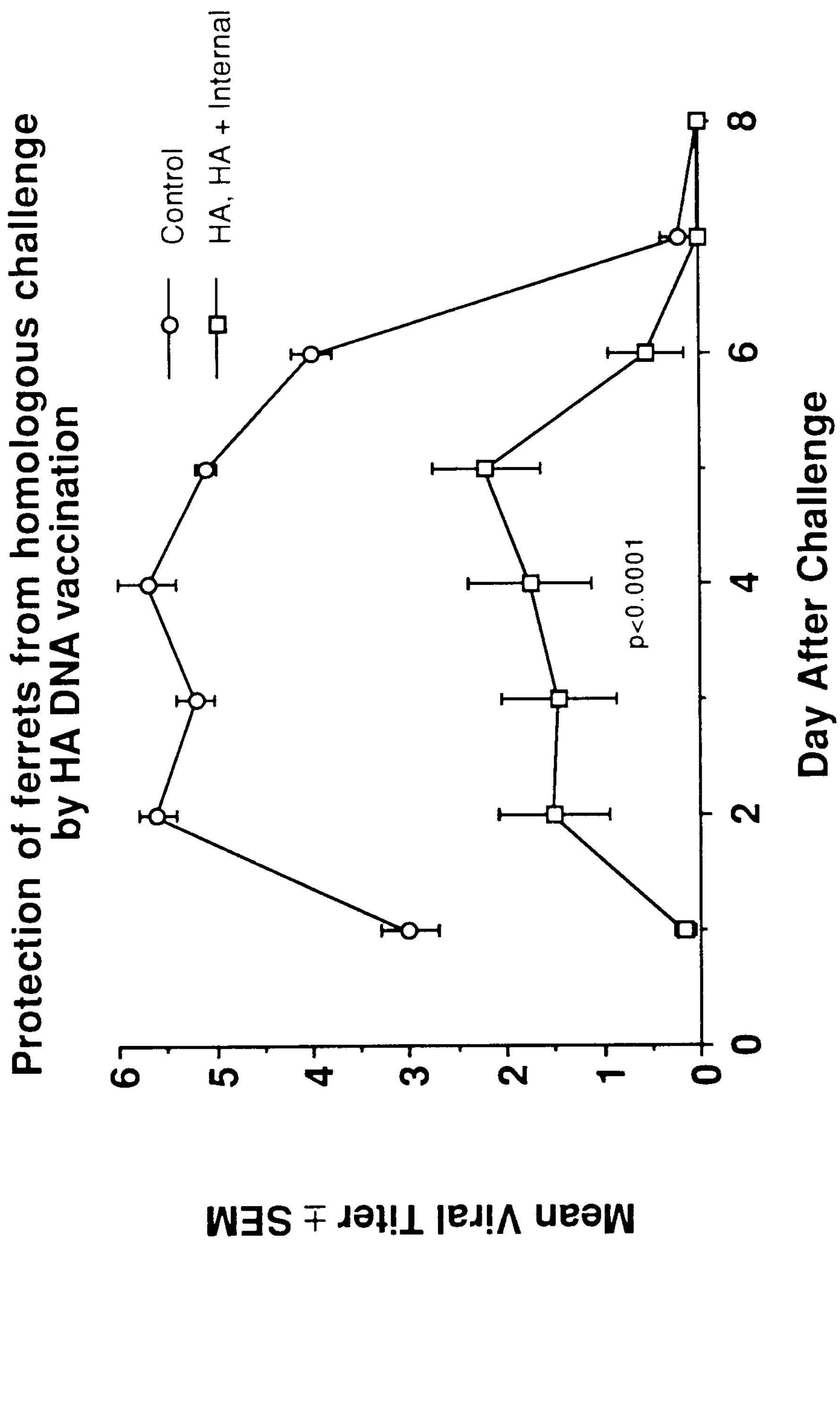
Protection of ferrets from homologous challenge
by HA DNA vaccination
Control
HA, HA + Internal
p<0.0001
Mean Viral Titer ± SEM
Day After Challenge
0
1
2
3
4
5
6
0
2
4
6
8

Figure 32

Homologous protection in ferrets after challenge with A/Georgia/93

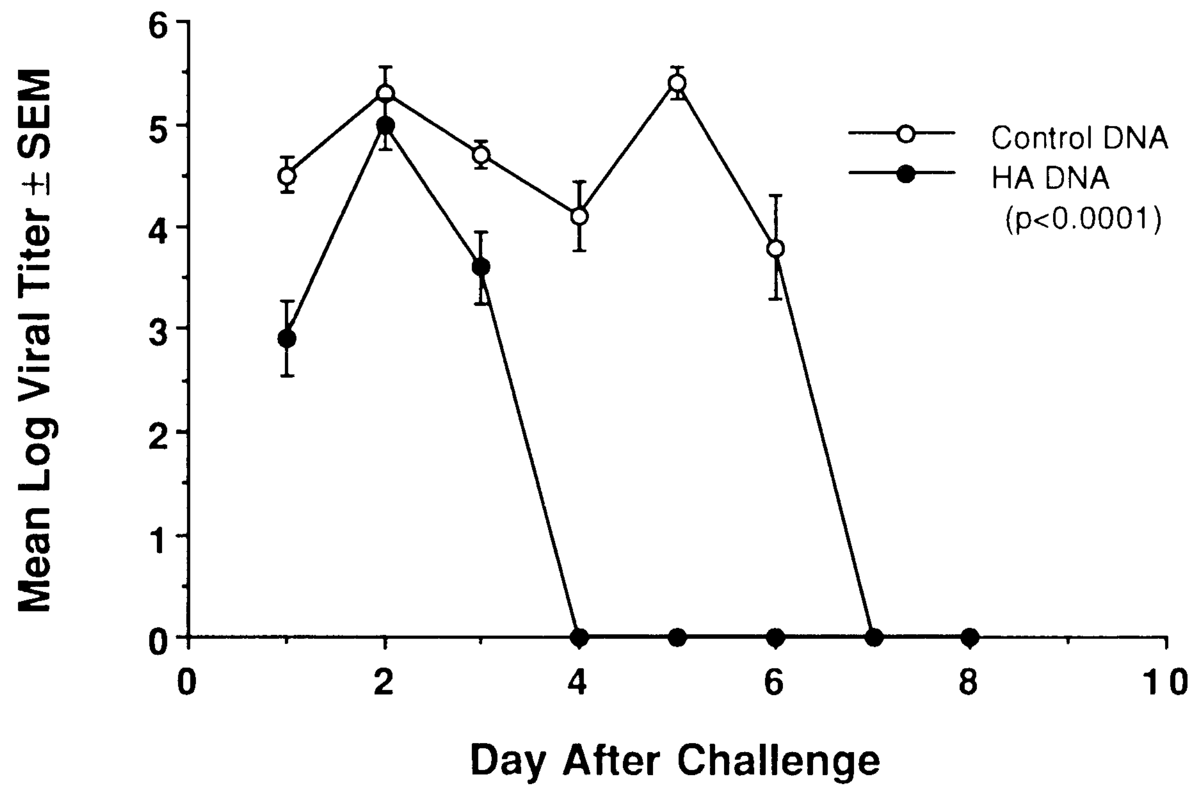

Figure 33

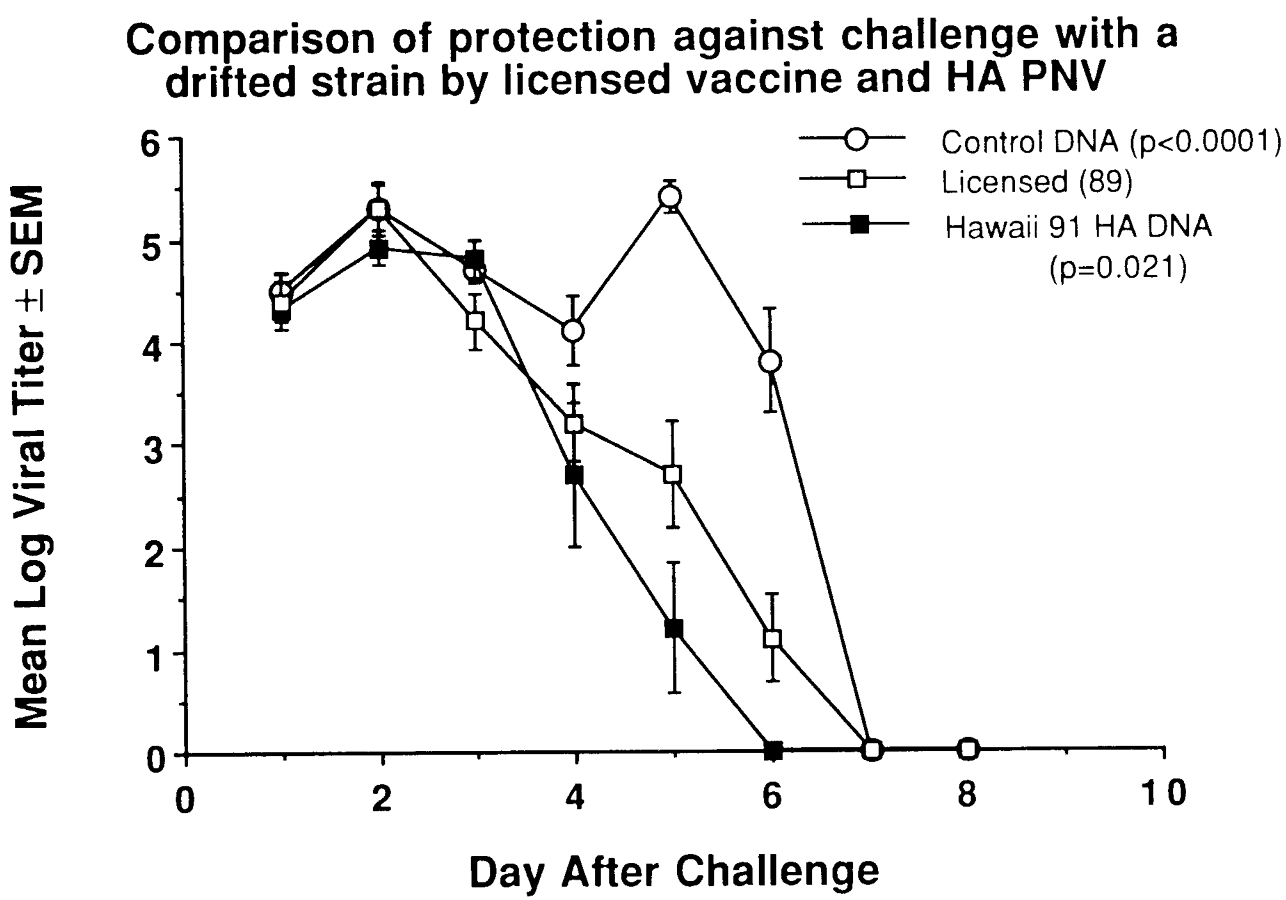
Comparison of protection against challenge with a drifted strain by licensed vaccine and HA PNV
Control DNA (p<0.0001)
Licensed (89)
Hawaii 91 HA DNA (p=0.021)
Mean Log Viral Titer ± SEM
0
1
2
3
4
5
6
0
2
4
6
8
10
Day After Challenge

Figure 34

**Comparison of PNV with licensed vaccine in ferrets after challenge with a drifted strain (A/Georgia/93)**

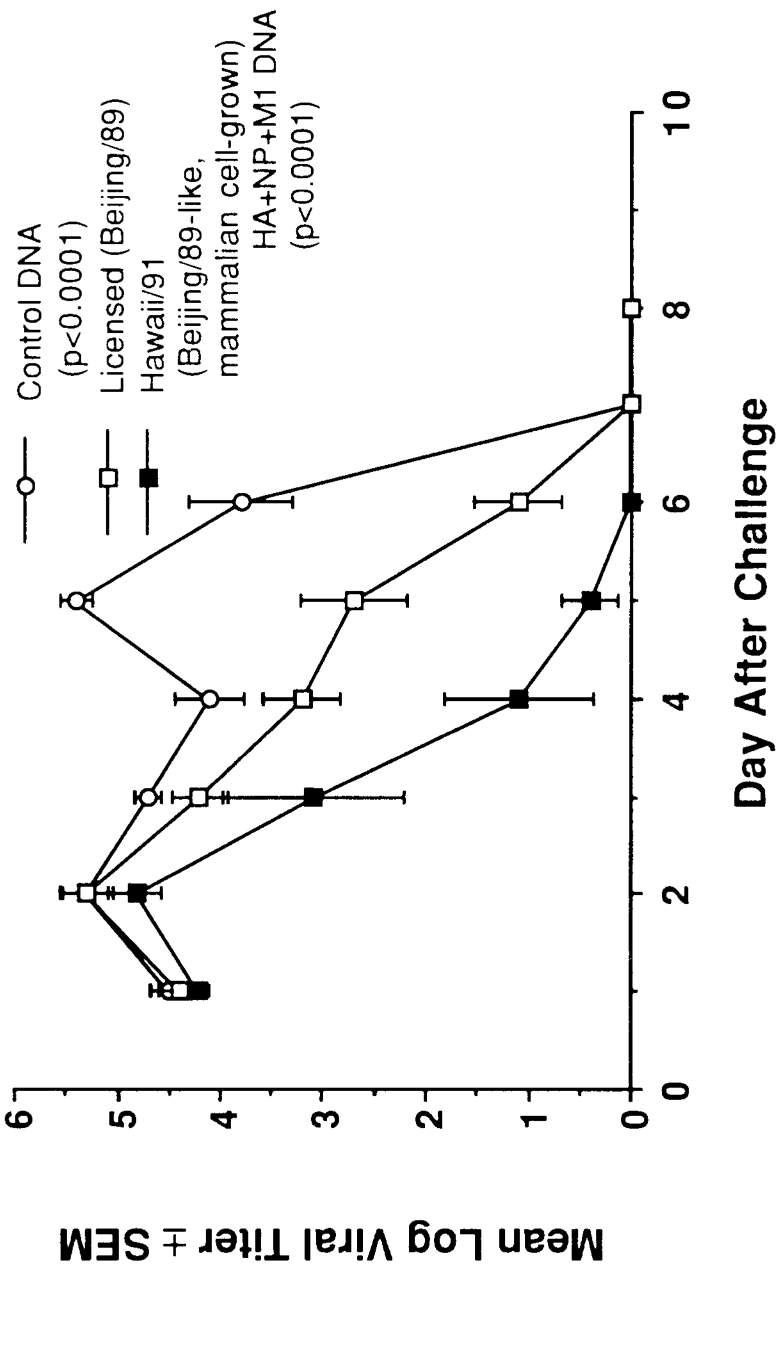

Figure 35

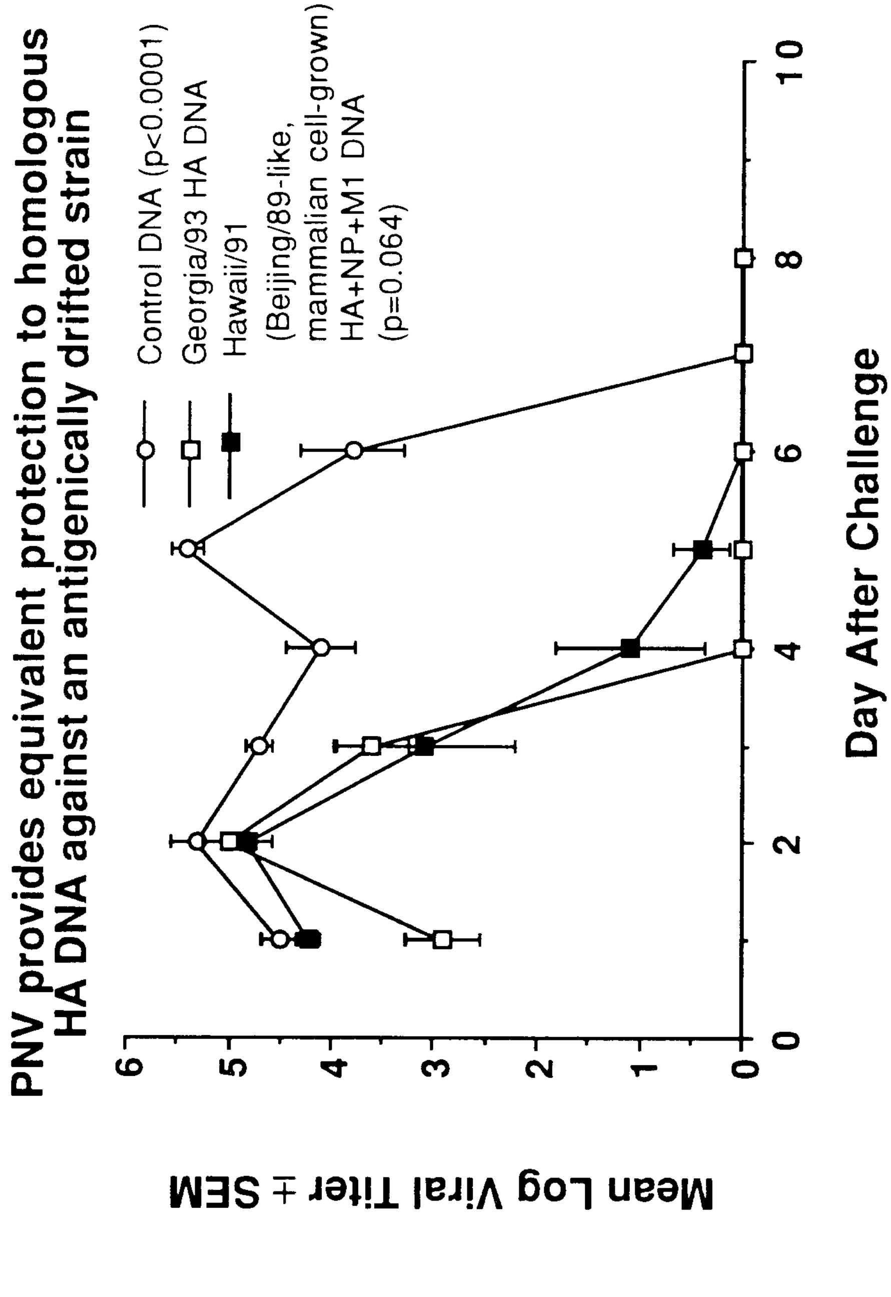

PNV provides equivalent protection to homologous HA DNA against an antigenically drifted strain
Control DNA (p<0.0001)
Georgia/93 HA DNA
Hawaii/91
(Beijing/89-like, mammalian cell-grown) HA+NP+M1 DNA (p=0.064)
Mean Log Viral Titer ± SEM
Day After Challenge
0
1
2
3
4
5
6
0
2
4
6
8
10

FIGURE 36, V1R SEQUENCE, SEQ.ID:45:

1 **GATATTGG** CTATTGGCCA

251 TTGCATACGT TGTATCCATA TCATAATATG TACATTTATA TTGGCTCATG

301 TCCAACATTA CCGCCATGTT GACATTGATT ATTGACTAGT TATTAATAGT

351 AATCAATTAC GGGGTCATTA GTTCATAGCC CATATATGGA GTTCCGCGTT

401 ACATAACTTA CGGTAAATGG CCCGCCTGGC TGACCGCCCA ACGACCCCCG

451 CCCATTGACG TCAATAATGA CGTATGTTCC CATAGTAACG CCAATAGGGA

501 CTTTCCATTG ACGTCAATGG GTGGAGTATT TACGGTAAAC TGCCCACTTG

551 GCAGTACATC AAGTGTATCA TATGCCAAGT ACGCCCCCTA TTGACGTCAA

601 TGACGGTAAA TGGCCCGCCT GGCATTATGC CCAGTACATG ACCTTATGGG

651 ACTTTCCTAC TTGGCAGTAC ATCTACGTAT TAGTCATCGC TATTACCATG

701 GTGATGCGGT TTTGGCAGTA CATCAATGGG CGTGGATAGC GGTTTGACTC

751 ACGGGGATTT CCAAGTCTCC ACCCCATTGA CGTCAATGGG AGTTTGTTTT

801 GGCACCAAAA TCAACGGGAC TTTCCAAAAT GTCGTAACAA CTCCGCCCCA

851 TTGACGCAAA TGGGCGGTAG GCGTGTACGG TGGGAGGTCT ATATAAGCAG

901 AGCTCGTTTA GTGAACCGTC AGATCGCCTG GAGACGCCAT CCACGCTGTT

951 TTGACCTCCA TAGAAGACAC CGGGACCGAT CCAGCCTCCG CGGCCGGGAA

1001 CGGTGCATTG GAACGCGGAT TCCCCGTGCC AAGAGTGACG TAAGTACCGC

1051 CTATAGAGTC TATAGGCCCA CCCCCTTGGC TTCTTATGCA TGCTATACTG

1101 TTTTTGGCTT GGGGTCTATA CACCCCCGCT TCCTCATGTT ATAGGTGATG

1151 GTATAGCTTA GCCTATAGGT GTGGGTTATT GACCATTATT GACCACTCCC

1201 CTATTGGTGA CGATACTTTC CATTACTAAT CCATAACATG GCTCTTTGCC

1251 ACAACTCTCT TTATTGGCTA TATGCCAATA CACTGTCCTT CAGAGACTGA

1301 CACGGACTCT GTATTTTTAC AGGATGGGGT CTCATTTATT ATTTACAAAT

1351 TCACATATAC AACACCACCG TCCCCAGTGC CCGCAGTTTT TATTAAACAT

Figure 36 (continued, p2/3)

1401 AACGTGGGAT CTCCACGCGA ATCTCGGGTA CGTGTTCCGG ACATGGGCTC

1451 TTCTCCGGTA GCGGCGGAGC TTCTACATCC GAGCCCTGCT CCCATGCCTC

1501 CAGCGACTCA TGGTCGCTCG GCAGCTCCTT GCTCCTAACA GTGGAGGCCA

1551 GACTTAGGCA CAGCACGATG CCCACCACCA CCAGTGTGCC GCACAAGGCC

1601 GTGGCGGTAG GGTATGTGTC TGAAAATGAG CTCGGGGAGC GGGCTTGCAC

1651 CGCTGACGCA TTTGGAAGAC TTAAGGCAGC GGCAGAAGAA GATGCAGGCA

1701 GCTGAGTTGT TGTGTTCTGA TAAGAGTCAG AGGTAACTCC CGTTGCGGTG

1751 CTGTTAACGG TGGAGGGCAG TGTAGTCTGA GCAGTACTCG TTGCTGCCGC

1801 GCGCGCCACC AGACATAATA GCTGACAGAC TAACAGACTG TTCCTTTCCA

1851 TGGGTCTTTT CTGCAGTCAC CGTCCTTAG ATCTGCTGTG CCTTCTAGTT

1901 GCCAGCCATC TGTTGTTTGC CCCTCCCCCG TGCCTTCCTT GACCCTGGAA

1951 GGTGCCACTC CCACTGTCCT TTCCTAATAA AATGAGGAAA TTGCATCGCA

2001 TTGTCTGAGT AGGTGTCATT CTATTCTGGG GGGTGGGGTG GGGCAGCACA

2051 GCAAGGGGGA GGATTGGGAA GACAATAGCA GGCATGCTGG GGATGCGGTG

2101 GGCTCTATGG GTAC GGCCGCAGCGGCC GTACCCAGGT GCTGAAGAAT

TGACCCGGTT CCTCGACCCGT AAAAAGGCCG

2601 CGTTGCTGGC GTTTTTCCAT AGGCTCCGCC CCCCTGACGA GCATCACAAA

2651 AATCGACGCT CAAGTCAGAG GTGGCGAAAC CCGACAGGAC TATAAAGATA

2701 CCAGGCGTTT CCCCCTGGAA GCTCCCTCGT GCGCTCTCCT GTTCCGACCC

2751 TGCCGCTTAC CGGATACCTG TCCGCCTTTC TCCCTTCGGG AAGCGTGGCG

2801 CTTTCTCAAT GCTCACGCTG TAGGTATCTC AGTTCGGTGT AGGTCGTTCG

2851 CTCCAAGCTG GGCTGTGTGC ACGAACCCCC CGTTCAGCCC GACCGCTGCG

2901 CCTTATCCGG TAACTATCGT CTTGAGTCCA ACCCGGTAAG ACACGACTTA

2951 TCGCCACTGG CAGCAGCCAC TGGTAACAGG ATTAGCAGAG CGAGGTATGT

3001 AGGCGGTGCT ACAGAGTTCT TGAAGTGGTG GCCTAACTAC GGCTACACTA

Figure 36 (continued, p3/3)

3051 GAAGGACAGT ATTTGGTATC TGCGCTCTGC TGAAGCCAGT TACCTTCGGA

3101 AAAAGAGTTG GTAGCTCTTG ATCCGGCAAA CAAACCACCG CTGGTAGCGG

3151 TGGTTTTTTT GTTTGCAAGC AGCAGATTAC GCGCAGAAAA AAAGGATCTC

3201 AAGAAGATCC TTTGATCTTT TCTACG**TGATCC** CGTAATGC TCTGCCAGTG

TTACAACCAA TTAACCAATT CTGATTAGAA

3751 AAACTCATCG AGCATCAAAT GAAACTGCAA TTTATTCATA TCAGGATTAT

3801 CAATACCATA TTTTTGAAAA AGCCGTTTCT GTAATGAAGG AGAAAACTCA

3851 CCGAGGCAGT TCCATAGGAT GGCAAGATCC TGGTATCGGT CTGCGATTCC

3901 GACTCGTCCA ACATCAATAC AACCTATTAA TTTCCCCTCG TCAAAAATAA

3951 GGTTATCAAG TGAGAAATCA CCATGAGTGA CGACTGAATC CGGTGAGAAT

4001 GGCAAAAGCT TATGCATTTC TTTCCAGACT TGTTCAACAG GCCAGCCATT

4051 ACGCTCGTCA TCAAAATCAC TCGCATCAAC CAAACCGTTA TTCATTCGTG

4101 ATTGCGCCTG AGCGAGACGA AATACGCGAT CGCTGTTAAA AGGACAATTA

4151 CAAACAGGAA TCGAATGCAA CCGGCGCAGG AACACTGCCA GCGCATCAAC

4201 AATATTTTCA CCTGAATCAG GATATTCTTC TAATACCTGG AATGCTGTTT

4251 TCCCGGGGAT CGCAGTGGTG AGTAACCATG CATCATCAGG AGTACGGATA

4301 AAATGCTTGA TGGTCGGAAG AGGCATAAAT TCCGTCAGCC AGTTTAGTCT

4351 GACCATCTCA TCTGTAACAT CATTGGCAAC GCTACCTTTG CCATGTTTCA

4401 GAAACAACTC TGGCGCATCG GGCTTCCCAT ACAATCGATA GATTGTCGCA

4451 CCTGATTGCC CGACATTATC GCGAGCCCAT TTATACCCAT ATAAATCAGC

4501 ATCCATGTTG GAATTTAATC GCGGCCTCGA GCAAGACGTT TCCCGTTGAA

4551 TATGGCTCAT AACACCCCTT GTATTACTGT TTATGTAAGC AGACAGTTTT

4601 ATTGTTCATG ATGATATATT TTTATCTTGT GCAATGTAAC ATCAGAGATT

4651 TTGAGACACA ACGTGGCTTT CC

European Patent Office

# PARTIAL EUROPEAN SEARCH REPORT

which under Rule 45 of the European Patent Convention shall be considered, for the purposes of subsequent proceedings, as the European search report

Application Number EP 94 20 0605

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | AVIAN DISEASES<br>vol. 36 , 1992<br>pages 515 - 520<br>BROWN ET AL. 'Assessment of retrovirus-expressed nucleoprotein as a vaccine against lethal influenza virus infection of chickens'<br>* the whole document *<br>--- | 1-8, 11-18,20 | C12N15/44<br>A61K48/00<br>A61K31/70 |
| P,X | WO-A-93 19183 (UNIVERSITY OF MASSACHUSETTS MEDICAL CENTER) 30 September 1993<br>* the whole document *<br>--- | 1-8, 11-18,20 | |
| D,A | WO-A-90 11092 (VICAL,INC.) 4 October 1990<br>* example 8 *<br>--- | 1-27 | |
| P,X | SCIENCE.<br>vol. 259 , 19 March 1993 , LANCASTER, PA US<br>pages 1745 - 1749<br>ULMER ET AL. 'Heterologous protection against influenza by injection of DNA encoding a viral protein'<br>* the whole document *<br>----- | 1-27 | TECHNICAL FIELDS SEARCHED (Int.Cl.5)<br>C12N<br>A61K<br>C07K |

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 5 July 1994 | Cupido, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

EP 94 20 0605

-C-

Remark: Although claims 16-21
are directed to a method of
treatment of (diagnostic method
practised on) the human/animal body
(Art. 52(4) EPC) the search has been
carried out and based on the
alleged effects of the compound/
composition